# EUROPEAN PATENT APPLICATION

(11) **EP 4 575 500 A2**
(43) Date of publication of application: **25.06.2025**
(21) Application number: 25162129.8
(22) Date of filing: 21.10.2021
(51) Int. Cl.: G01N 33/53

(54) **METHODS FOR SPATIAL ANALYSIS USING ROLLING CIRCLE AMPLIFICATION**

(30) Priority: 22.10.2020 US 202063104195 P; 19.11.2020 US 202063115763 P
(62) Divisional of application: 21807427.6
(71) Applicant: 10x Genomics, Inc., Pleasanton, CA 94588-3260 (US)
(72) Inventor: MIGNARDI, Marco, Pleasanton, 94588-3260 (US)
(74) Representative: J A Kemp LLP

(57) **Abstract**

Provided herein are methods of identifying a location of an analyte in a biological sample using padlock oligonucleotides and rolling circle amplification. For example, provided herein are methods for identifying a location of an analyte in a biological sample where (i) a padlock oligonucleotide hybridizes to an analyte and is ligated thereby creating a circularized padlock oligonucleotide, (ii) rolling circle amplification of the circularized padlock oligonucleotide results in generation of an amplified circularized padlock oligonucleotide, and (iii) a signal corresponding to the amplified circularized padlock oligonucleotide is detected and used to identify the location of the analyte in the biological sample. Also provided are methods for identifying whether a treatment of a biological sample facilitates the release and subsequent detection of an analyte from the biological sample using padlock oligonucleotides and rolling circle amplification.

## Description

### CROSS-REFERENCE TO RELATED APPLICATIONS

This application claims the benefit of U.S. Provisional Application Serial No. 63/104,195, filed October 22, 2020, and U.S. Provisional Application Serial No. 63/115,763, filed November 19, 2020, the entire contents of each of which are incorporated by reference herein.

### BACKGROUND

Cells within a tissue of a subject have differences in cell morphology and/or function due to varied analyte levels (e.g., gene and/or protein expression) within the different cells. The specific position of a cell within a tissue (e.g., the cell's position relative to neighboring cells or the cell's position relative to the tissue microenvironment) can affect, e.g., the cell's morphology, differentiation, fate, viability, proliferation, behavior, and signaling and crosstalk with other cells in the tissue.

Spatial heterogeneity has been previously studied using techniques that only provide data for a small handful of analytes in the context of an intact tissue or a portion of a tissue, or provides substantial analyte data for dissociated tissue (i.e., single cells), but fail to provide information regarding the position of the single cell in a parent biological sample (e.g., tissue sample).

Generally, spatial analysis requires determining the sequence of the analyte sequence or a complement thereof and the sequence of the spatial barcode or a complement thereof in a biological sample in order to identify the spatial location of the analyte in the biological sample. Typically, this requires sequencing which can be time and resource intensive. Therefore, there is a need to assess biological sample quality prior to traditional spatial analysis. There also remains a need to develop in situ methods of detection on a spatial array.

### SUMMARY

The present disclosure provides methods for detection of an analyte, even when the analytes are at low abundance, in a biological sample. The techniques disclosed herein facilitate downstream processing, including situations where sequencing is not desired. Instead, the location of the analyte is determined using rolling circle amplification and detection of a padlock oligonucleotide that hybridizes to an immobilized analyte (e.g., mRNA).

Furthermore, the present disclosure provides solutions, methods and compositions, for addressing problems that might be associated with incomplete permeabilization of a tissue for spatial analyte detection. Permeabilization of tissues is one important aspect of performing spatial transcriptomics. If a tissue is not efficiently or completely permeabilized such that the target analytes are impeded from hybridizing to their capture sequences on the spatial array, then incomplete spatial analyte information may result. As tissues differ, so might their permeabilization conditions such as permeabilization time, permeabilization enzyme concentrations, and the type of enzyme that is used. Further, some experimental conditions such as direct fluorescence detection on an optimization slide do not correlate with downstream experimental detection. As such, optimizing the best permeabilization for any particular tissue may be needed for complete analyte capture.

To address this issue, disclosed herein are compositions and methods to test tissue permeabilization, diffusion, and capture of analytes and analyte derived molecule, such as templated ligation (RTL) probes. The methods provided herein utilize capture probes that include a blocking moiety on the 3' ends of each capture probe so that further amplification of the capture probe itself is not performed. Instead, the methods utilize padlock probes and rolling circle amplification of the padlock probes to detect (e.g., using microscopy and digital quantification of a microscopy image) abundance and location of an analyte in order to test permeabilization conditions.

The present disclosure also provides *in situ* methods of detection of abundance and location of an analyte. Compared to traditional methods of analyte capture, the present disclosure provides decreased costs and increased resolution since capture areas do not need to be spotted. Further, because detection is performed *in situ* using a detection probe, capture probes do not necessarily need to include spatial barcodes. Instead, fiducial markers on the slide can be utilized. Additionally, since analytes or analyte derived molecules are captured *in situ,* diffusion of the analytes or analyte derived molecules is more limited compared to capture on a spatial array.

The present disclosure provides additional downstream advantages as well. For instance, the tissue can be removed, thereby limiting autofluorescence, limiting the need for a longer rolling circle amplification product, and generating denser signals. On the other hand, if desired, multiple rounds of padlocking, RCA, imaging can mitigate crowding on the array. Finally, the methods provided herein can utilize padlock probes and detection probes, which can be selectively sequenced instead of whole transcriptome sequencing.

Thus, disclosed herein are methods for identifying abundance and location of an analyte in a biological sample. In some instances, the methods comprises (a) contacting the biological sample with a substrate comprising a plurality of capture probes, wherein a capture probe comprises a capture domain and a blocking moiety at its 3' end; (b) delivering a plurality of templated ligation (RTL) probes, wherein the plurality of RTL probes comprises RTL pairs, wherein each RTL pair comprises a first RTL probe and a second RTL probe, wherein the first RTL probe and the second RTL probe each comprise sequences that are substantially complementary to sequences of the analyte, and wherein the second RTL probe comprises a capture probe capture domain that is complementary to all or a portion of the capture domain; (c) hybridizing the first probe and the second probe to the analyte; (d) generating a ligation product by ligating the first RTL probe and the second RTL probe; (e) releasing the ligation product from the analyte; (f) hybridizing the ligation product to the capture domain; (g) hybridizing a padlock oligonucleotide to the ligation product hybridized to the capture domain; (h) generating a circularized padlock oligonucleotide; (i) amplifying the circularized padlock oligonucleotide using rolling circle amplification, thereby generating an amplified circularized padlock oligonucleotide; and (j) detecting the amplified circularized padlock oligonucleotide, thereby identifying the abundance and the location of the analyte in the biological sample.

In a second embodiment, disclosed are methods of identifying abundance and location of an analyte in a biological sample. In some instances, the methods include: (a) contacting the biological sample with a substrate comprising a plurality of capture probes, wherein a capture probe comprises a capture domain and a blocking moiety at its 3' end; (b) hybridizing the analyte to the capture domain; (c) hybridizing a padlock oligonucleotide to the analyte hybridized to a capture domain; (d) generating a circularized padlock oligonucleotide; (e) amplifying the circularized padlock oligonucleotide using rolling circle amplification, thereby generating an amplified circularized padlock oligonucleotide; and (f) detecting the amplified circularized padlock oligonucleotide, thereby identifying the abundance and the location of the analyte in the biological sample.

In some instances, the method of the first or second embodiment further comprises determining one or more permeabilization conditions. In particular instances, the method comprises applying the one or more permeabilization conditions to the biological sample.

In a third embodiment, disclosed are methods of determining one or more permeabilization conditions for detection of the abundance and location of an analyte in a biological sample. In some instances, the methods include: (a) contacting the biological sample with a substrate comprising a plurality of capture probes, wherein a capture probe comprises a capture domain and a blocking moiety at its 3' end; (b) applying the one or more permeabilization conditions to the biological sample; (c) delivering a plurality of templated ligation (RTL) probes, wherein the plurality of RTL probes comprises RTL pairs, wherein each RTL pair comprises a first RTL probe and a second RTL probe, wherein the first RTL probe and the second RTL probe each comprise sequences that are substantially complementary to sequences of the analyte, and wherein the second RTL probe comprises a capture probe capture domain that is complementary to all or a portion of the capture domain; (d) hybridizing the first probe and the second probe to the analyte; (e) generating a ligation product by ligating the first RTL probe and the second RTL probe; (f) releasing the ligation product from the analyte; (g) hybridizing the ligation product to the capture domain; (h) hybridizing a padlock oligonucleotide to the ligation product hybridized to the capture domain; (i) generating a circularized padlock oligonucleotide; (j) amplifying the circularized padlock oligonucleotide using rolling circle amplification, thereby generating an amplified circularized padlock oligonucleotide; and (k) detecting the amplified circularized padlock oligonucleotide, thereby identifying the abundance and the location of the analyte in the biological sample.

In a fourth embodiment, disclosed are methods of determining one or more permeabilization conditions for detection of the abundance and location of an analyte in a biological sample. In some instances, the methods include: (a) contacting the biological sample with a substrate comprising a plurality of capture probes, wherein a capture probe comprises a capture domain and a blocking moiety at its 3' end; (b) applying the one or more permeabilization conditions to the biological sample; (c) hybridizing the analyte to the capture domain; (d) hybridizing a padlock oligonucleotide to the analyte hybridized to a capture domain; (e) generating a circularized padlock oligonucleotide; (f) amplifying the circularized padlock oligonucleotide using rolling circle amplification, thereby generating an amplified circularized padlock oligonucleotide; and (g) detecting the amplified circularized padlock oligonucleotide, thereby identifying the abundance and the location of the analyte in the biological sample.

In some instances, the one or more permeabilization conditions are quantitated, wherein the one or more permeabilization conditions are selected from time of permeabilization, temperature of permeabilization, pH of permeabilization, enzyme concentration, and any combination thereof.

In a fifth embodiment, disclosed are methods of identifying abundance and location of an analyte in a biological sample. In some instances, the methods include: (a) contacting the biological sample with a substrate comprising a plurality of capture probes, wherein a capture probe comprises a capture domain and a blocking moiety at its 3' end; (b) contacting the biological sample with a plurality of analyte capture agents, wherein an analyte capture agent of the plurality of analyte capture agents comprises: (i) an analyte binding moiety that binds specifically to the analyte, (ii) an analyte binding moiety barcode, and (iii) an analyte capture sequence, wherein the analyte capture sequence comprises a sequence that is complementary to the capture domain; (c) binding the analyte to the analyte binding moiety; (d) hybridizing the analyte capture sequence to the capture domain; (e) hybridizing a padlock oligonucleotide to the analyte binding moiety barcode bound to a capture domain; (f) generating a circularized padlock oligonucleotide; (g) amplifying the circularized padlock oligonucleotide using rolling circle amplification, thereby generating an amplified circularized padlock oligonucleotide; and (h) detecting the amplified circularized padlock oligonucleotide, thereby identifying the abundance and the location of the analyte in the biological sample. In some instances, the methods further include determining one or more permeabilization conditions. In some instances, the methods comprise applying the one or more permeabilization conditions to the biological sample; optionally, wherein the one

In a sixth embodiment, disclosed are methods of determining one or more permeabilization conditions for detection of the abundance and location of an analyte in a biological sample. In some instances, the methods include: (a) contacting the biological sample with a substrate comprising a plurality of capture probes, wherein a capture probe comprises a capture domain and a blocking moiety at its 3' end; (b) applying the one or more permeabilization conditions to the biological sample; (c) contacting the biological sample with a plurality of analyte capture agents, wherein an analyte capture agent of the plurality of analyte capture agents comprises: (i) an analyte binding moiety that binds specifically to the analyte, (ii) an analyte binding moiety barcode, and (iii) an analyte capture sequence, wherein the analyte capture sequence comprises a sequence that is complementary to the capture domain; (d) binding the analyte to the analyte binding moiety; (e) hybridizing the analyte capture sequence to the capture domain; (f) hybridizing a padlock oligonucleotide to the analyte binding moiety barcode bound to a capture domain; (g) generating a circularized padlock oligonucleotide; (h) amplifying the circularized padlock oligonucleotide using rolling circle amplification, thereby generating an amplified circularized padlock oligonucleotide; and (i) detecting the amplified circularized padlock oligonucleotide, thereby identifying the abundance and the location of the analyte in the biological sample.

In some instances, the one or more permeabilization conditions are quantitated, wherein the one or more permeabilization conditions are selected from time of permeabilization, temperature of permeabilization, pH of permeabilization, enzyme concentration, and any combination thereof.

In some instances, the analyte is selected from of a lipid, a carbohydrate, a peptide, a protein, a glycoproteins (N-linked or O-linked), a lipoprotein, a phosphoprotein, a specific phosphorylated or acetylated variants of a protein, a amidation variant of a protein, a hydroxylation variants of a protein, a methylation variant of a protein, a ubiquitylation variant of a proteins, a sulfation variant of a protein, a viral coat protein, an extracellular protein, an intracellular protein, an antibody, an antigen binding fragment, or any combination thereof. In some instances, the analyte is a protein.

Also disclosed herein in another aspect, this disclosure features methods for identifying a location of an analyte in a biological sample, including: (a) contacting an analyte or analyte derived molecule with a substrate including a plurality of capture probes, where a capture probe includes a capture domain, and where the analyte or analyte derived molecule includes a capture probe binding domain; (b) hybridizing a padlock oligonucleotide to the analyte or analyte derived molecule bound to a capture domain; (c) circularizing the padlock oligonucleotide; (d) amplifying the circularized padlock oligonucleotide using rolling circle amplification; and (e) detecting the amplified circularized padlock oligonucleotide thereby identifying the location of the analyte in the biological sample. In some embodiments, the padlock oligonucleotide includes: (i) a first sequence that is substantially complementary to a first portion of the analyte or analyte derived molecule, (ii) a backbone sequence including a barcode, and (iii) a second sequence that is substantially complementary to a second portion of the analyte or analyte derived molecule, where the first sequence and the second sequence are substantially complementary to adjacent sequences of the analyte or analyte derived molecule. In some embodiments, identifying the location of the analyte in the biological sample includes detecting a signal corresponding to the amplified padlock oligonucleotide. In some embodiments, detecting a signal includes detecting a fluorescently labelled detection probe that is hybridized to the amplified circularized padlock oligonucleotide. In some embodiments, the analyte in RNA or DNA. In some embodiments, the RNA is mRNA or the DNA is gDNA.

**In** another aspect, this disclosure features methods for identifying a location of a nucleic acid molecule in a biological sample including: (a) contacting the nucleic acid molecule with a substrate, including a plurality of capture probes, where a capture probe includes a capture domain, and where the nucleic acid molecule includes a capture probe binding domain; (b) hybridizing a padlock oligonucleotide to the nucleic acid molecule bound to a capture domain; (c) circularizing the padlock oligonucleotide; and (d) amplifying the circularized padlock oligonucleotide using rolling circle amplification; and (e) detecting the amplified circularized padlock oligonucleotide thereby identifying the location of the nucleic acid in the biological sample.

**In** some embodiments, the padlock oligonucleotide includes: (i) a first sequence that is substantially complementary to a first portion of the nucleic acid molecule, (ii) a backbone sequence including a barcode, and (iii) a second sequence that is substantially complementary to a second portion of the nucleic acid molecule, where the first sequence and the second sequence are substantially complementary to adjacent sequences of the nucleic acid molecule. **In** some embodiments, the nucleic acid is RNA or DNA. **In** some embodiments, the RNA is mRNA or the DNA is gDNA. **In** some embodiments, identifying the location of the nucleic acid molecule in the biological sample includes detecting a signal corresponding to the amplified padlock oligonucleotide. **In** some embodiments, detecting a signal includes detecting a fluorescently labelled detection probe that is hybridized to the amplified circularized padlock oligonucleotide.

**In** another aspect, this disclosure features methods for identifying a location of an analyte in a biological sample including: (a) contacting the biological sample with a substrate including a plurality of capture probes, where a capture probe includes a capture domain; (b) contacting the biological sample with a plurality of analyte capture agents, where an analyte capture agent of the plurality of analyte capture agents includes: (i) an analyte binding moiety that binds specifically to an analyte from the biological sample, (ii) an analyte binding moiety barcode, and (iii) an analyte capture sequence, where the analyte capture sequence binds specifically to the capture domain of the capture probe; (c) binding the analyte from the biological sample to the analyte binding moiety; (d) hybridizing the analyte capture sequence to the capture domain; (e) hybridizing a padlock oligonucleotide to the analyte binding moiety barcode bound to a capture domain; (f) circularizing the padlock oligonucleotide; (g) amplifying the circularized padlock oligonucleotide using rolling circle amplification; and (h) detecting the amplified circularized padlock oligonucleotide thereby identifying the location of the analyte in the biological sample.

In some embodiments of the aforementioned aspect, the method further comprises determining one or more permeabilization conditions. In certain embodiments, the method comprises applying the one or more permeabilization conditions to the biological sample. In additional embodiments, the one or more permeabilization conditions are quantitated, wherein the one or more permeabilization conditions are selected from time of permeabilization, temperature of permeabilization, pH of permeabilization, enzyme concentration, and any combination thereof.

In some embodiments, the padlock oligonucleotide includes: (i) a first sequence that is substantially complementary to a first portion of the analyte binding moiety barcode, (ii) a backbone sequence, and (iii) a second sequence that is substantially complementary to a second portion of the analyte binding moiety barcode, where the first sequence and the second sequence are substantially complementary to adjacent sequences of the analyte binding moiety barcode.

In some embodiments, the analyte is selected from the group consisting of: lipids, carbohydrates, peptides, proteins, glycoproteins (N-linked or O-linked), lipoproteins, phosphoproteins, specific phosphorylated or acetylated variants of proteins, amidation variants of proteins, hydroxylation variants of proteins, methylation variants of proteins, ubiquitylation variants of proteins, sulfation variants of proteins, viral coat proteins, extracellular and intracellular proteins, antibodies, and antigen binding fragments. In some embodiments, the analyte is a protein. In some embodiments, identifying the location of the analyte in the biological sample includes detecting a signal corresponding to the amplified padlock oligonucleotide. In some embodiments, detecting a signal includes detecting a fluorescently labelled detection probe that is hybridized to the amplified circularized padlock oligonucleotide.

In some embodiments, the method further includes: digesting the amplified circularized padlock oligonucleotide; hybridizing a second padlock oligonucleotide to a second analyte or analyte derived molecule, a second nucleic acid molecule, or a second analyte binding moiety barcode bound to the capture domain; circularizing the second padlock oligonucleotide; and amplifying the second circularized padlock oligonucleotide using rolling circle amplification, and detecting the amplified second circularized padlock oligonucleotide thereby identifying the location of the second analyte in the biological sample.

In some embodiments, the second padlock oligonucleotide includes: (i) a third sequence substantially complementary to the second analyte or analyte derived molecule, the second nucleic acid molecule, or the second analyte binding moiety barcode, (ii) a backbone sequence including a barcode, and (iii) a fourth sequence that is substantially complementary to a sequence adjacent to the third sequence in the second analyte or analyte derived molecule, the second nucleic acid molecule, or the second analyte binding moiety barcode;

In some embodiments, the second analyte derived molecule includes a sequence that is substantially different from the sequence of the first analyte derived molecule.

In some embodiments, digesting includes contacting the amplified circularized padlock oligonucleotide with an enzyme that cleaves nucleic acid sequences. **In** some embodiments, the enzyme is uracil-DNA glycosylase (UDG).

In some embodiments, the third sequence is different from the first sequence of the first padlock oligonucleotide; and where the fourth sequence is different from the second sequence of the first padlock oligonucleotide.

In some embodiments, the circularizing step includes ligating the first sequence of the padlock oligonucleotide to the second sequence of the padlock oligonucleotide to create a circularized padlock oligonucleotide.

In some embodiments, where ligating the third sequence of the second padlock oligonucleotide to the fourth sequence of the second padlock oligonucleotide, thereby creating a second circularized padlock oligonucleotide.

In some embodiments, where ligation includes enzymatic ligation or chemical ligation. In some embodiments, the enzymatic ligation utilizes ligase. In some embodiments, where ligase includes a T4 DNA ligase.

In some embodiments, the capture probe is affixed to the substrate at a 5' end of the capture probe. In some embodiments, the plurality of capture probes are uniformly distributed on a surface of the substrate. In some embodiments, the plurality of capture probes are not uniformly distributed on the surface of the substrate. In some embodiments, the capture domain includes a sequence that is at least partially complementary to the analyte or analyte derived molecule, the nucleic acid molecule, or the analyte binding moiety barcode.

In some embodiments, the capture domain of the capture probe includes a homopolymeric sequence. In some embodiments, the capture domain of the capture probe includes a poly(T) sequence. In some embodiments, the capture domain of the capture probe includes a non-homopolymeric sequence. In some embodiments, the capture domain is a defined sequence.

In some embodiments, the capture probe includes a functional domain.

In some embodiments, the capture probe includes a blocking moiety on the 3' end. In some embodiments, the blocking moiety is a reversible blocking moiety. In some embodiments, the blocking moiety is a removable blocking domain, where removal restores the free 3' end of the capture probe.

In some embodiments, the 3' end of the capture probe includes a removable hairpin, where the hairpin blocks extension of the 3' end of the capture probe. In some embodiments, removing the hairpin results in the capture probe including a free 3' end. In some embodiments, additional nucleotides are added in order to reveal and/or restore the blocked 3' end of the capture probe, thereby creating a free 3' end.

In some embodiments, the amplifying step includes: hybridizing one or more amplification primers to the padlock oligonucleotide, the analyte or analyte derived molecule, the nucleic acid molecule, or the analyte binding moiety barcode; and amplifying the padlock oligonucleotide with a polymerase. In some embodiments, the polymerase has strand displacement activity. In some embodiments, the polymerase is a Phi29 DNA polymerase. In some embodiments, the amplification primer is substantially complementary to the backbone sequence of the padlock oligonucleotide. In some embodiments, the amplification primer includes a sequence substantially complementary to the backbone sequence of the padlock oligonucleotide and a sequence substantially complementary to a portion of the analyte or analyte derived molecule, the nucleic acid molecule, or the analyte binding moiety barcode.

In some embodiments, the method further includes tethering the primer to the capture probe on the substrate. In some embodiments, the sequence of the primer that is substantially complementary a portion of the analyte, analyte derived molecule, nucleic acid molecule, or analyte binding moiety barcode is adjacent to the 3' end of the capture probe on the substrate. In some embodiments, tethering includes ligating the capture probe to the primer. In some embodiments, the method further includes generating a free 3' OH on the capture probe. In some embodiments, the tethering includes: generating a free 3' OH on the capture probe; extending the capture probe to generate an extended capture probe; and ligating the extended capture probe to the primer. In some embodiments, generating a free '3 OH on the capture probe includes: removing a blocking moiety from the 3' end of the capture probe; or adding additional nucleic acids to the 3' end of the capture probe. In some embodiments, removing the blocking moiety from the 3' end of the capture probe includes cleaving the oligonucleotide with an endonuclease.

In some embodiments, the detecting step further includes: contacting the amplified circularized padlock oligonucleotide with a plurality of detection probes, where a detection probe from the plurality of detection probes includes a sequence that is substantially complementary to a sequence of the padlock oligonucleotide and a detectable label. In some embodiments, the sequence of the detection probe that is substantially complementary to a sequence of the padlock oligonucleotide is substantially complementary to the barcode sequence of the padlock oligonucleotide. In some embodiments, the detectable label includes a fluorophore.

In some embodiments, the detecting step further includes: determining (i) all or a part of the sequence of the amplified circularized padlock oligonucleotide on the substrate and using the determined sequence of the padlock oligonucleotide to identify the location of the analyte in the biological sample. In some embodiments, the determined sequence of the amplified circularized padlock oligonucleotide includes the first sequence of the padlock oligonucleotide and the second sequence of the padlock oligonucleotide. In some embodiments, the determining step includes sequencing. In some embodiments, the sequencing step includes *in situ* sequencing. In some embodiments, the sequencing step includes generating a sequencing library of the one or more amplified circularized padlock oligonucleotides.

In some embodiments, the substrate includes a fiducial marker.

In some embodiments, the fluorophore is selected from the group consisting of: TOTO^{®}-1 / TO-PRO^{®}-1, TOTO^{®}-3 / TO-PRO^{®}-3, TO-PRO^{®}-5, Ethidium bromide, Ethidium homodimer-1 (EthD-1), YOYO^{®}-1 / YO-PRO^{®}-1, YOYO^{®}-3 / YO-PRO^{®}-3, 7-AAD (7-Aminoactinomycin D), and OliGreen^{®}.

In one aspect, this disclosure features methods for identifying a location of an analyte in a biological sample including: (a) contacting an analyte or analyte derived molecule with a substrate, where the substrate includes a plurality of capture probes, where an capture probe of the plurality of capture probes includes a capture domain, and where the analyte or analyte derived molecule includes a capture probe-binding domain that is capable of binding to the capture domain; (b) hybridizing the capture probe binding domain to the capture domain; (c) hybridizing a padlock oligonucleotide to the analyte molecule or analyte derived molecule bound to the capture domain, where the padlock oligonucleotide includes: (i) a first sequence that is substantially complementary to a first portion of the analyte or analyte derived molecule, (ii) a backbone sequence, and (iii) a second sequence that is substantially complementary to a second portion of the analyte or analyte derived molecule; (d) ligating the first sequence of the padlock oligonucleotide to the second sequence of the padlock oligonucleotide, thereby creating a circularized padlock oligonucleotide; and (e) amplifying the circularized padlock oligonucleotide using rolling circle amplification, thereby creating an amplified circularized padlock oligonucleotide, and using the amplified circularized padlock oligonucleotide to identify the location of the analyte in the biological sample. In some embodiments, the method further includes: (f) detecting a signal corresponding to the amplified circularized padlock oligonucleotide on the substrate, thereby identifying the location of the analyte in the biological sample. In some embodiments, the method further includes quantitating the detected signal. In some embodiments, the analyte derived molecule is from a templated ligation (RTL) reaction.

In another aspect, this disclosure features methods for identifying a location of an analyte in a biological sample including: (a) contacting the biological sample with a substrate, where the substrate includes a plurality of capture probes, where a capture probe of the plurality of capture probes includes a capture domain; (b) contacting the biological sample with a first probe oligonucleotide and a second probe oligonucleotide, where the first probe oligonucleotide and the second probe oligonucleotide are substantially complementary to adjacent sequences of the analyte, and where the second probe oligonucleotide includes a capture probe-binding domain that is capable of binding to a capture domain of the capture probe; (c) hybridizing the first probe oligonucleotide and the second probe oligonucleotide to adjacent sequences of the analyte; (d) ligating the first probe oligonucleotide and the second probe oligonucleotide, thereby creating a ligation product that is substantially complementary to the analyte; (e) releasing the ligation product from the analyte; (f) hybridizing the capture probe-binding domain of the ligation product to the capture domain of the capture probe; (g) hybridizing a padlock oligonucleotide to the ligation product bound to the hybridization domain such that the padlock oligonucleotide is circularized, where the padlock oligonucleotide includes: (i) a first sequence that is substantially complementary to a first portion of the ligation product, (ii) a backbone sequence, and (iii) a second sequence that is substantially complementary to a second portion of the ligation product; and (h) ligating and amplifying the circularized padlock oligonucleotide using rolling circle amplification, thereby creating an amplified circularized padlock oligonucleotide, and using the amplified circularized padlock oligonucleotide to identify the location of the analyte in the biological sample. In some embodiments, the method further includes: (i) detecting a signal corresponding to the amplified circularized padlock oligonucleotide on the substrate, thereby identifying the location of the analyte in the biological sample. In some embodiments, the method further includes quantitating the detected signal.

In some embodiments, prior to hybridizing the capture probe binding domain to the capture domain the biological sample is permeabilized. In some embodiments, parameters of permeabilization are quantitated, where the parameters include one or more parameters selected from time of permeabilization, temperature of permeabilization, pH of permeabilization, or protease concentration.

In another aspect, this disclosure features methods for determining a permeabilization condition for a biological sample including: (a) contacting the biological sample with a substrate, where the substrate includes a plurality of capture probes, where a capture probe of the plurality of capture probes includes a capture domain; where an analyte or analyte derived molecule from the biological samples includes a capture probe-binding domain that is capable of binding to the capture domain; (b) permeabilizing the biological sample; (c) hybridizing the capture probe binding domain to the capture domain; (d) hybridizing a padlock oligonucleotide to the analyte molecule or analyte derived molecule bound to the capture domain, where the padlock oligonucleotide includes: (i) a first sequence that is substantially complementary to a first portion of the analyte or analyte derived molecule, (ii) a backbone sequence, and (iii) a second sequence that is substantially complementary to a second portion of the analyte or analyte derived molecule; (e) ligating the first sequence of the padlock oligonucleotide to the second sequence of the padlock oligonucleotide, thereby creating a circularized padlock oligonucleotide; and (f) amplifying the circularized padlock oligonucleotide using rolling circle amplification, thereby creating an amplified circularized padlock oligonucleotide, and using the amplified circularized padlock oligonucleotide to determine the permeabilization of the biological sample. In some embodiments, the method further includes: (g) detecting a signal corresponding to the amplified circularized padlock oligonucleotide on the substrate, thereby identifying the amount of analytes or analyte derived molecules hybridized to the capture domains from the permeabilized biological sample. In some embodiments, the method further includes quantitating the detected signal. In some embodiments, the analyte derived molecule is from a templated ligation (RTL) reaction.

In some embodiments, the first sequence of the padlock oligonucleotide and the second sequence of the padlock oligonucleotide are substantially complementary to adjacent sequences of the analyte or the analyte derived molecule. In some embodiments, the first sequence of the padlock oligonucleotide is ligated to the second sequence of the padlock oligonucleotide, thereby creating a circularized padlock oligonucleotide. In some embodiments, the ligating step uses enzymatic ligation or chemical ligation to create a circularized padlock oligonucleotide. In some embodiments, the enzymatic ligation utilizes T4 DNA ligase.

In some embodiments, the capture probe is affixed to the substrate at a 5' end. In some embodiments, the capture domain includes a sequence that is at least partially complementary to the analyte or the analyte derived molecule. In some embodiments, the capture domain of the capture probe includes a poly(T) sequence. In some embodiments, the capture probe includes a 3' end that is blocked. In some embodiments, the 3' end is blocked by a blocking moiety. In some embodiments, the blocking moiety is a reversible blocking moiety. In some embodiments, the 3' end of the oligonucleotide includes a removable hairpin. In some embodiments, additional nucleotides are added in order to reveal and/or restore the blocked 3' end of the oligonucleotide, thereby creating a free 3' end.

In some embodiments, the amplifying step includes: hybridizing one or more amplification primers to a circularized padlock oligonucleotide, and amplifying the circularized padlock oligonucleotide using a polymerase. In some embodiments, the polymerase is a Phi29 DNA polymerase. In some embodiments, the amplification primer is substantially complementary to the backbone sequence of the padlock oligonucleotide. In some embodiments, the amplification primer includes a sequence substantially complementary to the backbone sequence of the padlock oligonucleotide and a sequence substantially complementary to a portion hybridized to the analyte or analyte derived molecule.

In some embodiments, the detecting step further includes: contacting the amplified circularized padlock oligonucleotide with a plurality of detection probes, where a detection probe from the plurality of detection probes includes a sequence that is substantially complementary to a sequence of the padlock oligonucleotide and a detectable label. In some embodiments, the detectable label includes a fluorophore. In some embodiments, the detectable label is a fluorescent dye. In some embodiments, the fluorescent dye is selected from the group consisting of Alexa Fluor^{®} 350, Alexa Fluor^{®} 430, Alexa Fluor^{®} 488, Alexa Fluor^{®} 532, Alexa Fluor^{®} 546, Alexa Fluor^{®} 555, Alexa Fluor^{®} 568, Alexa Fluor^{®} 594, Alexa Fluor^{®} 633, Alexa Fluor^{®} 647, Alexa Fluor^{®} 660, Alexa Fluor^{®} 680, Alexa Fluor^{®} 700, Alexa Fluor^{®} 750, FAM, FITC, GFP, Nile Red, Oregon Green^{®} 488, Oregon Green^{®} 500, Oregon Green^{®} 514, Pacific Blue, PBF1, PE (R-phycoerythrin), PE-Cy5, PE-Cy7, PE-Texas Red, PerCP (Peridinin chlorphyll protein), PerCP-Cy5.5 (TruRed), PharRed (APC-Cy7), ROX, HEX, and ATT0647N.

In some embodiments, detecting includes obtaining an image corresponding to the amplified circularized padlock oligonucleotide. In some embodiments, the method further includes registering image coordinates to a fiducial marker.

In some embodiments, the biological sample includes a FFPE sample. In some embodiments, the biological sample includes a tissue section. In some embodiments, the biological sample includes a fresh frozen sample. In some embodiments, the biological sample includes live cells.

In some embodiments, the analyte or analyte derived molecules include RNA and/or DNA. In some embodiments, the analyte or analyte derived molecules include one or more proteins.

In another aspect, this disclosure features a kit, including: (a) one or more padlock oligonucleotides and a ligase; (b) one or more primers and a polymerase; (c) a substrate including a plurality of capture probes, where a capture probe of the plurality of capture probes includes a capture domain; and (d) instructions for performing any of the methods described herein.

In another aspect, this disclosure features a kit, including: (a) one or more padlock oligonucleotides and a ligase; (b) one or more primers and a polymerase; (c) a substrate including a plurality of capture probes, where a capture probe of the plurality of capture probes includes a capture domain; (d) one or more fluorescent dyes and/or one or more detection probes; and (e) instructions for performing any of the methods described herein.

In another aspect, this disclosure features a kit, including: (a) one or more padlock oligonucleotides and a ligase; (b) one or more primers and a polymerase; (c) a substrate including a plurality of capture probes, where a capture probe of the plurality of capture probes includes a capture domain; (d) one or more fluorescent dyes and/or one or more detection probes; (e) a uracil-DNA glycosylase enzyme; and (f) instructions for performing any of the methods described herein.

In another aspect, this disclosure features a kit for use in any of the methods described herein, including:(a) a padlock oligonucleotide and a ligase; (b) one or more primers and a polymerase; (c) a substrate including a plurality of capture probe, where an capture probe of the plurality of capture probes includes a capture domain; (d) a second substrate including a plurality of capture probes, where a capture probe of the plurality of capture probes includes a spatial barcode and a capture domain; and (e) instructions for performing the method of any one of the preceding claims.

In another aspect, this disclosure features a kit for use in any of the methods described herein, including: (a) a first probe oligonucleotide and a second probe oligonucleotide, where the first probe oligonucleotide and the second probe oligonucleotide are substantially complementary to adjacent sequences of the analyte, and where the second probe oligonucleotide includes a hybridization-binding domain; (b) a padlock oligonucleotide and a ligase; (c) one or more amplification primers and a polymerase; (d) a substrate including a plurality of capture probe, where an capture probe of the plurality of capture probes includes a capture domain; (e) a second substrate including a plurality of capture probes, where a capture probe of the plurality of capture probes includes a spatial barcode and a capture domain; and (f) instructions for performing the method of any one of the preceding claims.

In another aspect, this disclosure features a kit for use in any of the methods described herein, including: (a) a first probe oligonucleotide and a second probe oligonucleotide, where the first probe oligonucleotide and the second probe oligonucleotide are substantially complementary to adjacent sequences of the analyte, and where the second probe oligonucleotide includes a hybridization-binding domain; (b) a padlock oligonucleotide and a ligase; (c) one or more amplification primers and a polymerase; (d) a substrate including a plurality of capture probe, where an capture probe of the plurality of capture probes includes a capture domain; and (e) instructions for performing the method of any one of the preceding claims.

In some embodiments of any of the kits described herein, a capture probe of the plurality of capture probes includes a spatial barcode.

In some embodiments of any of the kits described herein, the kit further includes a plurality of detection probes, where a detection probe from the plurality of detection probes includes a sequence that is substantially complementary to a sequence of the padlock oligonucleotide and a detectable label.

All publications, patents, and patent applications mentioned in this specification are herein incorporated by reference to the same extent as if each individual publication, patent, patent application, or item of information was specifically and individually indicated to be incorporated by reference. To the extent publications, patents, patent applications, and items of information incorporated by reference contradict the disclosure contained in the specification, the specification is intended to supersede and/or take precedence over any such contradictory material.

Where values are described in terms of ranges, it should be understood that the description includes the disclosure of all possible sub-ranges within such ranges, as well as specific numerical values that fall within such ranges irrespective of whether a specific numerical value or specific sub-range is expressly stated.

The term "each," when used in reference to a collection of items, is intended to identify an individual item in the collection but does not necessarily refer to every item in the collection, unless expressly stated otherwise, or unless the context of the usage clearly indicates otherwise.

Various embodiments of the features of this disclosure are described herein. However, it should be understood that such embodiments are provided merely by way of example, and numerous variations, changes, and substitutions can occur to those skilled in the art without departing from the scope of this disclosure. It should also be understood that various alternatives to the specific embodiments described herein are also within the scope of this disclosure.

### DESCRIPTION OF DRAWINGS

The following drawings illustrate certain embodiments of the features and advantages of this disclosure. These embodiments are not intended to limit the scope of the appended claims in any manner. Like reference symbols in the drawings indicate like elements.
**FIG. 1** is a schematic diagram showing an example of a barcoded capture probe.
**FIG. 2A** is a schematic diagram of an exemplary substrate and biological sample.
**FIG. 2B** is a schematic illustrating a cross-sectional view of the substrate, capture probe, and captured analyte from a biological sample in **FIG. 2A****.**
**FIG. 3A** is a schematic diagram of an exemplary padlock oligonucleotide.
**FIG. 3B** is a schematic diagram of an exemplary padlock oligonucleotide hybridized to a captured analyte bound to a capture probe on a substrate. In this example, the capture domain of the capture probe is blocked from extension.
**FIG. 3C** is a schematic diagram of an exemplary amplification where the block from the capture probe is released and an amplification primer is ligated to the capture probe for priming subsequent rolling circle amplification of a circularized padlock oligonucleotide.
**FIG. 3D** is a schematic illustrating the product of a rolling circle amplification reaction from **FIG 3C****.**
**FIG. 3E** is a schematic illustrating that the amplified circularized padlock oligonucleotide is tethered to the capture probe and the captured analyte to which the padlock oligonucleotide was hybridized has been removed.
**FIG. 3F** is a schematic showing a plurality of detection probes bound to the amplified circularized padlock oligonucleotide. The schematic also shows a second example where the capture domain of the capture probe is blocked and an amplification primer is hybridized to the padlock oligonucleotide for rolling circle amplification.
**FIG. 4A** is a schematic showing the product of a rolling circle amplification reaction from an amplified circularized padlock oligonucleotide hybridized to a first RNA molecule.
**FIG. 4B** is a schematic illustrating digestion of the amplified circularized padlock oligonucleotide from **FIG. 4A**.
**FIG. 4C** is a schematic showing the product of a rolling circle amplification reaction from an amplified circularized padlock oligonucleotide hybridized to a second RNA molecule.
**FIG. 5** is a schematic illustrating detected signal from a plurality of amplified circularized padlock oligonucleotides in an exemplary tissue sample. Solid squares, empty squares, solid triangles, and empty triangles represent signals from different amplified circularized padlock oligonucleotides.
**FIG. 6A** is a schematic diagram of an exemplary substrate with exemplary biological samples and templated ligation (RTL) probes.
**FIG. 6B** is a schematic illustrating exemplary, non-limiting, non-exhaustive steps for an RTL-mediated analyte capture in a biological sample.
**FIG. 7A** is a schematic diagram of an exemplary padlock oligonucleotide.
**FIG. 7B** is a schematic diagram of an exemplary padlock oligonucleotide hybridized to a ligation product from an RTL workflow that is hybridized to the capture domain of a capture probe on a substrate.
**FIG. 7C** is a schematic diagram of an exemplary amplification primer hybridized to an exemplary circularized padlock oligonucleotide.
**FIG. 7D** is a schematic illustrating the product of a rolling circle amplification reaction from an amplified circularized padlock oligonucleotide and a plurality of detection probes bound to the product of the rolling circle amplification reaction.
**FIG. 8A** is a schematic of an exemplary brain slice including signals corresponding to detection probes bound to RCA products from circularized padlock oligonucleotides after 15 minutes of RCA.
**FIG. 8B** is a schematic of an exemplary brain slice including signals corresponding to detection probes bound to RCA products from circularized padlock oligonucleotides after 30 minutes of RCA.
**FIG. 8C** is a schematic of an exemplary brain slice including signals corresponding to detection probes bound to RCA products from circularized padlock oligonucleotides after 45 minutes of RCA.
**FIG. 8D** is a schematic of an exemplary brain slice including signals corresponding to detection probes bound to RCA products from circularized padlock oligonucleotides after 60 minutes of RCA.
**FIG. 8E** is an exemplary plot showing the RCA count corresponding to the detection probes bound to RCA products from circularized padlock oligonucleotides after 15, 30, 45, and 60 minutes of RCA associated with the tissue or in the area surrounding the tissue.

### DETAILED DESCRIPTION

### A. Introduction

Disclosed herein are methods of increasing detection of an analyte, even when the analyte is in low abundance in a biological sample. The methods disclosed herein can also be used to identify the optimal reaction conditions for performing spatial transcriptomics in a biological sample, for example the optimization of tissue permeabilization conditions. The techniques disclosed herein facilitate downstream processing, including situations where sequencing is not desired. Instead, the location of the analyte is determined using rolling circle amplification and detection of a padlock oligonucleotide that hybridizes to an immobilized analyte (e.g., mRNA) or analyte proxy (e.g., ligation product from a templated ligation workflow). The padlock oligonucleotide that includes a first sequence, a backbone sequence, and a second sequence hybridizes to the analyte or analyte derived molecule bound to a capture domain of a capture probe on a substrate.

As used herein, an "analyte" includes but is not limited to an RNA molecule or a protein. Analytes can be broadly classified into one of two groups: nucleic acid analytes, and non-nucleic acid analytes.

Examples of nucleic acid analytes include DNA analytes such as genomic DNA, methylated DNA, specific methylated DNA sequences, fragmented DNA, mitochondrial DNA, *in situ* synthesized PCR products, and RNA/DNA hybrids.

Examples of nucleic acid analytes also include RNA analytes such as various types of coding and non-coding RNA. Examples of the different types of RNA analytes include messenger RNA (mRNA), ribosomal RNA (rRNA), transfer RNA (tRNA), microRNA (miRNA), and viral RNA. The RNA can be a transcript (e.g., present in a tissue section). The RNA can be small (e.g., less than 200 nucleic acid bases in length) or large (e.g., RNA greater than 200 nucleic acid bases in length). Small RNAs mainly include 5.8S ribosomal RNA (rRNA), 5S rRNA, transfer RNA (tRNA), microRNA (miRNA), small interfering RNA (siRNA), small nucleolar RNA (snoRNAs), Piwi-interacting RNA (piRNA), tRNA-derived small RNA (tsRNA), and small rDNA-derived RNA (srRNA). The RNA can be doublestranded RNA or single-stranded RNA. The RNA can be circular RNA. The RNA can be a bacterial rRNA (e.g., 16s rRNA or 23s rRNA).

Additional examples of analytes include mRNA and cell surface features (e.g., using the labelling agents described herein), mRNA and intracellular proteins (e.g., transcription factors), mRNA and cell methylation status, mRNA and accessible chromatin (e.g., ATAC-seq, DNase-seq, and/or MNase-seq), mRNA and metabolites (e.g., using the labelling agents described herein), a barcoded labelling agent (e.g., the oligonucleotide tagged antibodies described herein) and a V(D)J sequence of an immune cell receptor (e.g., T-cell receptor), mRNA and a perturbation agent (e.g., a CRISPR crRNA/sgRNA, TALEN, zinc finger nuclease, and/or antisense oligonucleotide as described herein). In some embodiments, a perturbation agent can be a small molecule, an antibody, a drug, an aptamer, a miRNA, a physical environmental (e.g., temperature change), or any other known perturbation agents.

Examples of non-nucleic acid analytes include, but are not limited to, lipids, carbohydrates, peptides, proteins, glycoproteins (N-linked or O-linked), lipoproteins, phosphoproteins, specific phosphorylated or acetylated variants of proteins, amidation variants of proteins, hydroxylation variants of proteins, methylation variants of proteins, ubiquitylation variants of proteins, sulfation variants of proteins, viral coat proteins, extracellular and intracellular proteins, antibodies, and antigen binding fragments. In some embodiments, the analyte can be an organelle (e.g., nuclei or mitochondria).

For the purpose of this disclosure, an "analyte" can include any biological substance, structure, moiety, or component to be analyzed. The term "target" can similarly refer to an analyte of interest.

As used herein, an "analyte derived molecule" includes an RTL product (e.g., a ligation product), an analyte capture agent, or an oligonucleotide from an analyte capture agent. Additional embodiments of an analyte and an analyte derived molecule are provided throughout the application.

The padlock oligonucleotide is ligated to create a circularized padlock oligonucleotide and amplified via rolling circle amplification (RCA). The circularized padlock oligonucleotide can be tethered to the capture probe to enable sequencing and identification of the location of the analyte without the need for a spatial barcode. In addition, the amplified circularized padlock oligonucleotide can be detected with detection probes such as a fluorophore labelled oligonucleotide.

This disclosure features methods of identifying the location of an analyte in a biological sample using a padlock oligonucleotide that can bind to an analyte, amplifying the padlock oligonucleotide, and using the amplified products from the circularized padlock oligonucleotide to identify the location of the analyte in the biological sample, where identifying the location of the analyte in the biological sample comprises detecting a signal corresponding to the location of the amplified circularized padlock oligonucleotide. In some cases detecting a signal includes contacting the amplified circularized padlock oligonucleotide with detection probes. In an exemplary process, a padlock oligonucleotide is hybridized to an analyte bound to a capture probe. The padlock oligonucleotide is ligated to create a circularized padlock oligonucleotide. An amplification primer is ligated to the capture probe or an amplification primer is hybridized to the padlock oligonucleotide. The circularized padlock oligonucleotide is amplified via rolling circle amplification, and a signal corresponding to the product of the amplified padlock oligonucleotide is detected using a plurality of detection probes. In some instances, a detection probe of the plurality of detection probes includes a fluorophore.

The methods provided herein can be applied to analyte or analyte derived molecules. Non-limiting examples of analyte derived molecules include an mRNA molecule, a gDNA molecule, a product of reverse transcription (e.g., an extended capture probe), and an analyte binding moiety barcode (e.g., a binding moiety barcode that identifies that analyte binding moiety (e.g., an antibody)).

Additionally, the present disclosure provides solutions, methods and compositions, for addressing problems that might be associated with incomplete permeabilization of a tissue for spatial analyte detection. Permeabilization of tissues is one important aspect of performing spatial transcriptomics. If a tissue is not efficiently or completely permeabilized such that the target analytes are impeded from hybridizing to their capture sequences on the spatial array, then incomplete spatial analyte information may result. As tissues differ, so might their permeabilization conditions. As such, optimizing the best permeabilization for any particular tissue may be needed for complete analyte capture.

The techniques disclosed herein facilitate the determination of whether a permeabilization condition is appropriate for a particular biological sample in advance of a spatial assay. For example, whether a permeabilization condition allows for analyte release for capturing on a spatial array. The adequacy of a permeabilization condition is determined by using amplification and subsequent detection of a padlock oligonucleotide that hybridizes to an analyte. The padlock oligonucleotide that includes a first sequence, a backbone sequence, and a second sequence hybridizes to the analyte molecule bound to a capture domain of a capture probe on a substrate. The padlock oligonucleotide is then ligated to create a circularized padlock oligonucleotide and amplified, for example, by rolling circle amplification. The amplified circularized padlock oligonucleotide can be detected with detection probes (e.g., a fluorophore).

In order to provide a quality control check and save time and resources, the ability of the biological sample (and the analytes within the biological sample) to release the analytes for capture can be assessed. The methods provided herein describe methods for assessing the quality of a biological sample based on the amplification and detection of an analyte. Detection of the signal corresponding to the analyte can be used to identify a biological sample as being sufficiently able to release analytes to be used in a traditional spatial analysis workflow. In some cases, the analyte bound to the capture domain can be transferred to a second substrate for further downstream processing. The process of transferring analytes from the first substrate to the second substrate can be referred to as a "sandwich process". The sandwich process is described in PCT Patent Application Publication No. WO 2020/123320, which is incorporated by reference in its entirety.

In some embodiments, analysis of the detected signal determines the location of the analyte in the biological sample. In some embodiments, based on the analysis of the detected signal, a region of interest and/or a biological sample can be identified and then selectively transferred to a second substrate. In such cases, the first substrate is used to assess the quality of the biological sample, based at least in part, on the analysis of the detected signal. This eliminates unnecessary downstream processing of regions that are not of interest or biological samples that are not of interest, which provides cost, resource and time savings.

In some embodiments, a method for determining whether a biological sample is appropriately prepared for analyte release includes treating the biological sample with conditions expected to release the analyte (e.g., non-limiting permeabilization conditions include different temperatures, times, enzymes, or enzyme concentrations) (a) contacting an analyte or analyte derived molecule with a substrate, wherein the substrate includes a plurality of capture probes, wherein a capture probe of the plurality of capture probes include a capture domain, and wherein the analyte or analyte derived molecule includes a capture probe binding domain that is capable of binding to the capture domain; (b) hybridizing the capture probe binding domain to the capture domain; (c) hybridizing a padlock oligonucleotide to the analyte molecule bound to the capture domain, wherein the padlock oligonucleotide includes: (i) a first sequence that is substantially complementary to a first portion of the analyte or analyte derived molecule, (ii) a backbone sequence, and (iii) a second sequence that is substantially complementary to a second portion of the analyte or analyte derived molecule; (d) ligating the first sequence of the padlock oligonucleotide to the second sequence of the padlock oligonucleotide, thereby creating a circularized padlock oligonucleotide; and (e) amplifying the circularized padlock oligonucleotide using rolling circle amplification, thereby creating an amplified circularized padlock oligonucleotide, and using the amplified circularized padlock oligonucleotide to identify the location of the analyte in the biological sample. The steps above can also be used to identify a location of an analyte in a biological sample. Identification of the location of the analyte further includes detecting a signal corresponding to the amplified circularized padlock oligonucleotide on the substrate. In some embodiments, the ligating step and the amplification step are performed in one reaction. In some embodiments, the signal being detected is a fluorescent moiety, such that the fluorescent moiety interacts at one or more than one location along the amplified circularized padlock oligonucleotide, with detection via fluorescence microscopy, for example.

Spatial analysis methodologies and compositions described herein can provide a vast amount of analyte and/or expression data for a variety of analytes within a biological sample at high spatial resolution, while retaining native spatial context. Spatial analysis methods and compositions can include, e.g., the use of a capture probe including a spatial barcode (e.g., a nucleic acid sequence that provides information as to the location or position of an analyte within a cell or a tissue sample (e.g., mammalian cell or a mammalian tissue sample) and a capture domain that is capable of binding to an analyte (e.g., a protein and/or a nucleic acid) produced by and/or present in a cell. Spatial analysis methods and compositions can also include the use of a capture probe having a capture domain that captures an intermediate agent for indirect detection of an analyte. For example, the intermediate agent can include a nucleic acid sequence (e.g., a barcode) associated with the intermediate agent. Detection of the intermediate agent is therefore indicative of the analyte in the cell or tissue sample.

Non-limiting aspects of spatial analysis methodologies and compositions are described in U.S. Patent Nos. 10,774,374, 10,724,078, 10,480,022, 10,059,990, 10,041,949, 10,002,316, 9,879,313, 9,783,841, 9,727,810, 9,593,365, 8,951,726, 8,604,182, 7,709,198, U.S. Patent Application Publication Nos. 2020/239946, 2020/080136, 2020/0277663, 2020/024641, 2019/330617, 2019/264268, 2020/256867, 2020/224244, 2019/194709, 2019/161796, 2019/085383, 2019/055594, 2018/216161, 2018/051322, 2018/0245142, 2017/241911, 2017/089811, 2017/067096, 2017/029875, 2017/0016053, 2016/108458, 2015/000854, 2013/171621, WO 2018/091676, WO 2020/176788, Rodriques et al., Science 363(6434):1463-1467, 2019; Lee et al., Nat. Protoc. 10(3):442-458, 2015; Trejo et al., PLoS ONE 14(2):e0212031, 2019; Chen et al., Science 348(6233):aaa6090, 2015; Gao et al., BMC Biol. 15:50, 2017; and Gupta et al., Nature Biotechnol. 36:1197-1202, 2018; the Visium Spatial Gene Expression Reagent Kits User Guide (e.g., Rev C, dated June 2020), and/or the Visium Spatial Tissue Optimization Reagent Kits User Guide (e.g., Rev C, dated July 2020), both of which are available at the 10x Genomics Support Documentation website, and can be used herein in any combination, and each of which is incorporated herein by reference in their entireties. Further non-limiting aspects of spatial analysis methodologies and compositions are described herein.

Some general terminology that may be used in this disclosure can be found in Section (I)(b) of WO 2020/176788 and/or U.S. Patent Application Publication No. 2020/0277663. Typically, a "barcode" is a label, or identifier, that conveys or is capable of conveying information (e.g., information about an analyte in a sample, a bead, and/or a capture probe). A barcode can be part of an analyte, or independent of an analyte. A barcode can be attached to an analyte. A particular barcode can be unique relative to other barcodes. For the purpose of this disclosure, an "analyte" can include any biological substance, structure, moiety, or component to be analyzed. The term "target" can similarly refer to an analyte of interest.

Analytes can be broadly classified into one of two groups: nucleic acid analytes, and non-nucleic acid analytes. Examples of non-nucleic acid analytes include, but are not limited to, lipids, carbohydrates, peptides, proteins, glycoproteins (N-linked or O-linked), lipoproteins, phosphoproteins, specific phosphorylated or acetylated variants of proteins, amidation variants of proteins, hydroxylation variants of proteins, methylation variants of proteins, ubiquitylation variants of proteins, sulfation variants of proteins, viral proteins (e.g., viral capsid, viral envelope, viral coat, viral accessory, viral glycoproteins, viral spike, etc.), extracellular and intracellular proteins, antibodies, and antigen binding fragments. In some embodiments, the analyte(s) can be localized to subcellular location(s), including, for example, organelles, e.g., mitochondria, Golgi apparatus, endoplasmic reticulum, chloroplasts, endocytic vesicles, exocytic vesicles, vacuoles, lysosomes, etc. In some embodiments, analyte(s) can be peptides or proteins, including without limitation antibodies and enzymes. Additional examples of analytes can be found in Section (I)(c) of WO 2020/176788 and/or U.S. Patent Application Publication No. 2020/0277663. In some embodiments, an analyte can be detected indirectly, such as through detection of an intermediate agent, for example, a ligation product or an analyte capture agent (e.g., an oligonucleotide-conjugated antibody), such as those described herein.

A "biological sample" is typically obtained from the subject for analysis using any of a variety of techniques including, but not limited to, biopsy, surgery, and laser capture microscopy (LCM), and generally includes cells and/or other biological material from the subject. In some embodiments, a biological sample can be a tissue section. In some embodiments, a biological sample can be a fixed and/or stained biological sample (e.g., a fixed and/or stained tissue section). Non-limiting examples of stains include histological stains (e.g., hematoxylin and/or eosin) and immunological stains (e.g., fluorescent stains). In some embodiments, a biological sample (e.g., a fixed and/or stained biological sample) can be imaged. Biological samples are also described in Section (I)(d) of WO 2020/176788 and/or U.S. Patent Application Publication No. 2020/0277663.

In some embodiments, a biological sample is permeabilized with one or more permeabilization reagents. For example, permeabilization of a biological sample can facilitate analyte capture. Exemplary permeabilization agents and conditions are described in Section (I)(d)(ii)(13) or the Exemplary Embodiments Section of WO 2020/176788 and/or U.S. Patent Application Publication No. 2020/0277663.

Array-based spatial analysis methods involve the transfer of one or more analytes from a biological sample to an array of features on a substrate, where each feature is associated with a unique spatial location on the array. Subsequent analysis of the transferred analytes includes determining the identity of the analytes and the spatial location of the analytes within the biological sample. The spatial location of an analyte within the biological sample is determined based on the feature to which the analyte is bound (e.g., directly or indirectly) on the array, and the feature's relative spatial location within the array.

A "capture probe" refers to any molecule capable of capturing (directly or indirectly) and/or labelling an analyte (e.g., an analyte of interest) in a biological sample. In some embodiments, the capture probe is a nucleic acid or a polypeptide. In some embodiments, the capture probe includes a barcode (e.g., a spatial barcode and/or a unique molecular identifier (UMI)) and a capture domain). In some embodiments, a capture probe can include a cleavage domain and/or a functional domain (e.g., a primer-binding site, such as for next-generation sequencing (NGS)). See, e.g., Section (II)(b) (e.g., subsections (i)-(vi)) of WO 2020/176788 and/or U.S. Patent Application Publication No. 2020/0277663. Generation of capture probes can be achieved by any appropriate method, including those described in Section (II)(d)(ii) of WO 2020/176788 and/or U.S. Patent Application Publication No. 2020/0277663.

In some embodiments, more than one analyte type (e.g., nucleic acids and proteins) from a biological sample can be detected (e.g., simultaneously or sequentially) using any appropriate multiplexing technique, such as those described in Section (IV) of WO 2020/176788 and/or U.S. Patent Application Publication No. 2020/0277663.

In some embodiments, detection of one or more analytes (e.g., protein analytes) can be performed using one or more analyte capture agents. As used herein, an "analyte capture agent" refers to an agent that interacts with an analyte (e.g., an analyte in a biological sample) and with a capture probe (e.g., a capture probe attached to a substrate or a feature) to identify the analyte. In some embodiments, the analyte capture agent includes: (i) an analyte binding moiety (e.g., that binds to an analyte), for example, an antibody or antigen-binding fragment thereof; (ii) analyte binding moiety barcode; and (iii) an analyte capture sequence. As used herein, the term "analyte binding moiety barcode" refers to a barcode that is associated with or otherwise identifies the analyte binding moiety. As used herein, the term "analyte capture sequence" refers to a region or moiety configured to hybridize to, bind to, couple to, or otherwise interact with a capture domain of a capture probe. In some cases, an analyte binding moiety barcode (or portion thereof) may be able to be removed (e.g., cleaved) from the analyte capture agent. Additional description of analyte capture agents can be found in Section (II)(b)(ix) of WO 2020/176788 and/or Section (II)(b)(viii) U.S. Patent Application Publication No. 2020/0277663.

There are at least two methods to associate a spatial barcode with one or more neighboring cells, such that the spatial barcode identifies the one or more cells, and/or contents of the one or more cells, as associated with a particular spatial location. One method is to promote analytes or analyte proxies (e.g., intermediate agents) out of a cell and towards a spatially-barcoded array (e.g., including spatially-barcoded capture probes). Another method is to cleave spatially-barcoded capture probes from an array and promote the spatially-barcoded capture probes towards and/or into or onto the biological sample.

In some cases, capture probes may be configured to prime, replicate, and consequently yield optionally barcoded extension products from a template (e.g., a DNA or RNA template, such as an analyte or an intermediate agent (e.g., a ligation product or an analyte capture agent), or a portion thereof), or derivatives thereof (see, e.g., Section (II)(b)(vii) of WO 2020/176788 and/or U.S. Patent Application Publication No. 2020/0277663 regarding extended capture probes). In some cases, capture probes may be configured to form ligation products with a template (e.g., a DNA or RNA template, such as an analyte or an intermediate agent, or portion thereof), thereby creating ligations products that serve as proxies for a template.

As used herein, an "extended capture probe" refers to a capture probe having additional nucleotides added to the terminus (e.g., 3' or 5' end) of the capture probe thereby extending the overall length of the capture probe. For example, an "extended 3' end" indicates additional nucleotides were added to the most 3' nucleotide of the capture probe to extend the length of the capture probe, for example, by polymerization reactions used to extend nucleic acid molecules including templated polymerization catalyzed by a polymerase (e.g., a DNA polymerase or a reverse transcriptase). In some embodiments, extending the capture probe includes adding to a 3' end of a capture probe a nucleic acid sequence that is complementary to a nucleic acid sequence of an analyte or intermediate agent specifically bound to the capture domain of the capture probe. In some embodiments, the capture probe is extended using reverse transcription. In some embodiments, the capture probe is extended using one or more DNA polymerases. The extended capture probes include the sequence of the capture probe and the sequence of the spatial barcode of the capture probe.

In some embodiments, extended capture probes are amplified (e.g., in bulk solution or on the array) to yield quantities that are sufficient for downstream analysis, e.g., via DNA sequencing. In some embodiments, extended capture probes (e.g., DNA molecules) act as templates for an amplification reaction (e.g., a polymerase chain reaction).

Additional variants of spatial analysis methods, including in some embodiments, an imaging step, are described in Section (II)(a) of WO 2020/176788 and/or U.S. Patent Application Publication No. 2020/0277663. Analysis of captured analytes (and/or intermediate agents or portions thereof), for example, including sample removal, extension of capture probes, sequencing (e.g., of a cleaved extended capture probe and/or a cDNA molecule complementary to an extended capture probe), sequencing on the array (e.g., using, for example, *in situ* hybridization or *in situ* ligation approaches), temporal analysis, and/or proximity capture, is described in Section (II)(g) of WO 2020/176788 and/or U.S. Patent Application Publication No. 2020/0277663. Some quality control measures are described in Section (II)(h) of WO 2020/176788 and/or U.S. Patent Application Publication No. 2020/0277663.

Spatial information can provide information of biological and/or medical importance. For example, the methods and compositions described herein can allow for: identification of one or more biomarkers (e.g., diagnostic, prognostic, and/or for determination of efficacy of a treatment) of a disease or disorder; identification of a candidate drug target for treatment of a disease or disorder; identification (e.g., diagnosis) of a subject as having a disease or disorder; identification of stage and/or prognosis of a disease or disorder in a subject; identification of a subject as having an increased likelihood of developing a disease or disorder; monitoring of progression of a disease or disorder in a subject; determination of efficacy of a treatment of a disease or disorder in a subject; identification of a patient subpopulation for which a treatment is effective for a disease or disorder; modification of a treatment of a subject with a disease or disorder; selection of a subject for participation in a clinical trial; and/or selection of a treatment for a subject with a disease or disorder. Exemplary methods for identifying spatial information of biological and/or medical importance can be found in U.S. Patent Application Publication No. 2021/0140982A1, U.S. Patent Application No. 2021/0198741A1, and/or U.S. Patent Application No. 2021/0199660.

Spatial information can provide information of biological importance. For example, the methods and compositions described herein can allow for: identification of transcriptome and/or proteome expression profiles (e.g., in healthy and/or diseased tissue); identification of multiple analyte types in close proximity (e.g., nearest neighbor analysis); determination of up- and/or down-regulated genes and/or proteins in diseased tissue; characterization of tumor microenvironments; characterization of tumor immune responses; characterization of cells types and their co-localization in tissue; and identification of genetic variants within tissues (e.g., based on gene and/or protein expression profiles associated with specific disease or disorder biomarkers).

Typically, for spatial array-based methods, a substrate functions as a support for direct or indirect attachment of capture probes to features of the array. A "feature" is an entity that acts as a support or repository for various molecular entities used in spatial analysis. In some embodiments, some or all of the features in an array are functionalized for analyte capture. Exemplary substrates are described in Section (II)(c) of WO 2020/176788 and/or U.S. Patent Application Publication No. 2020/0277663. Exemplary features and geometric attributes of an array can be found in Sections (II)(d)(i), (II)(d)(iii), and (II)(d)(iv) of WO 2020/176788 and/or U.S. Patent Application Publication No. 2020/0277663.

Generally, analytes and/or intermediate agents (or portions thereof) can be captured when contacting a biological sample with a substrate including capture probes (e.g., a substrate with capture probes embedded, spotted, printed, fabricated on the substrate, or a substrate with features (e.g., beads, wells) comprising capture probes). As used herein, "contact," "contacted," and/or "contacting," a biological sample with a substrate refers to any contact (e.g., direct or indirect) such that capture probes can interact (e.g., bind covalently or non-covalently (e.g., hybridize)) with analytes from the biological sample. Capture can be achieved actively (e.g., using electrophoresis) or passively (e.g., using diffusion). Analyte capture is further described in Section (II)(e) of WO 2020/176788 and/or U.S. Patent Application Publication No. 2020/0277663.

In some cases, spatial analysis can be performed by attaching and/or introducing a molecule (e.g., a peptide, a lipid, or a nucleic acid molecule) having a barcode (e.g., a spatial barcode) to a biological sample (e.g., to a cell in a biological sample). In some embodiments, a plurality of molecules (e.g., a plurality of nucleic acid molecules) having a plurality of barcodes (e.g., a plurality of spatial barcodes) are introduced to a biological sample (e.g., to a plurality of cells in a biological sample) for use in spatial analysis. In some embodiments, after attaching and/or introducing a molecule having a barcode to a biological sample, the biological sample can be physically separated (e.g., dissociated) into single cells or cell groups for analysis. Some such methods of spatial analysis are described in Section (III) of WO 2020/176788 and/or U.S. Patent Application Publication No. 2020/0277663.

In some cases, spatial analysis can be performed by detecting multiple oligonucleotides that hybridize to an analyte. In some instances, for example, spatial analysis can be performed using templated ligation (RTL). Methods of RTL have been described previously. See, e.g., Credle et al., Nucleic Acids Res. 2017 Aug 21; 45(14):e128. Typically, RTL includes hybridization of two oligonucleotides to adjacent sequences on an analyte (e.g., an RNA molecule, such as an mRNA molecule). In some instances, the oligonucleotides are DNA molecules. In some instances, one of the oligonucleotides includes at least two ribonucleic acid bases at the 3' end and/or the other oligonucleotide includes a phosphorylated nucleotide at the 5' end. In some instances, one of the two oligonucleotides includes a capture domain (e.g., a poly(A) sequence, a non-homopolymeric sequence). After hybridization to the analyte, a ligase (e.g., SplintR ligase) ligates the two oligonucleotides together, creating a ligation product. In some instances, the two oligonucleotides hybridize to sequences that are not adjacent to one another. For example, hybridization of the two oligonucleotides creates a gap between the hybridized oligonucleotides. In some instances, a polymerase (e.g., a DNA polymerase) can extend one of the oligonucleotides prior to ligation. After ligation, the ligation product is released from the analyte. In some instances, the ligation product is released using an endonuclease (e.g., RNAse H). The released ligation product can then be captured by capture probes (e.g., instead of direct capture of an analyte) on an array, optionally amplified, and sequenced, thus determining the location and optionally the abundance of the analyte in the biological sample.

During analysis of spatial information, sequence information for a spatial barcode associated with an analyte is obtained, and the sequence information can be used to provide information about the spatial distribution of the analyte in the biological sample. Various methods can be used to obtain the spatial information. In some embodiments, specific capture probes and the analytes they capture are associated with specific locations in an array of features on a substrate. For example, specific spatial barcodes can be associated with specific array locations prior to array fabrication, and the sequences of the spatial barcodes can be stored (e.g., in a database) along with specific array location information, so that each spatial barcode uniquely maps to a particular array location.

Alternatively, specific spatial barcodes can be deposited at predetermined locations in an array of features during fabrication such that at each location, only one type of spatial barcode is present so that spatial barcodes are uniquely associated with a single feature of the array. Where necessary, the arrays can be decoded using any of the methods described herein so that spatial barcodes are uniquely associated with array feature locations, and this mapping can be stored as described above.

When sequence information is obtained for capture probes and/or analytes during analysis of spatial information, the locations of the capture probes and/or analytes can be determined by referring to the stored information that uniquely associates each spatial barcode with an array feature location. In this manner, specific capture probes and captured analytes are associated with specific locations in the array of features. Each array feature location represents a position relative to a coordinate reference point (e.g., an array location, a fiducial marker) for the array. Accordingly, each feature location has an "address" or location in the coordinate space of the array.

Some exemplary spatial analysis workflows are described in the Exemplary Embodiments section of WO 2020/176788 and/or U.S. Patent Application Publication No. 2020/0277663. See, for example, the Exemplary embodiment starting with "In some non-limiting examples of the workflows described herein, the sample can be immersed... " of WO 2020/176788 and/or U.S. Patent Application Publication No. 2020/0277663. See also, e.g., the Visium Spatial Gene Expression Reagent Kits User Guide (e.g., Rev C, dated June 2020), and/or the Visium Spatial Tissue Optimization Reagent Kits User Guide (e.g., Rev C, dated July 2020).

In some embodiments, spatial analysis can be performed using dedicated hardware and/or software, such as any of the systems described in Sections (II)(e)(ii) and/or (V) of WO 2020/176788 and/or U.S. Patent Application Publication No. 2020/0277663, or any of one or more of the devices or methods described in Sections *Control Slide for Imaging, Methods of Using Control Slides and Substrates for, Systems of Using Control Slides and Substrates for Imaging,* and/or *Sample and Array Alignment Devices and Methods, Informational labels* of WO 2020/123320.

Suitable systems for performing spatial analysis can include components such as a chamber (e.g., a flow cell or sealable, fluid-tight chamber) for containing a biological sample. The biological sample can be mounted for example, in a biological sample holder. One or more fluid chambers can be connected to the chamber and/or the sample holder via fluid conduits, and fluids can be delivered into the chamber and/or sample holder via fluidic pumps, vacuum sources, or other devices coupled to the fluid conduits that create a pressure gradient to drive fluid flow. One or more valves can also be connected to fluid conduits to regulate the flow of reagents from reservoirs to the chamber and/or sample holder.

The systems can optionally include a control unit that includes one or more electronic processors, an input interface, an output interface (such as a display), and a storage unit (e.g., a solid state storage medium such as, but not limited to, a magnetic, optical, or other solid state, persistent, writeable and/or re-writeable storage medium). The control unit can optionally be connected to one or more remote devices via a network. The control unit (and components thereof) can generally perform any of the steps and functions described herein. Where the system is connected to a remote device, the remote device (or devices) can perform any of the steps or features described herein. The systems can optionally include one or more detectors (e.g., CCD, CMOS) used to capture images. The systems can also optionally include one or more light sources (e.g., LED-based, diode-based, lasers) for illuminating a sample, a substrate with features, analytes from a biological sample captured on a substrate, and various control and calibration media.

The systems can optionally include software instructions encoded and/or implemented in one or more of tangible storage media and hardware components such as application specific integrated circuits. The software instructions, when executed by a control unit (and in particular, an electronic processor) or an integrated circuit, can cause the control unit, integrated circuit, or other component executing the software instructions to perform any of the method steps or functions described herein.

In some cases, the systems described herein can detect (e.g., register an image) the biological sample on the array. Exemplary methods to detect the biological sample on an array are described in WO 2021/102003 and/or U.S. Patent Application Serial No. 16/951,854, each of which is incorporated herein by reference in their entireties.

Prior to transferring analytes from the biological sample to the array of features on the substrate, the biological sample can be aligned with the array. Alignment of a biological sample and an array of features including capture probes can facilitate spatial analysis, which can be used to detect differences in analyte presence and/or level within different positions in the biological sample, for example, to generate a three-dimensional map of the analyte presence and/or level. Exemplary methods to generate a two- and/or three-dimensional map of the analyte presence and/or level are described in PCT Application No. 2020/053655 and spatial analysis methods are generally described in WO 2021/102039 and/or U.S. Patent Application Serial No. 16/951,864, each of which is incorporated herein by reference in their entireties.

In some cases, a map of analyte presence and/or level can be aligned to an image of a biological sample using one or more fiducial markers, e.g., objects placed in the field of view of an imaging system which appear in the image produced, as described in the *Substrate Attributes* Section, *Control Slide for Imaging* Section of WO 2020/123320, WO 2021/102005, and/or U.S. Patent Application Serial No. 16/951,843, each of which is incorporated herein by reference in their entireties. Fiducial markers can be used as a point of reference or measurement scale for alignment (e.g., to align a sample and an array, to align two substrates, to determine a location of a sample or array on a substrate relative to a fiducial marker) and/or for quantitative measurements of sizes and/or distances.

Provided below are additional embodiments for the disclosure.

### B. Compositions and Methods for Tissue Optimization and Analyte Detection using Padlock Probes and Rolling Circle Amplification

### (a) Methods

Disclosed herein are methods to detect abundance and location of an analyte in a biological sample using detection of amplification products in spatial methods. In some instances, the methods include contacting the biological sample with a substrate comprising a plurality of capture probes, wherein a capture probe comprises a capture domain and a blocking moiety at its 3' end; capturing an analyte or an analyte derived molecule on the substrate using the array; hybridizing a padlock oligonucleotide to the ligation product hybridized to the capture domain; generating a circularized padlock oligonucleotide; amplifying the circularized padlock oligonucleotide using rolling circle amplification, thereby generating an amplified circularized padlock oligonucleotide; and detecting the amplified circularized padlock oligonucleotide, thereby identifying the abundance and the location of the analyte in the biological sample. The methods used herein can be employed to detect nucleic acids and non-nucleic acids such as proteins. In addition, as will be evident in certain instances, analyte derived molecules such as RTL probes or analyte capture agents are used in the methods. In some instances, the capture domain further includes a spatial barcode.

In some instances, the methods described above are used to optimize conditions for the detection of the abundance and the location of an analyte in a biological sample. As described herein, conditions that are optimized include type of enzyme used to permeabilize a sample; enzyme concentration; length of time to permeabilize a sample; modulating temperature, pH, and/or light exposure; an ion or salt concentration, and any combinations thereof.

### (b) Padlock Oligonucleotides

This disclosure features methods for identifying a location of an analyte in a biological sample using a padlock oligonucleotide. As used herein, a "padlock oligonucleotide" refers to an oligonucleotide that has, at its 5' and 3' ends, sequences (e.g., a first sequence at the 5' end and a second sequence at the 3' end) that are complementary to adjacent or nearby portions (e.g., a first portion and a second portion) of the analyte or analyte derived molecule. Upon hybridization of the padlock oligonucleotide to the first and second portions of the analyte or analyte derived molecule, the two ends of the padlock oligonucleotide are either brought into contact or an end is extended until the two ends are brought into contact, allowing circularization of the padlock oligonucleotide by ligation (e.g., ligation using any of the methods described herein). The oligonucleotide resulting from the ligation can be referred to as the "circularized padlock oligonucleotide." After circularization of the padlock oligonucleotide, rolling circle amplification can be used to amplify the circularized padlock oligonucleotide.

In some embodiments, a first sequence of a padlock oligonucleotide includes a sequence that is substantially complementary to a first portion of the analyte or analyte derived molecule. In some embodiments, the first portion of the analyte or analyte derived molecule is 5' to the second portion of the analyte or the analyte derived molecule. In some embodiments, the first sequence is at least 70% identical (e.g., at least 75% identical, at least 80% identical, at least 85% identical, at least 90% identical, at least 95% identical, or at least 99% identical) to the first portion.

In some embodiments, a backbone sequence of a padlock oligonucleotide includes a sequence that is substantially complementary to an amplification primer. The amplification primer can be a primer used in a rolling circle amplification reaction (RCA) of the ligated padlock oligonucleotide hybridized to the analyte or analyte derived molecules. RCA, or rolling circle amplification, is well known in the art and includes a process by which circularized nucleic acid molecules are amplified with a DNA polymerase with strand displacement capabilities (and other necessary reagents for amplification to occur), thereby creating multiple concatenated copies of the circularized nucleic acid molecules. In some embodiments, the backbone sequence includes a functional sequence. In some embodiments the backbone sequence includes a barcode sequence (e.g., any of the exemplary barcode sequences described herein). In some embodiments, the barcode sequence includes a sequence that is substantially complementary to an amplification primer.

In some embodiments, a second sequence of a padlock oligonucleotide includes a sequence that is substantially complementary to a second portion of the analyte or analyte derived molecule. In some embodiments, the second portion of the analyte or analyte derived molecule is 3' to the first portion of the analyte or the analyte derived molecule. In some embodiments, the second sequence is at least 70% identical (e.g., at least 75% identical, at least 80% identical, at least 85% identical, at least 90% identical, at least 95% identical, or at least 99% identical) to the second portion.

In some embodiments, the first sequence is substantially complementary to a first portion of the analyte or analyte derived molecule that is directly adjacent to the second portion of the analyte or analyte derived molecule to which the second sequence is substantially complementary. In such cases, the first sequence is ligated to the second sequence, thereby creating a circularized padlock oligonucleotide.

In some embodiments, the first sequence is substantially complementary to a first portion of the analyte or analyte derived molecule that is not directly adjacent to the second portion of the analyte or analyte derived molecule to which the second sequence is substantially complementary. In such cases, a "gap" exists between where the first sequence is hybridized to the first potion and where the second sequence is hybridized to the second portion. In some embodiments, there is a sequence (e.g., a gap) in the analyte between the first portion and the second portion of at least 1-100, 1-90, 1-80, 1-70, 1-60, 1-50, 1-40, 1-30, 1-20, 1-10, 1-9, 1-8, 1-7, 1-6, 1-5, 1-4, 1-3, 1-2 or 1 nucleotide(s). In a non-limiting example, a first sequence having a sequence that is complementary to a sequence 5' of the gap and a second sequence having a sequence that is complementary to a sequence 3' of the gap each bind to an analyte leaving a sequence (e.g., the "gap") in between the first and second sequences that is gap-filled thereby enabling ligation and generation of the circularized padlock oligonucleotide. In some instances, to generate a padlock oligonucleotide that includes a first sequence and a second sequence that are close enough to one another to initiate a ligation step, the second sequence is extended enzymatically (e.g., using a polymerase, such as a reverse transcriptase).

In some embodiments, the "gap" sequence between the first sequence and the second sequence include one nucleotide, two nucleotides, three nucleotides, four nucleotides, five nucleotides, six nucleotides, seven nucleotides, eight nucleotides, nine nucleotides, ten nucleotides, 11 nucleotides, 12 nucleotides, 13 nucleotides, 14 nucleotides, 15 nucleotides, 16 nucleotides, 17 nucleotides, 18 nucleotides, 19 nucleotides, 20 nucleotides, 21 nucleotides, 22 nucleotides, 23 nucleotides, 24 nucleotides, at least 25 nucleotides, at least 30 nucleotide, at least 35 nucleotides, at least 40 nucleotides, at least 45 nucleotides, or at least 50 nucleotides.

In some embodiments, extending the second sequence of the padlock oligonucleotide includes a nucleic acid extension reaction (e.g., any of the nucleic acid extension reactions described herein). In some embodiments, extending the second sequence of the padlock oligonucleotide includes reverse transcribing the analyte or analyte derived molecule. In some embodiments, extending the second sequence of the padlock oligonucleotide includes using a reverse transcriptase (e.g., any of the reverse transcriptases described herein). In some embodiments, extending the second sequence of the padlock oligonucleotide includes using a Moloney Murine Leukemia Virus (M-MulV) reverse transcriptase. In some embodiments, the reverse transcriptase includes strand displacement properties. In some embodiments, extending the second sequence of the padlock oligonucleotide generates a sequence that is complementary to the analyte or the analyte derived molecule. In some embodiments, extending the second sequence of the padlock oligonucleotide generates an extended second sequence of the padlock oligonucleotide that is complementary to the analyte or analyte derived molecule. In some embodiments, second sequence of the padlock oligonucleotide generates a sequence that is adjacent to the first sequence of the padlock oligonucleotide.

Once the first and second sequences in a padlock oligonucleotide are adjacent, ligation of the two ends can occur. In some embodiments, the ligation step includes ligating the second sequence to the first sequence of the padlock oligonucleotide using enzymatic or chemical ligation. In some embodiments where the ligation is enzymatic, the ligase is selected from a T4 RNA ligase (Rnl2), a SplintR ligase, a single stranded DNA ligase, or a T4 DNA ligase. In some embodiments, the ligase is a T4 RNA ligase (Rnl2) ligase. In some embodiments, the ligase is a pre-activated T4 DNA ligase as described herein. A non-limiting example describing methods of generating and using pre-activated T4 DNA include U.S. Patent App. No. 8,790,873, the entire contents of which are herein incorporated by reference.

### (c) Amplification of Padlock Oligonucleotides

This disclosure features a method for identifying a location of an analyte in a biological sample using amplification of the circularized padlock oligonucleotide. In a non-limiting example, the method includes an amplifying step where one or more amplification primers are hybridized to the circularized padlock oligonucleotide and the circularized padlock oligonucleotide is amplified using a polymerase. The amplifying step increases the copy number of the analyte or analyte derived molecule for detection. The amplification product(s), i.e., the increased copy number can be detected by detection probes and used to identify the location of the analyte in the biological sample and thereby determine whether the methods for releasing analytes from a biological sample has been successful. In some embodiments, the amplifying step includes rolling circle amplification (RCA).

As used herein, rolling circle amplification (RCA) refers to a polymerization reaction carried out using a single-stranded circular DNA (e.g., a circularized padlock oligonucleotide) as a template and an amplification primer that is substantially complementary to the single-stranded circular DNA (e.g., the circularized padlock oligonucleotide) to synthesize multiple continuous single-stranded copies of the template DNA (e.g., the circularized padlock oligonucleotide). In some embodiments, RCA includes hybridizing one or more amplification primers to the circularized padlock oligonucleotide and amplifying the circularized padlock oligonucleotide using a DNA polymerase with strand displacement activity, such as Phi29 DNA polymerase, *Bst* DNA polymerases (e.g., large fragment, 2.0 and 3.0), Klenow fragment, and Vent or DeepVent DNA polymerases. In some embodiments, a first RCA reaction includes a first padlock oligonucleotide and a first amplification primer (or plurality of first amplification primers). A first RCA reaction can include the first padlock oligonucleotide hybridizing to a first analyte or first analyte derived molecule.

In some embodiments, an RCA reaction is carried out using a DNA polymerase and a dNTP mix including uracil, adenine, guanine and cytosine. In such cases, the uracils are incorporated into the amplified padlock oligonucleotide. In some embodiments, an RCA reaction is carried out using a DNA polymerase and a dNTP mix including uracil, adenine, guanine, cytosine and thymine. In such cases, uracils and thymines are both incorporated into the amplified padlock oligonucleotide.

In some embodiments, the method further includes a second RCA reaction. For example, following a first RCA reaction, a second padlock oligonucleotide and a second amplification primer are added to the substrate. In some embodiments, the second padlock oligonucleotide hybridizes to a second analyte or second analyte derived molecule and is ligated thereby creating a second circularized padlock oligonucleotide. The second circularized padlock oligonucleotide can then be amplified using RCA.

In some embodiments, the second padlock oligonucleotides includes a third sequence and a fourth sequence where each are substantially complementary to sequences on a second analyte (e.g., a different analyte then was bound by the first padlock oligonucleotide in the first RCA reaction). In some embodiments, the second padlock oligonucleotide includes: (i) a third sequence substantially complementary to the second analyte or second analyte derived molecule, (ii) a backbone sequence comprising a barcode, and (iii) a fourth sequence that is substantially complementary to a sequence adjacent to the third sequence in the second analyte or second analyte derived molecule. In some embodiments, the third sequence is at least 70% identical (e.g., at least 75% identical, at least 80% identical, at least 85% identical, at least 90% identical, at least 95% identical, or at least 99% identical) to a portion of the second analyte or second analyte derived molecule. In some embodiments, the fourth sequence is at least 70% identical (e.g., at least 75% identical, at least 80% identical, at least 85% identical, at least 90% identical, at least 95% identical, or at least 99% identical) to a sequence adjacent to the third sequence in the second analyte or second analyte derived molecule.

In some embodiments, the method further includes digesting the amplified circularized padlock oligonucleotide by using an enzyme that cleaves a nucleic acid sequence (e.g., an enzyme that cleaves nucleic acid sequences that include uracil). Digesting and/or removing the amplified circularized padlock oligonucleotide can alleviate the crowding that may result from the physical size of the RCA product and/or the detection probe. In some embodiments, the enzyme includes a uracil-DNA glycosylase (UDG) that cleaves nucleic acid sequences that include uracil incorporated into the amplified padlock oligonucleotide. In some embodiments, the enzyme is a combination of enzymes, such as UDG and DNA glycosylase lyase such as Endonuclease VIII.

**In** some embodiments, the method includes a second RCA reaction where the second RCA includes digesting the amplified circularized padlock oligonucleotide generated in the first RCA reaction and hybridizing, ligating, amplifying, and subsequently identifying a second padlock oligonucleotide. For example, the method further includes digesting the amplified circularized padlock oligonucleotide; hybridizing a second padlock oligonucleotide to a second analyte or second analyte derived molecule bound to the capture domain of the capture probes, wherein the second padlock oligonucleotide comprises: (i) a third sequence substantially complementary to the second analyte or second analyte derived molecule, (ii) a backbone sequence comprising a barcode, and (iii) a fourth sequence that is substantially complementary to a sequence adjacent to the third sequence in the second analyte or second analyte derived molecule; ligating the third sequence of the second padlock oligonucleotide to the fourth sequence of the second padlock oligonucleotide, thereby creating a second circularized padlock oligonucleotide; and amplifying the second circularized padlock oligonucleotide using rolling circle amplification, thereby creating an amplified second circularized padlock oligonucleotide, and using detection probes that hybridize to a portion of the amplified second circularized padlock oligonucleotide to identify the location of the second analyte in the biological sample.

**In** some embodiments, an amplification primer includes a sequence that is substantially complementary to one or more of the first sequence, the backbone sequence, or the second sequence of the padlock oligonucleotide. For example, the amplification primer can be substantially complementary to the backbone sequence. **In** some embodiments, the amplification primer includes a sequence that is substantially complementary to the padlock oligonucleotide and an additional portion of the analyte or analyte derived molecule. For example, the amplification primer can be substantially complementary to the backbone sequence and a portion of the analyte or analyte derived molecule that does not include the first and second portion of the padlock oligonucleotide. **In** some embodiments, the amplification primer includes a sequence that is substantially complementary to two or more of the first sequence, the backbone sequence, or the second sequence of the padlock oligonucleotide and an additional portion of the analyte or analyte derived molecule. By substantially complementary, it is meant that the amplification primer is at least 70%, at least 75%, at least 80%, at least 85%, at least 90%, at least 91%, at least 92%, at least 93%, at least 94%, at least 95%, at least 96%, at least 97%, at least 98%, at least 99%, or 100% complementary to a sequence in the circularized padlock oligonucleotide. By substantially complementary, it is meant that the amplification primer is at least 70%, at least 75%, at least 80%, at least 85%, at least 90%, at least 91%, at least 92%, at least 93%, at least 94%, at least 95%, at least 96%, at least 97%, at least 98%, at least 99%, or 100% complementary to a sequence in the analyte or analyte derived molecule.

### (d) Detection Probes, Signal Detection, and Analyte Detection

This disclosure features a method for identifying a location of an analyte in a biological sample using detection of a signal that corresponds to an amplified circularized padlock oligonucleotide (which thereby corresponds to an analyte or analyte binding molecule). **In** some embodiments, the method includes a step of detecting a signal corresponding to the amplified circularized padlock oligonucleotide on the substrate, thereby identifying the location of the analyte in the biological sample. **In** some embodiments, the method includes a detecting step that includes determining (i) all or a part of the sequence of the amplified circularized padlock oligonucleotide on the substrate and using the determined sequence of the padlock oligonucleotide to identify the location of the analyte in the biological sample.

Additionally, this disclosure features a method for identifying which conditions are optimal for releasing an analyte from a biological sample by detection of a signal that corresponds to an amplified circularized padlock oligonucleotide (which thereby corresponds to an analyte or analyte derived molecule). **In** some embodiments, the method includes a step of detecting a signal corresponding to the amplified circularized padlock oligonucleotide on the substrate, thereby identifying whether a reaction condition, such as a permeabilization condition, results in the detection of an analyte in the biological sample.

**In** some embodiments, the detecting step includes contacting the amplified circularized padlock oligonucleotide with a plurality of detection probes. **In** some embodiments, a detection probe of the plurality of detection probes includes a sequence that is substantially complementary to a sequence of the padlock oligonucleotide, circularized padlock oligonucleotide, or amplified circularized padlock oligonucleotide and a detectable label. For example, the detection probe of the plurality of detection probes can include a sequence that is substantially complementary to a sequence of amplified circularized padlock oligonucleotide and a detectable label. By substantially complementary, it is meant that the detection probe is at least 70%, at least 75%, at least 80%, at least 85%, at least 90%, at least 91%, at least 92%, at least 93%, at least 94%, at least 95%, at least 96%, at least 97%, at least 98%, at least 99%, or 100% complementary to a sequence in the padlock oligonucleotide, circularized padlock oligonucleotide or the amplified circularized padlock oligonucleotide.

In some embodiments, the detectable label is a fluorophore. For example, the fluorophore can be from a group that includes: 7-AAD (7-Aminoactinomycin D), Acridine Orange (+DNA), Acridine Orange (+RNA), Alexa Fluor^{®} 350, Alexa Fluor^{®} 430, Alexa Fluor^{®} 488, Alexa Fluor^{®} 532, Alexa Fluor^{®} 546, Alexa Fluor^{®} 555, Alexa Fluor^{®} 568, Alexa Fluor^{®} 594, Alexa Fluor^{®} 633, Alexa Fluor^{®} 647, Alexa Fluor^{®} 660, Alexa Fluor^{®} 680, Alexa Fluor^{®} 700, Alexa Fluor^{®} 750, Allophycocyanin (APC), AMCA / AMCA-X, 7-Amino-4-methylcoumarin, 6-Aminoquinoline, Aniline Blue, ANS, APC-Cy7, ATTO-TAG^{™} CBQCA, ATTO-TAG^{™} FQ, Auramine O-Feulgen, BCECF (high pH), BFP (Blue Fluorescent Protein), BFP / GFP FRET, BOBO^{™}-1 / BO-PRO^{™}-1, BOBO^{™}-3 / BO-PRO^{™}-3, BODIPY^{®} FL, BODIPY^{®} TMR, BODIPY^{®} TR-X, BODIPY^{®} 530/550, BODIPY^{®} 558/568, BODIPY^{®} 564/570, BODIPY^{®} 581/591, BODIPY^{®} 630/650-X, BODIPY^{®} 650-665-X, BTC, Calcein, Calcein Blue, Calcium Crimson^{™}, Calcium Green-1^{™}, Calcium Orange^{™}, Calcofluor^{®} White, 5-Carboxyfluoroscein (5-FAM), 5-Carboxynaphthofluoroscein, 6-Carboxyrhodamine 6G, 5-Carboxytetramethylrhodamine (5-TAMRA), Carboxy-X-rhodamine (5-ROX), Cascade Blue^{®}, Cascade Yellow^{™}, CCF2 (GeneBLAzer^{™}), CFP (Cyan Fluorescent Protein), CFP / YFP FRET, Chromomycin A3, Cl-NERF (low pH), CPM, 6-CR 6G, CTC Formazan, Cy2^{®}, Cy3^{®}, Cy3.5^{®}, Cy5^{®}, Cy5.5^{®}, Cy7^{®}, Cychrome (PE-Cy5), Dansylamine, Dansyl cadaverine, Dansylchloride, DAPI, Dapoxyl, DCFH, DHR, DiA (4-Di-16-ASP), DiD (DilC18(5)), DIDS, Dil (DilC18(3)), DiO (DiOC18(3)), DiR (DilC18(7)), Di-4 ANEPPS, Di-8 ANEPPS, DM-NERF (4.5-6.5 pH), DsRed (Red Fluorescent Protein), EBFP, ECFP, EGFP, ELF^{®} -97 alcohol, Eosin, Erythrosin, Ethidium bromide, Ethidium homodimer-1 (EthD-1), Europium (III) Chloride, 5-FAM (5-Carboxyfluorescein), Fast Blue, Fluorescein-dT phosphoramidite, FITC, Fluo-3, Fluo-4, FluorX^{®}, Fluoro-Gold^{™} (high pH), Fluoro-Gold^{™} (low pH), Fluoro-Jade, FM^{®} 1-43, Fura-2 (high calcium), Fura-2 / BCECF, Fura Red^{™} (high calcium), Fura Red^{™} / Fluo-3, GeneBLAzer^{™} (CCF2), GFP Red Shifted (rsGFP), GFP Wild Type, GFP / BFP FRET, GFP / DsRed FRET, Hoechst 33342 & 33258, 7-Hydroxy-4-methylcoumarin (pH 9), 1,5 IAEDANS, Indo-1 (high calcium), Indo-1 (low calcium), Indodicarbocyanine, Indotricarbocyanine, JC-1, 6-JOE, JOJO^{™}-1 / JO-PRO^{™}-1, LDS 751 (+DNA), LDS 751 (+RNA), LOLO^{™}-1 / LO-PRO^{™}-1, Lucifer Yellow, LysoSensor^{™} Blue (pH 5), LysoSensor^{™} Green (pH 5), LysoSensor^{™} Yellow/Blue (pH 4.2), LysoTracker^{®} Green, LysoTracker^{®} Red, LysoTracker^{®} Yellow, Mag-Fura-2, Mag-Indo-1, Magnesium Green^{™}, Marina Blue^{®}, 4-Methylumbelliferone, Mithramycin, MitoTracker^{®} Green, MitoTracker^{®} Orange, MitoTracker^{®} Red, NBD (amine), Nile Red, Oregon Green^{®} 488, Oregon Green^{®} 500, Oregon Green^{®} 514, Pacific Blue, PBF1, PE (R-phycoerythrin), PE-Cy5, PE-Cy7, PE-Texas Red, PerCP (Peridinin chlorphyll protein), PerCP-Cy5.5 (TruRed), PharRed (APC-Cy7), C-phycocyanin, R-phycocyanin, R-phycoerythrin (PE), PI (Propidium Iodide), PKH26, PKH67, POPO^{™}-1 / PO-PRO^{™}-1, POPO^{™}-3 / PO-PRO^{™}-3, Propidium Iodide (PI), PyMPO, Pyrene, Pyronin Y, Quantam Red (PE-Cy5), Quinacrine Mustard, R670 (PE-Cy5), Red 613 (PE-Texas Red) , Red Fluorescent Protein (DsRed), Resorufin, RH 414, Rhod-2, Rhodamine B, Rhodamine Green^{™}, Rhodamine Red^{™}, Rhodamine Phalloidin, Rhodamine 110, Rhodamine 123, 5-ROX (carboxy-X-rhodamine), S65A, S65C, S65L, S65T, SBFI, SITS, SNAFL^{®}-1 (high pH), SNAFL^{®}-2, SNARF^{®}-1 (high pH), SNARF^{®}-1 (low pH), Sodium Green^{™}, SpectrumAqua^{®}, SpectrumGreen^{®} #1, SpectrumGreen^{®} #2, SpectrumOrange^{®}, SpectrumRed^{®}, SYTO^{®} 11, SYTO^{®} 13, SYTO^{®} 17, SYTO^{®} 45, SYTOX^{®} Blue, SYTOX^{®} Green, SYTOX^{®} Orange, 5-TAMRA (5-Carboxytetramethylrhodamine), Tetramethylrhodamine (TRITC), Texas Red^{®} / Texas Red^{®}-X, Texas Red^{®}-X (NHS Ester), Thiadicarbocyanine, Thiazole Orange, TOTO^{®}-1 / TO-PRO^{®}-1, TOTO^{®}-3 / TO-PRO^{®}-3, TO-PRO^{®}-5, Tri-color (PE-Cy5), TRITC (Tetramethylrhodamine), TruRed (PerCP-Cy5.5), WW 781, X-Rhodamine (XRITC) , Y66F, Y66H, Y66W, YFP (Yellow Fluorescent Protein), YOYO^{®}-1 / YO-PRO^{®}-1, YOYO^{®}-3 / YO-PRO^{®}-3, 6-FAM (Fluorescein), 6-FAM (NHS Ester), 6-FAM (Azide), HEX, TAMRA (NHS Ester), Yakima Yellow, MAX, TET, TEX615, ATTO 488, ATTO 532, ATTO 550, ATTO 565, ATTO Rho101, ATTO 590, ATTO 633, ATTO 647N, TYE 563, TYE 665, TYE 705, 5' IRDye^{®} 700, 5' IRDye^{®} 800, 5' IRDye^{®} 800CW (NHS Ester), WellRED D4 Dye, WellRED D3 Dye, WellRED D2 Dye, Lightcycler^{®} 640 (NHS Ester), and Dy 750 (NHS Ester).

In some embodiments, the detectable label includes a luminescent or chemiluminescent moiety. Common luminescent/chemiluminescent moieties include, but are not limited to, peroxidases such as horseradish peroxidase (HRP), soybean peroxidase (SP), alkaline phosphatase, and one or more luciferases. These protein moieties can catalyze chemiluminescent or bioluminescent reactions given the appropriate chemical or biological substrates (e.g., an oxidizing reagent plus a chemiluminescent compound). A number of compound families are known to provide chemiluminescence under a variety of conditions. Non-limiting examples of chemiluminescent compound families include 2,3-dihydro-l,4-phthalazinedione luminol, 5-amino-6,7,8-trimethoxy- and the dimethylamino[ca]benz analog. These compounds can luminesce in the presence of alkaline hydrogen peroxide or calcium hypochlorite and base. Other examples of chemiluminescent compound families include, e.g., 2,4,5-triphenylimidazoles, para-dimethylamino and - methoxy substituents, oxalates such as oxalyl active esters, p-nitrophenyl, N-alkyl acridinum esters, luciferins, lucigenins, or acridinium esters.

In some embodiments, the plurality of detection probes include a pool of detection probes where each detection probe includes a sequence different from the other detection probes, thereby enabling detection of signals from two or more different sequences (e.g., two or more different amplified circularized padlock oligonucleotides). In some embodiments, each of the two or more different sequences is located on the same amplified circularized padlock oligonucleotide. In some embodiments, each of the two or more different sequences is located on two or more different amplified circularized padlock oligonucleotides (e.g., each amplified circularized padlock oligonucleotide is derived from a different analyte or analyte derived molecule).

In some embodiments, the detecting step includes contacting the amplified circularized padlock oligonucleotide with a detectable label that can hybridize to nucleic acid sequences in a non-sequence dependent manner. In such cases, the single-stranded copies of the template DNA produced by RCA can be detected using fluorescent dyes that non-specifically bind to the single-stranded nucleic acid (e.g., the amplified circularized padlock oligonucleotide). For example, in the case where an analyte is an amplified circularized padlock oligonucleotide, a fluorescent dye can bind, either directly or indirectly, to the single stranded nucleic acid. The dye can then be detected as a signal corresponding to the amplified circularized padlock oligonucleotide (i.e., the location and/or the abundance of the circularized padlock oligonucleotide). Non-limiting examples of fluorescent dyes that can bind to single stranded nucleic acids include: TOTO^{®}-1 / TO-PRO^{®}-1, TOTO^{®}-3 / TO-PRO^{®}-3, TO-PRO^{®}-5, Ethidium bromide, Ethidium homodimer-1 (EthD-1), YOYO^{®}-1 / YO-PRO^{®}-1, YOYO^{®}-3 / YO-PRO^{®}-3, 7-AAD (7-Aminoactinomycin D), and OliGreen^{®}.

In some embodiments, the detecting step includes contacting the amplified circularized padlock oligonucleotide with a detection probe (e.g., any of the exemplary detection probes described herein (e.g., a detection probe that includes a fluorophore)) and a fluorescent dye that can label single-stranded DNA in a non-sequence dependent manner (e.g., any of the exemplary fluorescent dyes described herein). In such cases, the fluorescent dye could be used to normalize the fluorescent signal detected from the detection probe.

In some embodiments, detecting the signal or signals that correspond to the amplified circularized padlock oligonucleotide on the substrate includes obtaining an image corresponding to the analyte and/or analyte derived molecule on the substrate. In some embodiments, the method further includes registering image coordinates to one or more fiducial markers.

In some embodiments, the method includes repeating the detecting step with a second plurality of detection probes. In such cases, the method includes removing the detection probes from the first detecting step and contacting the amplified circularized padlock oligonucleotide with a second plurality of detection probes. A detection probe of the second plurality of detection probes includes a sequence that is substantially complementary to a sequence of the padlock oligonucleotide that is non-overlapping, partially overlapping, or completely overlapping with the sequence to which a detection probe from the first plurality of detection probes is substantially complementary.

In some embodiments, determining (i) all or a part of the sequence of the amplified circularized padlock oligonucleotide on the substrate includes determining the barcode sequence on the padlock oligonucleotide, wherein the location of the analyte in the biological sample can be identified. Detecting the sequence of the analyte or analyte derived molecule, the nucleic acid molecule, the analyte binding moiety barcode, or the ligation product process is described in PCT Patent Application Publication No. WO 2020/047005, which is incorporated by reference in its entirety. In a non-limiting example, in order to determine the location of the analyte on the substrate, a sequencing anchor can be hybridized to the barcode sequence of the padlock oligonucleotide. Then, the padlock oligonucleotide is contacted with a composition that includes multiple different sequencing probes. In general, the sequencing probes are of the same length, but include different nucleic acids in at least one non-degenerate position in each sequence.

The composition includes four different sequencing probes, each of which has a different nucleic acid in the first sequence position adjacent the 3' end. The sequencing probe that is complementary to the barcode sequence at the non-degenerate position is ligated to the sequencing anchor, and hybridizes to the barcode sequence. The non-hybridized sequencing probes are removed (e.g., washed away). Then, an image of the first label of the hybridized sequencing probe is obtained thereby determining the nucleic acid at this position. This is repeated until all or part of the sequence of the barcode on the padlock oligonucleotide is determined, thereby identifying the location of the padlock oligonucleotide, which is a proxy for the mRNA molecule.

### (e) Substrates, Capture Probes, Biological Samples, and Analytes

This disclosure features a method for identifying a location of an analyte in a biological sample using a substrate that includes a plurality of capture probes, where a capture probe of the plurality of capture probes include a capture domain. **In** some embodiments, the capture probe is affixed to the substrate at a 5' end. **In** some embodiments, the plurality of capture probes are uniformly distributed on a surface of the substrate, following no particular pattern. **In** some embodiments, the plurality of capture probes are located on a surface of the substrate but are distributed on the substrate according to a pattern. **In** some embodiments, the substrate includes a plurality of capture probes, where a capture probe of the plurality of capture probes includes a capture domain and a spatial barcode, whereas in other embodiments the captures probes do not contain a spatial barcode.

**In** some embodiments, the substrate includes a fiducial marker (e.g., any of the fiducial markers described herein). The fiducial markers can be used as a point of reference on the substrate. For example, the spatial location of a signal detected from a detection probe can be determined by using the fiducial as a point of reference on the substrate comparative to an image that includes signals from a detection probe. In some cases, the fiducial marker can be present on a substrate to provide an aid in orientation of the biological sample. In some embodiments, a fiducial marker can be an immobilized molecule with which a detectable signal molecule (e.g., a detectable label from a detection probe) can interact to generate a signal. For example, a marker nucleic acid can be linked or coupled to a chemical moiety capable of fluorescing when subjected to light of a specific wavelength (or range of wavelengths).

This disclosure features a method of identifying a location of an analyte in a biological sample using a substrate that includes a plurality of capture probes, where a capture probe of the plurality of capture probes includes a capture domain. Additionally, the disclosure features a method of identifying a permeabilization condition applied to a biological sample that will release an analyte that is capturable on a substrate that includes a plurality of capture probes, where a capture probe of the plurality of capture probes includes a capture domain.

Multiple substrates used herein are disclosed. **In** some embodiments, disclosed herein are substrates having capture probes, each of which includes both a capture domain and a spatial barcode providing a location on the substrate. **In** some embodiments, disclosed herein are substrates having capture probes, each of which includes both a capture domain but does not include a spatial barcode. Thus, in some instances, the capture probe includes a spatial barcode, but in other instances, the capture probe does not include a spatial barcode.

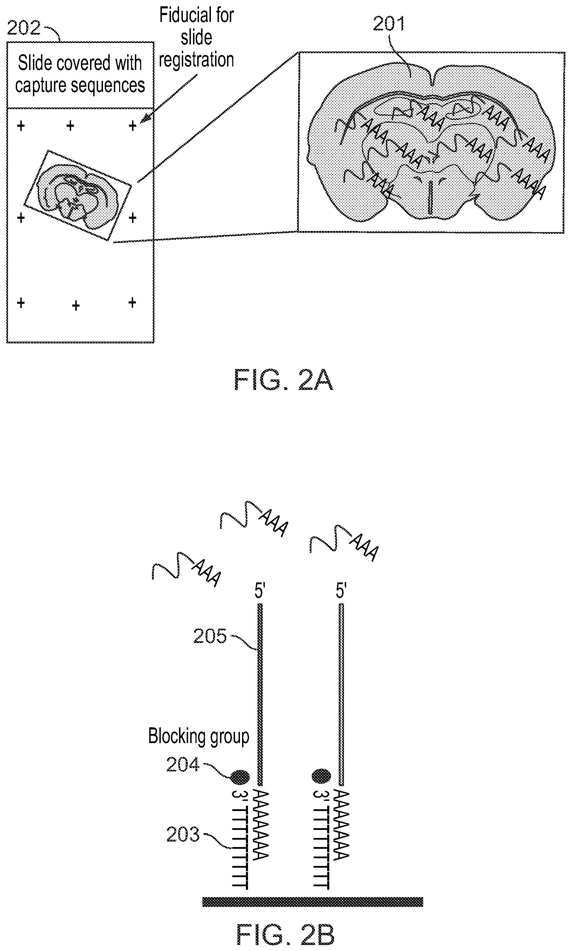

In some embodiments, a capture probe of the plurality of capture probes is affixed to the substrate at the 5' end of the capture probe. In such cases, in order to prevent extension of the capture probe during the amplifying step, the capture probe includes a blocking moiety or blocking group (used interchangeably) on the 3' end as shown e.g., in FIG. 2A. Blocking or modifying the capture probes, particularly at the free 3' end of the capture domain, prior to contacting the biological sample with the array, prevents (1) modification of the capture probes, e.g., prevents the addition of a poly(A) tail or complement thereof to the free 3' end of the capture probes and (2) extension of the capture probe during amplification of the circularized padlock oligonucleotide. It further prevents amplification of the capture probe itself.

In some embodiments, the ability to prevent capture probe extension is temporary (e.g., blocking extension of the capture probe during the amplifying step but allowing extension during, for example, a step that purposely extends the capture probe). In such cases, the blocking moiety on the 3' end can be removed and the capture probe can be extended. In some embodiments, the blocking moiety is released such that an amplification primer can be ligated to the 3' end of the capture probe. For example, the method can include a reversible blocking moiety on the 3' end of the capture probe in a blocking position during amplification but in a non-blocking position (e.g., removed by cleavage) during a capture probe extension step or capture probe ligation step.

In some embodiments where a capture probe of the plurality of capture probes is attached to the substrate at the 5' end, the capture probe is blocked at the 3' end. In some embodiments, the capture probes can be reversibly masked or modified such that the capture domain of the capture probe does not include a free 3' end. In some embodiments, the 3' end is removed, modified, or made inaccessible so that the capture domain is not susceptible to the process used to modify the nucleic acid of the biological sample, e.g., ligation or extension. A capture probe can be blocked at the 3' end by a blocking moiety. The blocking moiety can be a reversible blocking moiety. In some cases, the 3' end of the capture probe includes a removable hairpin. In some cases, the 3' end includes a 3' blocking group present on the sugar moiety of the nucleotide at the 3' end. In such cases, the 3' blocking group can be chemically removed from the nucleic acid.

In some embodiments, the free 3' end of the capture domain can be blocked by chemical modification, e.g., addition of an azidomethyl group as a chemically reversible capping moiety such that the capture probes do not include a free 3' end. Non-limiting examples of 3' modifications include dideoxy C-3' (3'-ddC), 3' inverted dT, 3' C3 spacer, 3'Amino, and 3' phosphorylation.

In some embodiments where a capture probe of the plurality of capture probes is attached to the substrate at the 5' end and the 3' is blocked, additional nucleotides are added to the 3' end in order to reveal and/or restore the blocked 3' end of the capture probe, thereby creating a free 3' end.

In some embodiments, the capture domain includes a sequence that is at least partially complementary to the analyte or the analyte derived molecule. In some embodiments, the capture domain of the capture probe includes a poly(T) sequence. In some embodiments, the capture domain includes a functional domain. In some embodiments, the functional domain includes a primer sequence. In some embodiments, the capture probe includes a cleavage domain. In some embodiments, the cleavage domain includes a cleavable linker from the group consisting of a photocleavable linker, a UV-cleavable linker, an enzyme-cleavable linker, or a pH-sensitive cleavable linker.

In some embodiments, the capture domain of the capture probe comprises a non-homopolymeric sequence. In some embodiments, the capture domain is a defined sequence. In such cases, the defined sequence can be substantially complementary to a portion of the amplified padlock oligonucleotide. When the capture probe is substantially complementary to a portion of the amplified oligonucleotide, the capture domain can be used to capture all or a portion of the padlock oligonucleotide. In some instances, a defined sequence is about 5 to 50 nucleotides in length (e.g., about 5 to 25, about 5 to 20, about 10 to 25, about 10 to 20). In some instances, the length of the defined sequence is about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 nucleotides long. Defined sequences can include natural or synthetic nucleic acids. It is appreciated that a skilled artisan can design the defined sequence in order to capture a particular target or particular targets of interest.

In preferred embodiments, the capture probe is blocked on the 3' end using any blocking method as previously described. The analyte or analyte derived molecule is captured by the capture domain of the capture probe, padlock oligonucleotides are hybridized and circularized and RCA performed, and detection of the analyte or analyte derived molecule determined via one or more detection probes. In this particular embodiment, a blocked capture probe mitigates any modification or extension of the capture probe during amplification of the circularized padlock oligonucleotide.

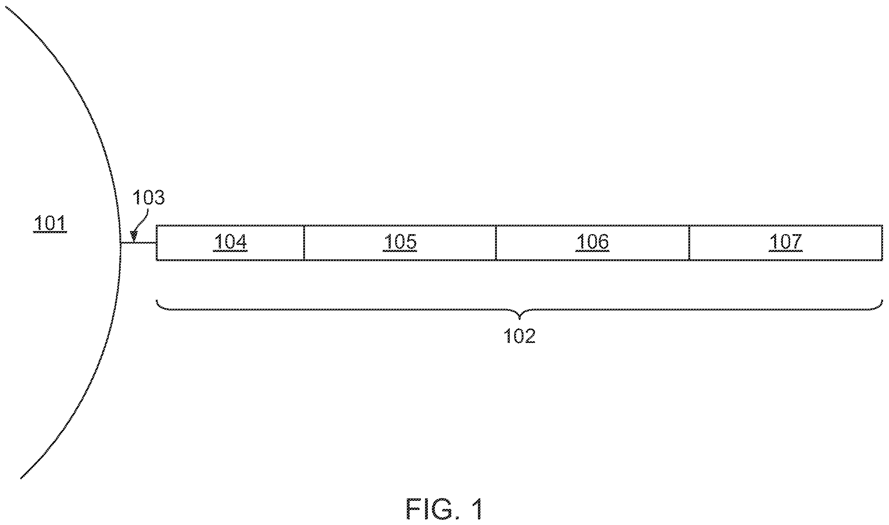

A schematic diagram of an exemplary barcoded capture probe, as described herein, is provided in **FIG. 1****.** As shown, the capture probe **102** is optionally coupled to a feature **101** by a cleavage domain **103,** such as a disulfide linker. The capture probe can include functional sequences that are useful for subsequent processing, such as functional sequence **104,** which can include a sequencer specific flow cell attachment sequence, e.g., a P5 or P7 sequence, as well as functional sequence **106,** which can include sequencing primer sequences, e.g., a R1 primer binding site, a R2 primer binding site. In some embodiments, sequence **104** is a P7 sequence and sequence **106** is a R2 primer binding site. A spatial barcode **105** can be included within the capture probe for use in barcoding the target analyte. The functional sequences can generally be selected for compatibility with any of a variety of different sequencing systems, e.g., Ion Torrent Proton or PGM, Illumina sequencing instruments, PacBio, Oxford Nanopore, etc., and the requirements thereof. In some embodiments, functional sequences can be selected for compatibility with non-commercialized sequencing systems. Examples of such sequencing systems and techniques, for which suitable functional sequences can be used, include (but are not limited to) Ion Torrent Proton or PGM sequencing, Illumina sequencing, PacBio SMRT sequencing, and Oxford Nanopore sequencing. Further, in some embodiments, functional sequences can be selected for compatibility with other sequencing systems, including non-commercialized sequencing systems.

In some embodiments, the spatial barcode 105, functional sequences 104 (e.g., flow cell attachment sequence) and 106 (e.g., sequencing primer sequences) can be common to all of the probes attached to a given feature. The spatial barcode can also include a capture domain 107 to facilitate capture of a target analyte.

In some embodiments, the biological sample comprises a tissue section. In some embodiments, the biological sample comprises a fresh frozen sample. In some embodiments, the biological sample comprises live cells. In some embodiments, the biological sample comprises a FFPE sample.

In some embodiments, the method includes obtaining an image of the biological sample; registering the image data to a spatial location. In some embodiments, the imaging includes a brightfield image. In some embodiments, the method further includes contacting the biological sample with one or more stains. In some embodiments, the one or more stains comprise a histology stain (e.g., any of the histology stains described herein or known in the art). In some embodiments, the one or more stains comprises hematoxylin and eosin. In some embodiments, the one or more stains comprise one or more optical labels (e.g., any of the optical labels described herein). In some embodiments, the one or more optical labels are selected from the group consisting of: fluorescent, radioactive, chemiluminescent, calorimetric, or colorimetric labels.

In some embodiments, the methods are applied to analyte, or analyte derived molecules (e.g., an mRNA molecule, a gDNA molecule, a ligation product from a templated ligation (RTL) assay, a product of reverse transcription (e.g., an extended capture probe), and an analyte binding moiety barcode (e.g., a binding moiety barcode that identifies that analyte binding moiety (e.g., an antibody))). In some embodiments, the analyte or analyte derived molecules comprise RNA and/or DNA. In some embodiments, the analyte or analyte derived molecules comprise one or more proteins.

### (f) RNA Capture

The methods disclosed herein can utilize RNA capture. Generally, analytes (e.g., mRNA) can be captured when contacting a biological sample with a substrate including capture probes. As used herein, "contact," "contacted," and/ or "contacting," a biological sample with a substrate refers to any contact (e.g., direct or indirect) such that capture probes can interact (e.g., capture) with analytes from the biological sample. For example, a substrate may be near or adjacent to the biological sample without direct physical contact, yet capable of capturing analytes from the biological sample. In some embodiments, a biological sample is in direct physical contact with a substrate. In some embodiments, a biological sample is in indirect physical contact with a substrate. For example, a liquid layer may be between the biological sample and the substrate. In some embodiments, analytes diffuse through a liquid layer. In some embodiments, capture probes diffuse through a liquid layer. In some embodiments, reagents may be delivered via a liquid layer between a biological sample and a substrate. In some embodiments, indirect physical contact may include a second substrate (e.g., a hydrogel, a film, a porous membrane) between the biological sample and the first substrate comprising capture probes. In some embodiments, reagents may be delivered by a second substrate to a biological sample.

Capture probes on a substrate (or on a feature on the substrate) may interact with released analytes through a capture domain, described elsewhere, to capture analytes. In some embodiments and as provided herein, certain steps are performed to determine optimal conditions of transfer or capture of analytes to the capture probes of the array. Examples of such modifications include, but are not limited to, adjusting conditions for contacting the substrate with a biological sample (e.g., time, temperature, orientation, pH levels, pre-treating of biological samples, etc.), using force to transport analytes (e.g., electrophoretic, centrifugal, mechanical, etc.).

In some embodiments, the capture domain can include a poly(T) sequence, which poly(T) sequences are configured to interact with messenger RNA (mRNA) molecules via the poly(A) tail of an mRNA transcript. Thus, in some embodiments, the capture domain includes a poly(T) DNA oligonucleotide, e.g., a series of consecutive deoxythymidine residues linked by phosphodiester bonds, which is capable of hybridizing to the poly(A) tail of mRNA. In some embodiments, the capture domain can include nucleotides that are functionally or structurally analogous to a poly(T) tail. For example, a poly(U) oligonucleotide or an oligonucleotide included of deoxythymidine analogues. In some embodiments, the capture domain includes at least 10, 11, 12, 13, 14, 15, 16, 17, 18, 19, or 20 nucleotides. In some embodiments, the capture domain includes at least 25, 30, or 35 nucleotides.

### (g) Templated Ligation

The methods disclosed herein can utilize templated ligation (RTL) to generate analyte derived molecules. RTL is a process by which individual oligonucleotides in a probe pair hybridize to adjacent sequences of an analyte (e.g., an RNA molecule). After hybridizing, the RTL probe oligonucleotides are ligated together. An advantage to using RTL is that it allows for enhanced detection of analytes (e.g., low expressing analytes) because both probe oligonucleotides must hybridize to the analyte in order for the ligation reaction to occur. The ligation product that results from the ligation of the two probe oligonucleotides can serve as a proxy for the target analyte, as such an analyte derived molecule. Further, it is appreciated that probe oligonucleotide pairs can be designed to cover any gene of interest. For example, a pair of probe oligonucleotides can be designed so that each analyte in a genome (e.g., the human genome) can conceivably be detected using a probe oligonucleotide pair.

The methods for RTL include the following steps. In some instances, RTL probe oligonucleotides are added to a biological sample, and each probe hybridizes to an adjacent analyte sequence. The probe oligonucleotides are then ligated together, creating a ligation product. After ligation of the RTL probes, an endonuclease such as RNAse H is added to the sample to digest the RNA analyte from the RNA:DNA hybrid. In some instances, at least one of the RTL probes includes a probe capture sequence such as a poly-A sequence, an oligo-d(T) sequence, or a particular capture sequence (in the setting of targeted RNA analysis), which can hybridize to a capture probe for spatial analysis.

In some embodiments, the methods as disclosed herein include hybridizing of one or more probe oligonucleotide probe pairs (e.g., RTL probes) to adjacent or nearby sequences of a target analyte (e.g., RNA; e.g., mRNA) of interest. In some embodiments, the probe oligonucleotide pairs include sequences that are complementary or substantially complementary to an analyte. For example, in some embodiments, each probe oligonucleotide includes a sequence that is complementary or substantially complementary to an mRNA of interest (e.g., to a portion of the sequence of an mRNA of interest). In some embodiments, each target analyte includes a first target region and a second target region. In some instances, the methods include providing a plurality of first probe oligonucleotides and a plurality of second probe oligonucleotides, wherein a pair of probe oligonucleotides for a target analyte comprises both a first and second probe oligonucleotide. In some instances, a first probe oligonucleotide hybridizes to a first target region of the analyte, and the second probe oligonucleotide hybridizes to a second, adjacent or nearly adjacent target region of the analyte.

In some instances, the probe oligonucleotides are DNA molecules. In some instances, the first probe oligonucleotide is a DNA molecule. In some instances, the second probe oligonucleotide is a DNA molecule. In some instances, the first probe oligonucleotide comprises at least two ribonucleic acid bases at the 3' end. In some instances, the second probe oligonucleotide comprises a phosphorylated nucleotide at the 5' end.

In some embodiments, one of the probe oligonucleotides of the pair of probe oligonucleotides for RTL includes a poly(A) sequence or a complement thereof. In some instances, the poly(A) sequence or a complement thereof is on the 5' end of one of the probe oligonucleotides. In some instances, the poly(A) sequence or a complement thereof is on the 3' end of one of the probe oligonucleotides. In some embodiments, one probe oligonucleotide of the pair of probe oligonucleotides for RTL includes a degenerate or UMI sequence. In some embodiments, the UMI sequence is specific to a particular target or set of targets. In some instances, the UMI sequence or a complement thereof is on the 5' end of one of the probe oligonucleotides. In some instances, the UMI sequence or a complement thereof is on the 3' end of one of the probe oligonucleotides.

In some instances, the first and second target regions of an analyte are directly adjacent to one another. In some embodiments, the complementary sequences to which the first probe oligonucleotide and the second probe oligonucleotide hybridize are 1, 2, 3, 4, 5, 6, 7, 8, 9, 10, about 15, about 20, about 25, about 30, about 35, about 40, about 45, about 50, about 55, about 60, about 65, about 70, about 75, about 80, about 85, about 90, about 95, about 100, about 125, or about 150 nucleotides away from each other. Gaps between the probe oligonucleotides may first be filled prior to ligation, using, for example, dNTPs in combination with a polymerase such as polymerase mu, DNA polymerase, RNA polymerase, reverse transcriptase, VENT polymerase, Taq polymerase, and/or any combinations, derivatives, and variants (e.g., engineered mutants) thereof. In some embodiments, when the first and second probe oligonucleotides are separated from each other by one or more nucleotides, deoxyribonucleotides are used to extend and ligate the first and second probe oligonucleotides.

In some instances, the first probe oligonucleotide and the second probe oligonucleotide hybridize to an analyte on the same transcript. In some instances, the first probe oligonucleotide and the second probe oligonucleotide hybridize to an analyte on the same exon. In some instances, the first probe oligonucleotide and the second probe oligonucleotide hybridize to an analyte on different exons. In some instances, the first probe oligonucleotide and the second probe oligonucleotide hybridize to an analyte that is the result of a translocation event (e.g., in the setting of cancer). The methods provided herein make it possible to identify alternative splicing events, translocation events, and mutations that change the hybridization rate of one or both probe oligonucleotides (e.g., single nucleotide polymorphisms, insertions, deletions, point mutations).

In some embodiments, the first and/or second probe as disclosed herein includes at least two ribonucleic acid bases at the 3' end; a functional sequence; a phosphorylated nucleotide at the 5' end; and/or a capture probe binding domain. In some embodiments, the functional sequence is a primer sequence. The capture probe binding domain is a sequence that is complementary to a particular capture domain present in a capture probe. In some embodiments, the capture probe binding domain includes a poly(A) sequence. In some embodiments, the capture probe binding domain includes a poly-uridine sequence, a poly-thymidine sequence, or a combination thereof. In some embodiments, the capture probe binding domain includes a random sequence (e.g., a random hexamer or octamer). In some embodiments, the capture probe binding domain is complementary to a capture domain in a capture probe that detects a particular target(s) of interest. In some embodiments, a capture probe binding domain blocking moiety that interacts with the capture probe binding domain is provided. In some embodiments, a capture probe binding domain blocking moiety includes a sequence that is complementary or substantially complementary to a capture probe binding domain. In some embodiments, a capture probe binding domain blocking moiety prevents the capture probe binding domain from binding the capture probe when present. In some embodiments, a capture probe binding domain blocking moiety is removed prior to binding the capture probe binding domain (e.g., present in a ligation product) to a capture probe. In some embodiments, a capture probe binding domain blocking moiety comprises a poly-uridine sequence, a poly-thymidine sequence, or a combination thereof.

Hybridization of the probe oligonucleotides to the target analyte can occur at a target having a sequence that is 100% complementary to the probe oligonucleotide(s). In some embodiments, hybridization can occur at a target having a sequence that is at least (e.g. at least about) 80%, at least (e.g. at least about) 85%, at least (e.g. at least about) 90%, at least (e.g. at least about) 95%, at least (e.g. at least about) 96%, at least (e.g. at least about) 97%, at least (e.g. at least about) 98%, or at least (e.g. at least about) 99% complementary to the probe oligonucleotide(s). After hybridization, in some embodiments, the first probe oligonucleotide is extended. After hybridization, in some embodiments, the second probe oligonucleotide is extended. For example, in some instances a first probe oligonucleotide hybridizes to a target sequence upstream of a second oligonucleotide probe, whereas in other instances a first probe oligonucleotide hybridizes to a target sequence downstream of a second probe oligonucleotide.

In some embodiments, methods disclosed herein include a wash step after hybridizing the first and the second probe oligonucleotides. The wash step removes any unbound oligonucleotides and can be performed using any technique known in the art. In some embodiments, a pre-hybridization buffer is used to wash the sample. In some embodiments, a phosphate buffer is used. In some embodiments, multiple wash steps are performed to remove unbound oligonucleotides. For example, it is advantageous to decrease the amount of unhybridized probes present in a biological sample as they may interfere with downstream applications and methods.

In some embodiments, after hybridization of probe oligonucleotides (e.g., first and the second probe oligonucleotides) to the target analyte, the probe oligonucleotides (e.g., the first probe oligonucleotide and the second probe oligonucleotide) are ligated together, creating a single ligation product that is complementary to the target analyte. Ligation can be performed enzymatically or chemically, as described herein. For example, the first and second probe oligonucleotides are hybridized to the first and second target regions of the analyte, and the probe oligonucleotides are subjected to a nucleic acid reaction to ligate them together. For example, the probes may be subjected to an enzymatic ligation reaction using a ligase (e.g., T4 RNA ligase (Rnl2), a SplintR ligase, or a T4 DNA ligase). See, e.g., Zhang L., et al.; Archaeal RNA ligase from thermoccocus kodakarensis for template dependent ligation RNA Biol. 2017; 14(1): 36-44 for a description of KOD ligase. A skilled artisan will understand that various reagents, buffers, cofactors, etc. may be included in a ligation reaction depending on the ligase being used.

In some embodiments, after ligation of the first and second probe oligonucleotides to create a ligation product, the ligation product is released from the analyte. To release the ligation product, an endoribonuclease is used. An endoribonuclease such as RNAse H specifically cleaves RNA in RNA:DNA hybrids. In some embodiments, the ligation product is released enzymatically. In some embodiments, an endoribonuclease is used to release the probe from the analyte. In some embodiments, the endoribonuclease is one or more of RNase H. In some embodiments, the RNase H is RNase H1 or RNase H2.

In some embodiments, after creating a ligation product from the pair of probe oligonucleotides, the biological sample is permeabilized. In some embodiments, permeabilization occurs using a protease. In some embodiments, the protease is an endopeptidase. Endopeptidases that can be used include but are not limited to trypsin, chymotrypsin, elastase, thermolysin, pepsin, clostripan, glutamyl endopeptidase (GluC), ArgC, peptidyl-asp endopeptidase (ApsN), endopeptidase LysC and endopeptidase LysN. In some embodiments, the endopeptidase is pepsin.

In some embodiments, the ligation product (e.g., the analyte derived molecule) includes a capture probe binding domain, which can hybridize to a capture probe (e.g., a capture probe immobilized, directly or indirectly, on a substrate). Methods provided herein include contacting a biological sample with a substrate, wherein the capture probe is affixed to the substrate (e.g., immobilized to the substrate, directly or indirectly). After hybridization of the ligation product to the capture probe, downstream methods as disclosed herein (e.g., RCA) can be performed.

The methods provided herein can be applied to analyte or analyte derived molecules including, without limitation, a ligation product from a templated ligation (RTL) assay, a product of reverse transcription, and an analyte binding moiety barcode.

In some instances, RTL is performed between two oligonucleotides that each are affixed to an analyte binding moiety (i.e., a protein-binding moiety). Generally, the methods of RTL in this setting is as follows. In some embodiments, provided herein is a method of determining a location of at least one analyte in a biological sample including: (a) hybridizing a first analyte-binding moiety to a first analyte in the biological sample, wherein the first analyte-binding moiety is bound to a first oligonucleotide, wherein the first oligonucleotide comprises: (i) a functional sequence; (ii) a first barcode; and (iii) a first bridge sequence; (b) hybridizing a second analyte-binding moiety to a second analyte in the biological sample, wherein the second analyte-binding moiety is bound to a second oligonucleotide; wherein the second oligonucleotide comprises: (i) capture probe binding domain sequence, (ii) a second barcode; and (ii) a second bridge sequence; (c) contacting the biological sample with a third oligonucleotide; (d) hybridizing the third oligonucleotide to the first bridge sequence of the first oligonucleotide and second bridge sequence of the second oligonucleotide; (e) ligating the first oligonucleotide and the second oligonucleotide, creating a ligation product; (f) contacting the biological sample with a substrate, wherein a capture probe is affixed to the substrate, wherein the capture probe comprises a spatial barcode and the capture domain; and (g) allowing the capture probe binding domain sequence of the second oligonucleotide to specifically bind to the capture domain. In some instances, the ligation product is cleaved from the analyte biding moieties.

In some instances, two analytes (e.g., two different proteins) in close proximity in a biological sample are detected by a first analyte-binding moiety and a second analyte-binding moiety, respectively. In some embodiments, a first analyte-binding moiety and/or the second analyte-binding moiety is an analyte capture agent (e.g., any of the exemplary analyte capture agents described herein). In some embodiments, the first analyte-binding moiety and/or the second analyte-binding moiety is a first protein. In some embodiments, the first analyte-binding moiety and/or the second analyte-binding moiety is an antibody. For example, the antibody can include, without limitation, a monoclonal antibody, recombinant antibody, synthetic antibody, a single domain antibody, a single-chain variable fragment (scFv), and or an antigen-binding fragment (Fab). In some embodiments, the first analyte-binding moiety binds to a cell surface analyte (e.g., any of the exemplary cell surface analytes described herein). In some embodiments, binding of the analyte is performed metabolically. In some embodiments, binding of the analyte is performed enzymatically. In some embodiments, the methods include a secondary antibody that binds to a primary antibody, enhancing its detection.

In some embodiments, the first analyte-binding moiety and the second analyte-binding moiety each bind to the same analyte. In some embodiments, the first analyte-binding moiety and/or second analyte-binding moiety each bind to a different analyte. For example, in some embodiments, the first analyte-binding moiety binds to a first polypeptide and the second analyte-binding moiety binds to a second polypeptide.

In some embodiments of any of the methods of determining a location of at least one analyte in a biological sample, a first and/or a second oligonucleotide are bound (e.g., conjugated or otherwise attached using any of the methods described herein) to a first analyte-binding moiety and/or a second analyte-binding moiety, respectively.

In some embodiments of any of the methods of determining a location of at least one analyte in a biological sample as described herein, a second oligonucleotide is bound (e.g., conjugated or otherwise attached using any of the methods described herein) to a second analyte-binding moiety. For example, the second oligonucleotide can be covalently linked to the second analyte-binding moiety. In some embodiments, the second oligonucleotide is bound to the second analyte-binding moiety via its 5' end. In some embodiments, the second oligonucleotide includes a free 3' end. In some embodiments the second oligonucleotide is bound to the second analyte-binding moiety via its 3' end. In some embodiments, the second oligonucleotide includes a free 5' end.

In some embodiments, the oligonucleotides are bound to the first and/or second analyte-binding moieties via a linker (e.g., any of the exemplary linkers described herein). In some embodiments, the linker is a cleavable linker. In some embodiment, the linker is a linker with photo-sensitive chemical bonds (e.g., photo-cleavable linkers). In some embodiments, the linker is a cleavable linker that can undergo induced dissociation.

In some embodiments, the oligonucleotides are bound (e.g., attached via any of the methods described herein) to an analyte-binding domain via a 5' end.

In some embodiments, a barcode is used to identify the analyte-binding moiety to which it is bound. The barcode can be any of the exemplary barcodes described herein. In some embodiments, the first and/or second oligonucleotide include a capture probe binding domain sequence. For example, a capture probe binding domain sequence can be a poly(A) sequence when the capture domain sequence is a poly(T) sequence.

In some embodiments, a third oligonucleotide (e.g., a splint oligonucleotide) hybridizes to both the first and second oligonucleotides and enables ligation of the first oligonucleotide and the second oligonucleotide. In some embodiments, a ligase is used. In some aspects, the ligase includes a DNA ligase. In some aspects, the ligase includes a RNA ligase. In some aspects, the ligase includes T4 DNA ligase. In some embodiments, the ligase is a SplintR ligase.

In some embodiments, a method for identifying a location of an analyte in a biological sample exposed to different permeabilization conditions includes (a) contacting the biological sample with a substrate, wherein the substrate comprises a plurality of capture probes, wherein a capture probe of the plurality of capture probes comprises a capture domain; (b) contacting the biological sample with a first probe oligonucleotide and a second probe oligonucleotide, wherein the first probe oligonucleotide and the second probe oligonucleotide are substantially complementary to adjacent sequences of the analyte, and wherein the second probe oligonucleotide comprises a capture probe-binding domain that is capable of binding to a capture domain of the capture probe; (c) hybridizing the first probe oligonucleotide and the second probe oligonucleotide to adjacent sequences of the analyte; (d) ligating the first probe oligonucleotide and the second probe oligonucleotide, thereby creating a ligation product that is substantially complementary to the analyte; (e) releasing the ligation product from the analyte; (f) hybridizing the capture probe-binding domain of the ligation product to the hybridization domain of the capture probe; (g) hybridizing a padlock oligonucleotide to the ligation product bound to the capture domain (e.g., such that the padlock oligonucleotide is circularized), wherein the padlock oligonucleotide comprises: (i) a first sequence that is substantially complementary to a first portion of the ligation product, (ii) a backbone sequence, and (iii) a second sequence that is substantially complementary to a second portion of the ligation product; and (i) ligating and amplifying the circularized padlock oligonucleotide (e.g., using rolling circle amplification using the circularized padlock oligonucleotide as a template), thereby creating an amplified circularized padlock oligonucleotide, and using the amplified circularized padlock oligonucleotide to identify the location of the analyte in the biological sample.

### (h) Analyte Capture Agents

This disclosure features methods for identifying the location of an analyte (e.g., a protein) molecule in a biological sample using analyte capture agents, rolling circle amplification, and detection probes. In a non-limiting example, a method of identifying the location of analyte (e.g., a protein) molecule in a biological sample where the biological sample is contacted with a substrate comprising capture probes and contacted with a plurality of analyte capture agents that bind to the analyte molecules and can bind to a capture domain on a capture probe on the substrate. A capture agent of the plurality of capture agents include: an analyte binding moiety that binds specifically to an analyte from the biological sample, an analyte binding moiety barcode, and an analyte capture sequence, wherein the analyte capture sequence binds specifically to the capture domain of the capture probe. The analyte binding moiety barcode that is bound to a capture domain of a capture probe is hybridized to a padlock oligonucleotide, the padlock oligonucleotide is circularized, the padlock oligonucleotide is amplified using rolling circle amplification (RCA), and a signal corresponding to the amplified RCA product is detected using a plurality of detection probes (e.g., a detection probe of the plurality of detection probes includes a sequence that is substantially complementary to a sequence of the padlock oligonucleotide and a fluorophore).

In some instances, an analyte capture agent refers to a molecule that interacts with a target analyte and with a capture probe to identify the analyte. In some embodiments, an analyte capture agent includes a label (e.g., fluorescent label). In some embodiments, the analyte capture agent can include an analyte binding moiety and a capture agent barcode domain. An analyte binding moiety is a molecule capable of binding to a specific analyte. In some embodiments, the analyte binding moiety includes an antibody or antibody fragment. In some embodiments, the analyte binding moiety includes a polypeptide and/or an aptamer. In some embodiments, the analyte binding moiety includes a DNA aptamer. In some embodiments, the analyte binding moiety includes a RNA aptamer. In some embodiments, the analyte binding moiety includes an aptamer of mixed natural or unnatural occurring nucleotides (e.g., LNA, PNA). In some embodiments, the analyte is a protein (e.g., a protein on a surface of a cell or an intracellular protein).

A capture agent barcode domain can include an analyte capture sequence which can hybridize to at least a portion or an entirety of a capture domain of a capture probe. In some embodiments, the analyte capture sequence includes a poly (A) tail. In some embodiments, the analyte capture sequence includes a sequence capable of binding a poly (T) domain. In some embodiments, the analyte capture sequence can have a GC content between 1-100% , e.g., 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 35%, 40%, 45%, 50%, 60%, 70%, 80%, etc.). In some embodiments, the analyte capture sequence has a GC content of at least 30%. In some embodiments, one or more pluralities of analyte capture agents can be provided to a biological sample, wherein one plurality of analyte capture agent differs from another plurality of analyte capture agent by the analyte capture sequence. For example, analyte capture sequence A can be correlated with analyte binding moiety A, and analyte capture sequence B can be correlated with analyte binding moiety B. In some embodiments, the two pluralities of analyte capture agents can have the same analyte binding moiety barcode sequence.

In some embodiments, the analyte capture sequence comprises a non-homopolymeric sequence. In some embodiments, the analyte capture sequence is a defined sequence. In such cases, the defined sequence can be substantially complementary to a portion of the capture domain. In some embodiments, the analyte capture sequence and the capture domain are both defined sequences. In some instances, the analyte capture sequence can include a defined sequence that is about 5 to 50 nucleotides in length (e.g., about 5 to 25, about 5 to 20, about 10 to 25, about 10 to 20). In some instances, the analyte capture sequence can include a defined sequence that is about 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 nucleotides long.

In some embodiments, the capture domain includes a poly (T) tail. In some embodiments, the capture domain includes a sequence capable of binding a poly (A) domain. In some embodiments, the capture domain can have a GC content between 1-100% , e.g., 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 35%, 40%, 45%, 50%, 60%, 70%, 80%, etc. In some embodiments, the capture domain has a GC content of at least 30%.

In some embodiments, the capture agent barcode domain includes an analyte binding moiety barcode. The analyte binding moiety barcode refers to a barcode that is associated with or otherwise identifies the analyte binding moiety. In some embodiments, the analyte binding moiety barcode is correlated with the type of analyte binding moiety, such that more than one plurality of analyte capture agents can be provided to a biological sample at one time. For example, analyte binding moiety barcode A can be correlated with analyte binding moiety A, and analyte binding moiety barcode B is correlated with analyte binding moiety B. The two pluralities of analyte capture agents can have the same analyte capture sequence (e.g., poly(A)). In some embodiments, one analyte binding moiety barcode plurality is correlated with one analyte capture sequence plurality. In other embodiments, an analyte binding moiety barcode plurality is not necessarily correlated with an analyte capture sequence plurality.

In some embodiments, a capture agent barcode domain includes optional sequences, such as, without limitation, a PCR handle, a sequencing priming site, a domain for hybridizing to another nucleic acid molecule, and combinations thereof. In some embodiments, the PCR handle is identical on all capture analyte barcode domains. In some embodiments, the PCR handle is included for PCR amplification. In some embodiments, an analyte capture agent includes one or more optional sequences and one or more barcode sequences (e.g., one or more analyte binding moiety barcodes and/or one or more UMIs). In some embodiments, the capture probe capture domain and/or the analyte capture agent include a cleavage domain. In some embodiments, a capture agent barcode domain can be dissociated from the analyte binding moiety by cleaving the analyte binding moiety from the capture agent barcode domain via a cleavage domain in the capture agent barcode domain. Other embodiments of an analyte capture agent useful in spatial protein detection are described herein.

Provided herein are methods for spatially profiling a biological analyte, e.g., any of the analytes as described herein, in a biological sample that use a spatially-tagged analyte capture agent. A biological analyte can be bound by an analyte capture agent at a distinct spatial position on a substrate and detected. The bound biological analyte can then be correlated with a barcode of the capture probe at a distinct spatial position of the substrate. In some embodiments, these methods can include spatially profiling the biological analyte from one or more of: an intracellular region of a cell in a biological sample, a cell surface region of a cell in a biological sample, a particular type of cell in a biological sample, and a region of interest of a biological sample.

### (i) Blocking probes

In some embodiments, an analyte capture sequence of a capture agent barcode domain is blocked prior to adding the analyte capture agent to a biological sample. In some embodiments, an analyte capture sequence of a capture agent barcode domain is blocked prior to adding the analyte capture agent to a capture probe array. In some embodiments, blocking probes are added to blocking buffer or other solutions applied in an IHC and/or IF protocol. In some embodiments, a blocking probe is used to block or modify the free 3' end of the capture agent barcode domain. In some embodiments, a blocking probe is used to block or modify the free 3' end of the analyte capture sequence of the capture agent barcode domain. In some embodiments, a blocking probe can be hybridized to the analyte capture sequence of a capture agent barcode domain to mask the free 3' end of the capture agent barcode domain. In some embodiments, a blocking probe can be a hairpin probe or partially double stranded probe. In some embodiments, the free 3' end of the analyte capture sequence of the capture agent barcode domain can be blocked by chemical modification, e.g., addition of an azidomethyl group as a chemically reversible capping moiety such that the capture probes do not include a free 3' end. Blocking or modifying the capture agent barcode domains, particularly at the free 3' end of the capture agent barcode domain, prior to contacting the analyte capture agents with the substrate, prevents binding of the analyte capture sequence to capture probe capture domain (e.g., prevents the binding of an analyte capture sequence poly(A) tail to a poly(T) capture domain).

In some embodiments, a blocking probe is used to block or modify the free 3' end of a capture probe. In some embodiments, a blocking probe is used to block or modify the free 3' end of a capture probe capture domain. In some embodiments, the analyte capture sequence is blocked prior to adding the analyte capture agent to a capture probe array. In some embodiments, blocking probes are added to blocking buffer or other solutions applied in an IHC and/or IF protocol. In some embodiments, a blocking probe can be hybridized to the capture domain to mask the free 3' end of the capture domain. In some embodiments, a blocking probe can be a hairpin probe or partially double stranded probe. In some embodiments, the free 3' end of the capture domain can be blocked by chemical modification, e.g., addition of an azidomethyl group as a chemically reversible capping moiety such that the capture probes do not include a free 3' end. Blocking or modifying the capture domains, particularly at the free 3' end of the capture domain, prior to contacting the analyte capture agents with the capture probe array, prevents binding of the analyte capture sequence to capture probe capture domain (e.g., prevents the binding of an analyte capture sequence poly(A) tail to a poly(T) capture domain).

In some embodiments, the blocking probes can be reversibly removed. For example, blocking probes can be applied to block the free 3' end of either or both the capture agent barcode domain and/or the capture probes. Blocking interaction between the analyte capture agent and the capture probe array can reduce non-specific background staining in IHC and/or IF applications. After the analyte binding agents are bound to the target analyte, the blocking probes can be removed from the 3' end of the capture agent barcode domain and/or the capture probe, and the analyte-bound analyte binding agents can migrate to and become bound by the capture probe array. In some embodiments, the removal includes denaturing the blocking probe from the analyte binding moiety barcode and/or capture probe. In some embodiments, the removal includes removing a chemically reversible capping moiety. In some embodiments, the removal includes digesting the blocking probe with an RNAse (e.g., RNAse H).

In some embodiments, the blocking probes are oligo (dT) blocking probes. In some embodiments, the oligo (dT) blocking probes can have a length of 15-30 nucleotides. In some embodiments, the oligo (dT) blocking probes can have a length of 10-50 nucleotides, e.g., 10-50, 10-45, 10-40, 10-35, 10-30, 10-25, 10-20, 10-15, 15-50, 15-45, 15-40, 15-35, 15-30, 15-25, 15-20, 20-50, 20-45, 20-40, 20-35, 20-30, 20-25, 25-50, 25-45, 25-40, 25-35, 25-30, 30-50, 30-45, 30-40, 30-35, 35-50, 35-45, 35-40, 40-50, 40-45, or 45-50 nucleotides. In some embodiments, the analyte capture agents can be blocked at different temperatures (e.g., 4°C and 37°C). In some embodiments, the analyte capture agents can be blocked from binding to the capture probes more effectively at lower temperatures when using shorter blocking probes.

### (ii) Spatially-tagged capture agents

A "spatially-tagged analyte capture agent" can be a molecule that interacts with an analyte (e.g., an analyte in a sample) and with a capture probe to identify the spatial location of the analyte. In some embodiments, a spatially-tagged analyte capture agent can be an analyte capture agent with an extended capture agent barcode domain that includes a sequence complementary to a spatial barcode of a capture probe. In some embodiments, an analyte capture agent is introduced to an analyte and a capture probe at the same time. In some embodiments, an analyte capture agent is introduced to an analyte and a capture probe at different times. In some embodiments, the spatially-tagged analyte capture agent is denatured from the capture probe before the biological sample is introduced. In some embodiments, the spatially-tagged analyte capture agent binds to a biological analyte within a biological sample before the spatially-tagged analyte capture agent is denatured from the capture probe. In some embodiments, the capture probe is cleaved from the substrate while attached to the spatially-tagged analyte capture agent. In some embodiments, once the capture domain of the capture probe is bound to the analyte binding moiety barcode, the analyte capture sequence is extended towards the 3' tail to include a sequence that is complementary to the sequence of the capture probe spatial barcode (e.g., producing a spatially-tagged analyte capture agent).

For example, an analyte capture agent can be introduced to a biological sample, wherein the analyte binding moiety binds to a target analyte, and then the biological sample can be treated to release the analyte-bound analyte capture agent from the sample. The analyte-bound analyte capture agent can then migrate and bind to a capture probe capture domain.

In another example, an analyte capture agent can be hybridized to a capture probe capture domain on a capture probe array, wherein the capture agent barcode domain is extended to include a sequence complementary to the spatial barcode of the capture probe. A biological sample can be contacted with the analyte capture agent modified capture probe array. Analytes from the biological sample can be released from the sample, migrated to the analyte capture agent modified capture probe array, and captured by an analyte binding moiety.

In some embodiments, a spatially-tagged analyte capture agent can attach to a surface of a cell through a combination of lipophilic and covalent attachment. For example, a spatially-tagged analyte capture agent can include an oligonucleotide attached to a lipid to target the oligonucleotide to a cell membrane, and an amine group that can be covalently linked to a cell surface protein(s) via any number of chemistries described herein. **In** these embodiments, the lipid can increase the surface concentration of the oligonucleotide and can promote the covalent reaction.

**In** a non-limiting example, a method for identifying a location of an analyte in a biological sample includes (a) contacting the biological sample with a substrate, where the substrate includes a plurality of capture probes, where a capture probe of the plurality of capture probes includes a capture domain; and (b) contacting the biological sample with a plurality of analyte capture agents, where an analyte capture agent of the plurality of analyte capture agents includes:(i) an analyte binding moiety that binds specifically to an analyte from the biological sample,(ii) an analyte binding moiety barcode, and (iii) an analyte capture sequence, where the analyte capture sequence binds specifically to the capture domain of the capture probe; (c) binding the analyte from the biological sample to the analyte binding moiety; (d) hybridizing the analyte capture sequence to the capture domain of the capture probes; (e) hybridizing a padlock oligonucleotide to the analyte binding moiety barcode bound to the capture domain of the capture probes, where the padlock oligonucleotide includes: (i) a first sequence that is substantially complementary to a first portion of the analyte binding moiety barcode, (ii) a backbone sequence, and (iii) a second sequence that is substantially complementary to a second portion of the analyte binding moiety barcode, where the first sequence and the second sequence are substantially complementary to adjacent sequences of the analyte binding moiety barcode; (f) ligating the first sequence of the padlock oligonucleotide to the second sequence of the padlock oligonucleotide, thereby creating a circularized padlock oligonucleotide; and (g) amplifying the circularized padlock oligonucleotide using rolling circle amplification, thereby creating an amplified circularized padlock oligonucleotide, and using the amplified circularized padlock oligonucleotide to identify the location of the analyte in the biological sample. **In** some embodiments, the method includes identifying the location of the analyte in the biological sample includes detecting a signal corresponding to the amplified padlock oligonucleotide.

### (i) Tethering the Amplification Primer to the Capture Probe

Disclosed herein is a method for identifying a location of an analyte in a biological sample where an amplified circularized padlock oligonucleotide is tethered to the capture probe. Tethering the amplified circularized padlock oligonucleotide to the capture probe retains the spatial location by attaching the amplified circularized padlock oligonucleotide at the spatial location of the capture probe. When combined with detection and/or sequencing, tethering removes the need for a capture probe to include a spatial barcode because the sequence of the analyte can be determined at the location on the substrate that corresponds to the location of the analyte in the biological sample.

**In** some embodiments, the sequence of the amplification primer can be substantially complementary a portion of the analyte or analyte derived molecule that is directly adjacent to the 3' end of the capture probe. **In** such instances, the 3' end of the capture probe can be ligated (e.g., ligated using any of the exemplary ligases described herein) to the 5' end of the amplification primer. Prior to ligating an amplification primer to the capture probe, any existing 3' blocking moiety is removed and/or the 3'end of the capture probe is restored as described herein.

**In** some embodiments where the amplification primer and the capture probe are not directly adjacent and the capture probe includes a 3' blocking moiety, tethering the capture probe to the amplification primer includes: generating a free 3' OH on the capture probe; extending the capture probe to generate an extended capture probe; and ligating the extended capture probe to the primer. Extending the capture probe can be performed using a polymerase (e.g., any of the polymerase (e.g., DNA polymerase) described herein). In some embodiments, generating a free `3 OH on the capture probe comprises: removing a blocking moiety from the 3' end of the capture probe; or adding additional nucleic acids to the 3' end of the capture probe. In some embodiments, removing the blocking moiety from the 3' end of the capture probe comprises cleaving the oligonucleotide with an endonuclease.

### (j) Transfer to a Gene Expression Slide Based on the Analyzed Signal

This disclosure features a method for identifying and transferring a subset of the biological sample to a second substrate for spatial analysis based in part on detecting a signal corresponding to the amplified circularized padlock oligonucleotide on the substrate. **In** such cases, detection of the signal corresponding to the amplified circularized padlock oligonucleotide identifies the subset (e.g., one or more regions of interest) of the biological sample that is transferred to a second substrate for spatial analysis.

**In** some embodiments, the disclosure provides a method for identifying, based on the detected signal, one or more regions of interest corresponding to a subset of the biological sample; and transferring, from a portion of the first substrate that corresponds to the region of interest of the biological sample, the analyte or the analyte derived molecule to a second substrate, wherein the second substrate includes a plurality of capture probes, where a capture probe of the plurality of capture probes includes a spatial barcode and a capture domain. For example, when contacted with the first substrate under conditions that allow for transfer, the capture domain of the capture probe on the second substrate can hybridize to an analyte, an analyte derived molecule, and/or an extended oligonucleotide from the first substrate.

In some embodiments, the capture domain of the capture probe on the second substrate includes a sequence that is substantially complementary to the analyte, the analyte derived molecule and/or the extended capture probe. For example, the capture domain includes a sequence that is substantially complementary to at least a portion of the extended capture probe on the first substrate. In another example, the capture domain includes a sequence that is substantially complementary to a homopolymeric sequence of the analyte, analyte derive molecule and/or the extended capture probe. In such cases, the capture domain includes at least one of a poly(T) sequence, a poly(A) sequence, a poly(C) sequence, or a poly(G) sequence. In some embodiments, the capture domain includes a sequence that is substantially complementary to the reverse complement of a template switch oligonucleotide (TSO). For example, the capture domain includes a sequence that is substantially similar to a template switch oligonucleotide primer (e.g., the TSO primer includes a sequence that is substantially complementary to the reverse complement of a TSO).

In some embodiments, the method includes transferring the analyte, analyte derived molecule, or extended capture probe from the first substrate to the second substrate includes contacting the first substrate and the second substrate. In some embodiments, the method includes aligning the first substrate and second substrate prior to transferring the analyte or analyte molecule.

In some embodiments, transferring the analyte or analyte derived molecule includes: hybridizing the analyte or analyte derived molecule to a capture probe on the second substrate; aligning the first substrate and second substrate prior to transferring the analyte or analyte molecule; contacting the first substrate and the second substrate; cleaving the analyte or analyte derived molecule from the first substrate; and hybridizing the analyte or analyte derived molecule to the capture probe on the second substrate.

In some embodiments, the method further includes extending the capture probe on the first substrate and transferring the extended capture probe to the second substrate. In such cases, the extended capture probe on the first substrate is transferred from the first substrate to the second substrate. In some embodiments, extending the capture probe (i.e., capture probe on the first substrate) includes using the analyte or analyte derived molecule as a template to generate an extended capture probe. In some embodiments, extending the capture probe on the first substrate includes performing a nucleic acid extension reaction. In some embodiments, extending the capture probe on the first substrate includes reverse transcribing the analyte or analyte derived molecule or complementary sequence thereof. In some embodiments, extending the capture probe includes generating a sequence that is complementary to a portion of the analyte or analyte derived molecule. In some embodiments, extending the capture probe includes attaching a template switching oligonucleotide to the analyte or analyte derived molecule. In some embodiments, reverse transcribing the analyte or analyte derived molecule generates a reverse complement of a template switching oligonucleotide. In some embodiments, the method further includes generating a second strand cDNA, wherein the second strand cDNA includes a sequence complementary to the analyte or analyte derived molecule and/or extended capture probe. In some embodiments, the second strand cDNA includes annealing a template switching oligonucleotide primer to a reverse complement of the template switching oligonucleotide. In some embodiments, including generating the second strand cDNA using a polymerase. In some embodiments, the polymerase is a DNA polymerase (e.g., any of the exemplary DNA polymerases described herein or known in the art).

In some embodiments, the methods include transferring the analyte, analyte derived molecule, and/or the extended capture probe includes migrating the analyte or analyte derived molecule from the first substrate to the second substrate using methods selected from the group consisting of electrophoresis, applying a chemical gradient, applying a pressure gradient, directing a fluid flow, and/or applying a magnetic field.

### (k) Compositions and Kits

In some instances, disclosed herein are compositions and systems that are used to carry out the methods described herein. In some embodiments, the kit includes one or more padlock oligonucleotides and a ligase (e.g., a T4 DNA ligase (Rnl2), a SplintR ligase, a single stranded DNA ligase, or a T4 DNA ligase). In some embodiments, the kit further includes one or more primers (e.g., two or more primers, three or more primers, four or more primers, five or more primers, six or more primers, seven or more primers, eight or more primers, nine or more primers, or ten or more primers) and a polymerase (e.g., a Phi29 DNA polymerase or other strand displacing polymerase).

In some embodiments, the kit further includes a substrate including a plurality of capture probes, where a capture probe of the plurality of capture probes includes a capture domain, wherein the analyte is capable of hybridizing to the capture domain. In some embodiments, the kit includes a substrate including a plurality of capture probes, where a capture probe of the plurality of capture probes includes a capture domain and a spatial barcode. It is appreciated that the kit can include any of the elements of the substrate, array, or capture probes as described herein.

In some embodiments, the kit further includes a plurality of detection probes, wherein a detection probe from the plurality of detection probes comprises a sequence that is substantially complementary to a sequence of the padlock oligonucleotide and a detectable label (e.g., any of the exemplary detectable labels described herein (e.g., a fluorophore)).

In some embodiments, the kit includes a first probe oligonucleotide and a second probe oligonucleotide. In some embodiments, the first probe oligonucleotide and the second probe oligonucleotide in the kit are substantially complementary to adjacent sequences of an analyte. In some instances, the kit includes multiple sets of probe oligonucleotides (e.g., a set includes a first probe oligonucleotide and a second probe oligonucleotide), allowing for detection of multiple analytes using one kit. For example, in some instances, the kit includes at least 2 sets, at least 3 sets, at least 4 sets, at least 5 sets, at least 6 sets, at least 7 sets, at least 8 sets, at least 9 sets, or at least 10 sets, or more sets of probe oligonucleotides.

In some embodiments, a kit used to carry out the methods described herein includes:
(a) one or more padlock oligonucleotides and a ligase; (b) one or more primers and a polymerase;
(c) a substrate including a plurality of capture probes, wherein a capture probe of the plurality of capture probes includes a capture domain; and (d) instructions for performing the method of any one of the preceding claims.

In some embodiments, a kit used to carry out the methods described herein includes: (a) one or more padlock oligonucleotides and a ligase; (b) one or more primers and a polymerase; (c) a substrate including a plurality of capture probes, wherein a capture probe of the plurality of capture probes includes a capture domain; (d) one or more fluorescent dyes and/or one or more detection probes; and (e) instructions for performing the method of any one of the preceding claims.

In some embodiments, a kit used to carry out the methods described herein includes:
(a) one or more padlock oligonucleotides and a ligase; (b) one or more primers and a polymerase;
(c) a substrate including a plurality of capture probes, wherein a capture probe of the plurality of capture probes includes a capture domain; (d) one or more fluorescent dyes and/or one or more detection probes; (e) a uracil-DNA glycosylase enzyme; and (f) instructions for performing the method of any one of the preceding claims.

In some embodiments, a kit used to carry out the methods described herein includes: (a) a padlock oligonucleotide and a ligase; (b) one or more primers and a polymerase; (c) a substrate comprising a plurality of capture probes, wherein an capture probe of the plurality of capture probes comprises a capture domain; (d) a second substrate including a plurality of capture probes, wherein a capture probe of the plurality of capture probes includes a spatial barcode and a capture domain; and (e) instructions for performing the method of any one of the preceding claims. In some embodiments, the kits does not include a second substrate.

In some embodiments, a kit used to carry out the methods described herein includes: (a) a first probe oligonucleotide and a second probe oligonucleotide, wherein the first probe oligonucleotide and the second probe oligonucleotide are substantially complementary to adjacent sequences of the analyte, and wherein the second probe oligonucleotide includes a hybridization-binding domain; (b) a padlock oligonucleotide and a ligase; (c) one or more amplification primers and a polymerase; (d) a substrate comprising a plurality of capture probes, wherein a capture probe of the plurality of capture probes comprises a capture domain; (e) a second substrate including a plurality of capture probes, wherein a capture probe of the plurality of capture probes includes a spatial barcode and a capture domain; and (f) instructions for performing the method of any one of the preceding claims. In some embodiments, the kit does not include a second substrate.

### EXAMPLES

### EXAMPLE 1 - Method for identifying the location of mRNA molecules using RCA and digestion

This example provides an exemplary method of identifying the location of an mRNA molecule in a biological sample where the mRNA molecule is bound to a capture domain of a capture probe and is hybridized to a padlock oligonucleotide, an amplification primer is tethered to a capture probe, the padlock oligonucleotide is amplified using rolling circle amplification (RCA), and a signal corresponding to the amplified RCA product is detected using detection probes.

In a non-limiting example, a padlock oligonucleotide that includes a first sequence, a backbone sequence, and a second sequence is hybridized to an analyte molecule (e.g., an mRNA molecule) bound to the capture domain of a capture probe on a substrate. An amplification primer is tethered to the capture probe in order to retain the spatial location of any subsequent amplification product. The padlock oligonucleotide is ligated to create a circularized padlock oligonucleotide and amplified using RCA. The amplified circularized padlock oligonucleotide is contacted with a plurality of detection probes. The signal from the detection probes is detected and used to determine and correlate the location of the mRNA molecule in the biological sample. In an alternative embodiment, the sequence of the amplified circularized padlock oligonucleotide is determined using *in situ* sequencing.

A sample (e.g., an FFPE sample) is decrosslinked to remove formaldehyde crosslinks within the sample thereby releasing the mRNA molecule. Briefly, the tissue samples are incubated with an HCl solution for 1 minute, repeated twice for a total of 3 minutes. Following HCl incubations, the tissue sections are incubated at 70°C for 1 hour in TE pH 9.0. TE is removed and the tissues are incubated in 1xPBS-Tween for 15 minutes.

As shown in **FIGs. 2A-2B****,** a biological sample **201** is contacted with a substrate **202** including a plurality of capture probes **203,** where each capture probe includes a capture domain (in this example, a polyT sequence) and the capture probe further includes a blocking moiety (e.g., blocking group) **204** on the 3' end. The biological sample is permeabilized and the mRNA molecule is released from the biological sample. In particular, after 30 minutes, the tissues are washed and permeabilized by adding 1.25mg/ml Proteinase K, incubated at 37°C for at least 5 minutes and then are washed to remove the protease. The released mRNA molecules 205 are allowed to hybridize to the capture domain on the capture probe **203** immobilized on the spatial array via the polyA tail on the 3' end of the mRNA molecule. Excess mRNA molecules not bound to a capture probe can be washed off.

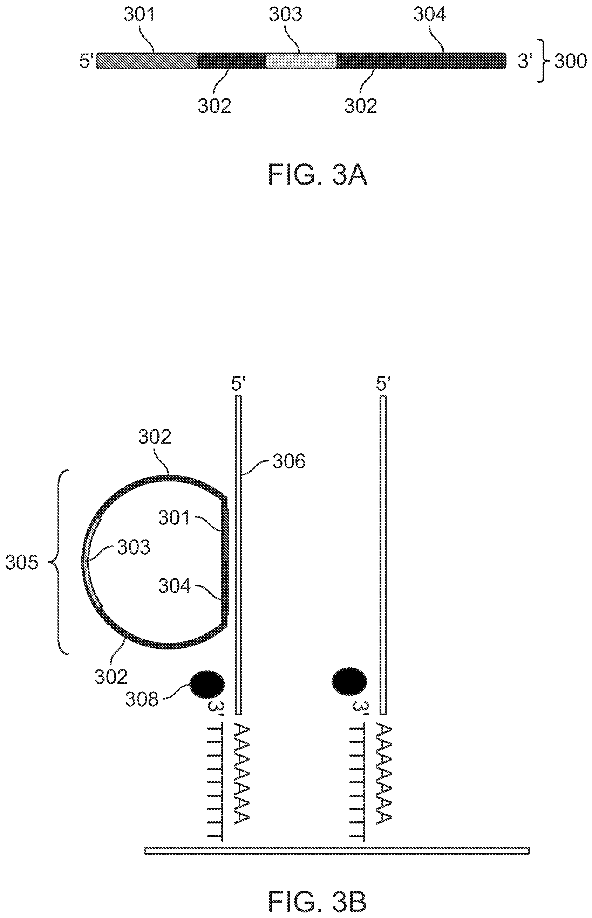

The mRNA bound to the capture probe is contacted with a padlock oligonucleotide under conditions that allow the padlock oligonucleotide to hybridize to the mRNA molecule. As shown in **FIG. 3A****,** a padlock oligonucleotide **300** includes a first sequence **301** that is substantially complementary to a first portion of the mRNA molecule **306,** a backbone sequence **302** that includes a barcode sequence **303,** and a second sequence **304** that is substantially complementary to a second portion of the mRNA molecule. The first sequence and the second sequence are adjacent when hybridized to the mRNA molecule. As a result, as shown in **FIG. 3B****,** the second sequence **304** can be ligated to the first sequence **301,** thereby creating a circularized padlock oligonucleotide **305.**

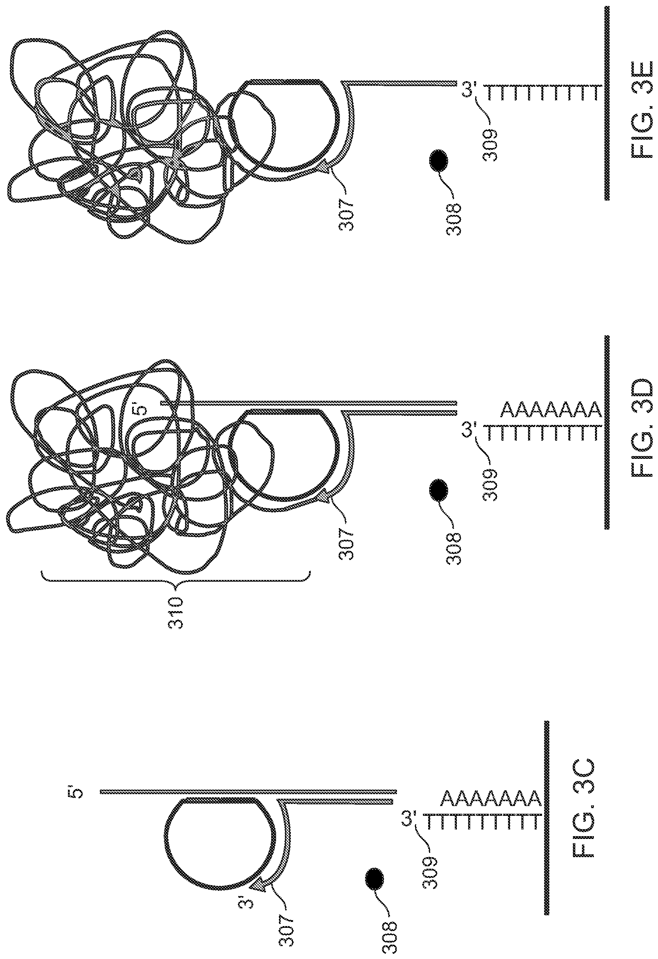

The amplification primer 307 includes a sequence that is substantially complementary to the circularized padlock oligonucleotide and a sequence that is substantially complementary to the mRNA molecule. In some embodiments, as shown in **FIG. 3C****,** the blocking moiety **308** is removed from the capture probe and the amplification primer is ligated to the free 3' end **(309)** of the capture probe. Rolling circle amplification (RCA), as shown in **FIG. 3D****,** is used to amplify the circularized padlock oligonucleotide **305** using, for example, a Phi29 DNA polymerase. **FIG. 3E** shows the release and removal of the captured analyte and the amplified circularized padlock oligonucleotide **310** tethered to the capture probe where the continuous single-stranded copies produced by RCA remains associated with the spatial location on the substrate via the tether to the capture probe.

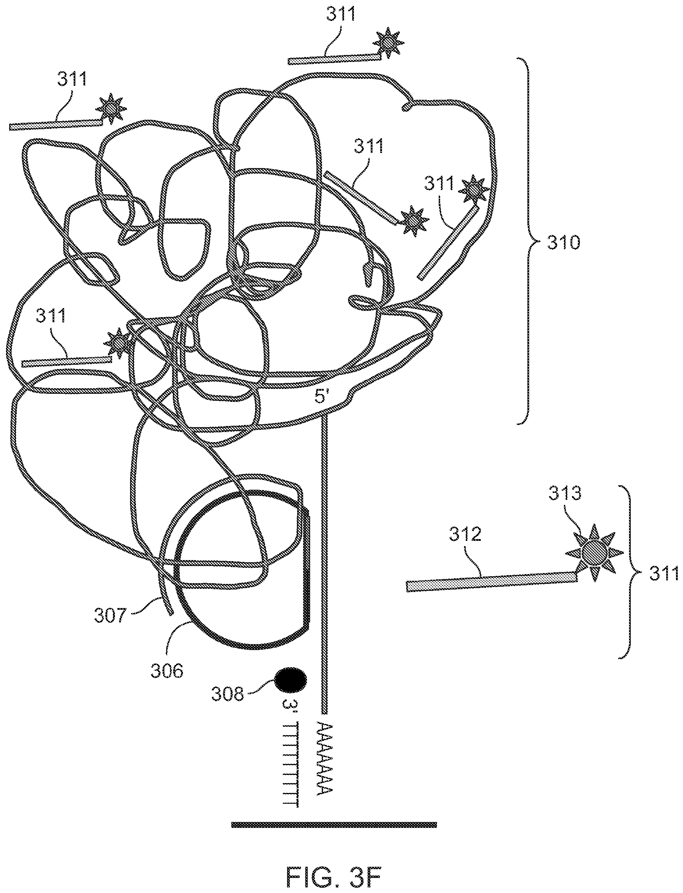

In other embodiments, as shown in **FIG. 3F****,** the blocking moiety **308** is not removed from the capture probe, and the amplification primer **307** is hybridized to the circularized padlock oligonucleotide **305** itself. RCA is used to amplify the circularized padlock oligonucleotide **305.** The continuous single-stranded copies (e.g., amplified circularized padlock oligonucleotide) of the circularized padlock oligonucleotide **310** produced by RCA are either (1) contacted with a plurality of detection probes **311,** where a detection probe **311** includes a sequence **312** that is substantially complementary to a sequence of the padlock oligonucleotide and a fluorophore **313** or (2) subjected to *in situ* sequencing to determine the location of the RNA molecule in the biological sample. *In situ* sequence can be performed as described in U.S. Patent No. 8,551,710, which is incorporated by reference in its entirety.

### EXAMPLE 2 - Digestion of the Amplified Padlock Oligonucleotide and a second RCA reaction

This example provides an exemplary method of identifying the location of a first mRNA molecule in a biological sample and a second mRNA molecule where each of the first and second mRNA molecules are bound (e.g., hybridized) to a capture domain of a capture probe and hybridized to a padlock oligonucleotide, the padlock oligonucleotide is amplified using rolling circle amplification (RCA), a signal corresponding to the amplified RCA product is detected using detection probes or by practicing *in situ* sequencing, the amplified circularized padlock oligonucleotide is digested, a second round of RCA, including a second padlock oligonucleotide, is performed, and a signal corresponding to the second RCA reaction is detected using detection probes or by practicing *in situ* sequencing.

In a non-limiting example, a padlock oligonucleotide that includes a first sequence, a backbone sequence, and a second sequence is bound (e.g., hybridized) to an analyte molecule (e.g., an mRNA molecule) hybridized to the capture domain of a capture probe on a substrate. The padlock oligonucleotide is ligated to create a circularized padlock oligonucleotide and amplified using RCA. The amplified circularized padlock oligonucleotide is contacted with a plurality of detection probes and a signal is detected or the sequence is determined using *in situ* sequencing. The amplified circularized padlock oligonucleotide is digested using uracil-DNA glycosylase. A second padlock oligonucleotide and a second amplification primer are hybridized and a second RCA reaction produces a second amplified circularized padlock oligonucleotide that is detectable using detection probes or *in situ* sequencing.

A sample (e.g., an FFPE sample) is decrosslinked to remove formaldehyde crosslinks within the sample thereby releasing the mRNA molecule. Briefly, the tissue samples are incubated with an HCl solution for 1 minute, repeated twice for a total of 3 minutes. Following HCl incubations, the tissue sections are incubated at 70°C for 1 hour in TE pH 9.0. TE is removed and the tissues are incubated in 1xPBS-Tween for 15 minutes.

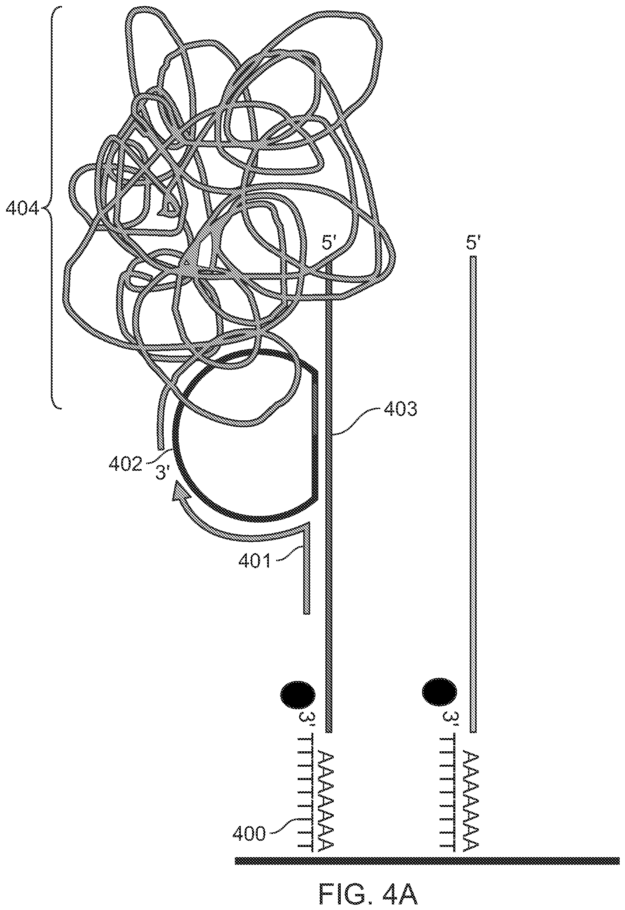

As described in Example 1 and shown in **FIG. 4A**, a first RCA reaction produces continuous single-stranded copies (e.g., amplified first circularized padlock oligonucleotide) of the first circularized padlock oligonucleotide produced by RCA. Briefly, a first mRNA molecule **403** bound to a first capture probe **400** is contacted with a first padlock oligonucleotide **402** under conditions that allow the first padlock oligonucleotide **402** to hybridize to the first mRNA molecule **403.** The first RCA reaction includes a first amplification primer **401** that includes a sequence substantially complementary to the first padlock oligonucleotide **402** and a sequence substantially complementary to the first analyte **403.** The first amplification primer hybridizes to the first padlock oligonucleotide **402** and the first analyte **403.** The first padlock oligonucleotide is circularized (e.g., ligated) and amplified to create an amplified first circularized padlock oligonucleotide **404.** A location of the first amplified circularized padlock oligonucleotide **404** is detected using either detection probes or *in situ* sequencing for identifying the location of the first mRNA molecule in the biological sample.

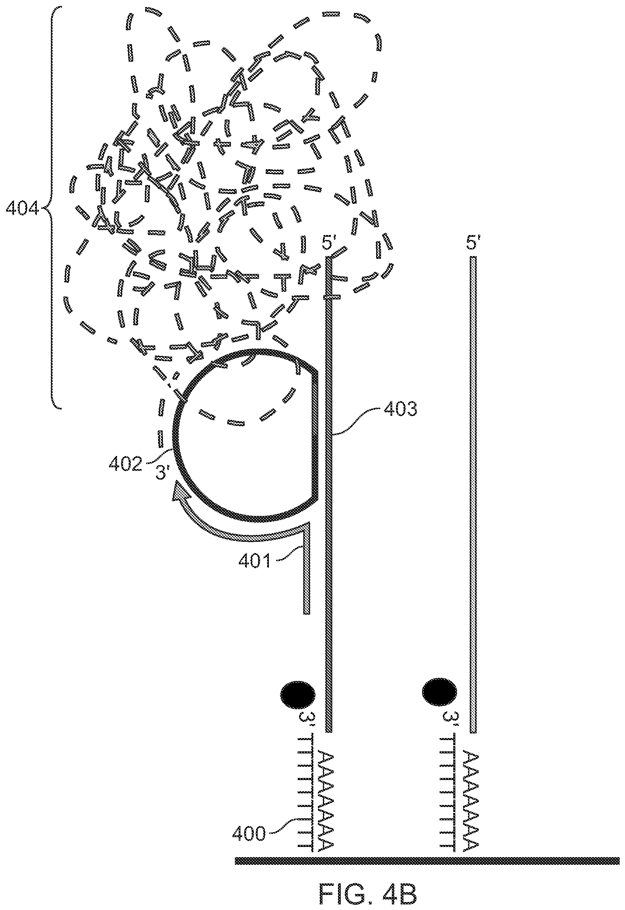

The amplified first circularized padlock oligonucleotide is digested using uracil-DNA glycosylase **(****FIG. 4B****).** The digested amplified first circularized padlock oligonucleotide can be removed with a washing step.

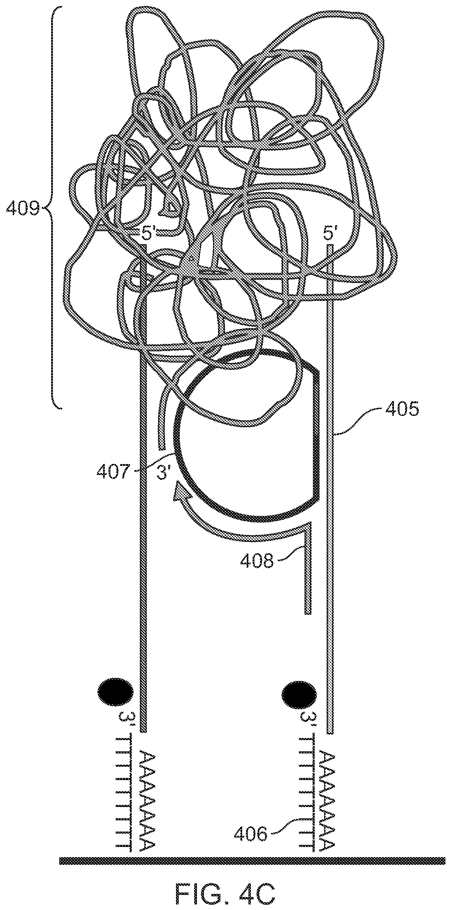

As shown in **FIG. 4C****,** a second mRNA molecule **405** bound to a second capture probe **406** is contacted with a second padlock oligonucleotide **407** under conditions that allow the second padlock oligonucleotide **407** to hybridize to the second mRNA molecule **405.** A second padlock oligonucleotide includes a third sequence that is substantially complementary to a first portion of the second mRNA molecule **405,** a second backbone sequence that includes a second barcode sequence, and a fourth sequence that is substantially complementary to a second portion of the second mRNA molecule **405.** The first sequence and the second sequence are adjacent when hybridized to the mRNA molecule. As a result, the second sequence can be ligated to the first sequence, thereby creating a circularized padlock oligonucleotide. The second padlock oligonucleotide is circularized (e.g., ligated).

A second RCA reaction produces continuous single-stranded copies of the second circularized padlock oligonucleotide thereby producing amplified second circularized padlock oligonucleotides **409.** The second RCA reaction includes a second amplification primer **408** that includes a sequence substantially complementary to the second padlock oligonucleotide **407** and a sequence substantially complementary to the second mRNA molecule **405.** The second amplification primer **408** hybridizes to the second padlock oligonucleotide **407** and the second RNA molecule **405.**

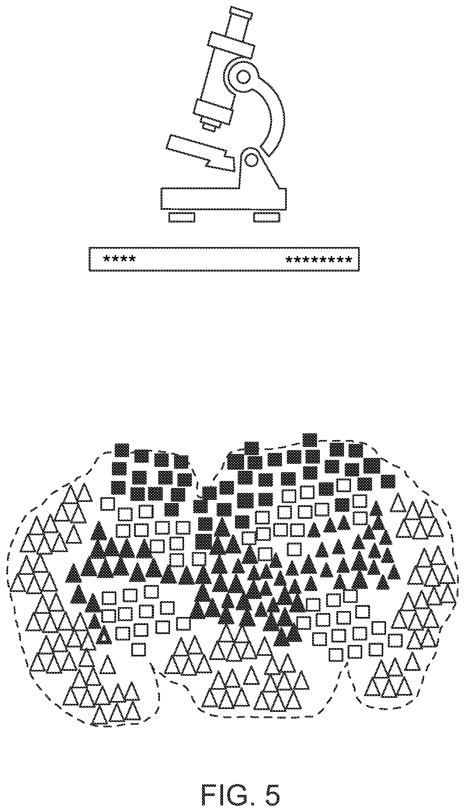

The amplified second circularized padlock oligonucleotide **409** produced by the second RCA reaction is either (1) contacted with a plurality of detection probes, where a detection probe of the plurality of detection probes include a sequence that is substantially complementary to a sequence of the second padlock oligonucleotide and a fluorophore or (2) subjected to *in situ* sequencing to determine the location of the second RNA molecule in the biological sample. **FIG. 5** shows the detected signals from a plurality of amplified circularized padlock oligonucleotides on the substrate, each shape (e.g., solid square, empty square, solid triangle, and empty triangle) representing a different analyte or analyte derived molecule.

### EXAMPLE 3 - Method for identifying a location of an RTL ligation product using RCA and detection probes

This example provides an exemplary method of identifying the location of an analyte (e.g., a ligation product) in a biological sample where a templated ligation (RTL) assay creates a ligation product that serves as a proxy for the target analyte, the ligation product is hybridized to the capture domain of a capture probe, a padlock oligonucleotide is hybridized to the ligation product, the padlock oligonucleotide is amplified using rolling circle amplification (RCA), and a signal corresponding to the amplified RCA product is detected using detection probes. The detection of the analyte can be correlated to the success of, for example, optimization of permeabilization conditions for a biological sample. For instance, optimization can include varying the type of enzyme used to permeabilize a sample; enzyme concentration; length of time to permeabilize a sample; modulating temperature, pH, and/or light exposure; an ion or salt concentration, and any combinations thereof.

In a non-limiting example, a padlock oligonucleotide that includes a first sequence, a backbone sequence, and a second sequence is hybridized to an analyte molecule (e.g., a ligation product from an RLT workflow) bound to the capture domain of a capture probe on a substrate. The padlock oligonucleotide is ligated to create a circularized padlock oligonucleotide and amplified using RCA. The amplified circularized padlock oligonucleotide is contacted with a plurality of detection probes. The signal from the detection probes is detected and used to determine and correlate the location of the analyte associated with the ligation product in the biological sample.

A sample (e.g., an FFPE sample) is decrosslinked to remove formaldehyde crosslinks within the sample thereby releasing the analytes for RNA templated ligation. Briefly, the tissue samples are incubated with an HCl solution for 1 minute, repeated twice for a total of 3 minutes. Following HCl incubations, the tissue sections are incubated at 70°C for 1 hour in TE pH 9.0. TE is removed and the tissues are incubation in 1xPBS-Tween for 15 minutes.

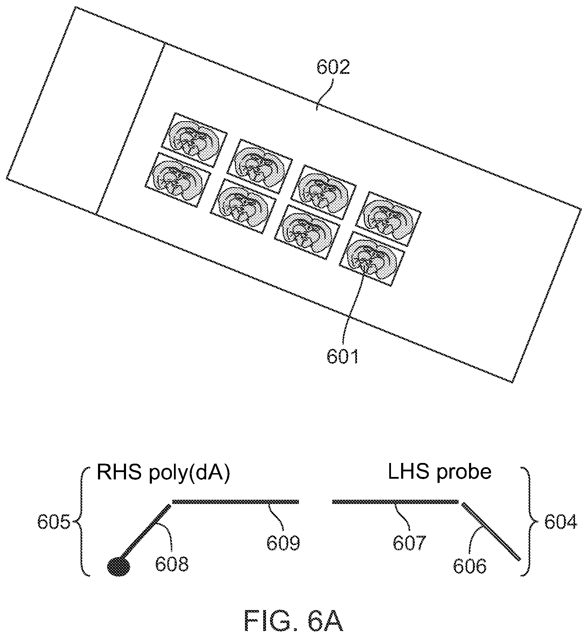

RTL probe pairs are designed to hybridize to adjacent sequences of an analyte of interest. Without limitation, one or more RTL probes can be designed to hybridize to a plurality of (e.g. two or more), or all, analytes in a genome (e.g., a human genome). As seen in **FIG. 6A****,** one probe oligonucleotide **604** (e.g., the LHS probe or the 3' probe) comprises a non-target functional sequence at its 5' end while the other probe oligonucleotide **605** (e.g., the RHS probe or the 5' probe) comprise a non-target poly A sequence at its 3' end.

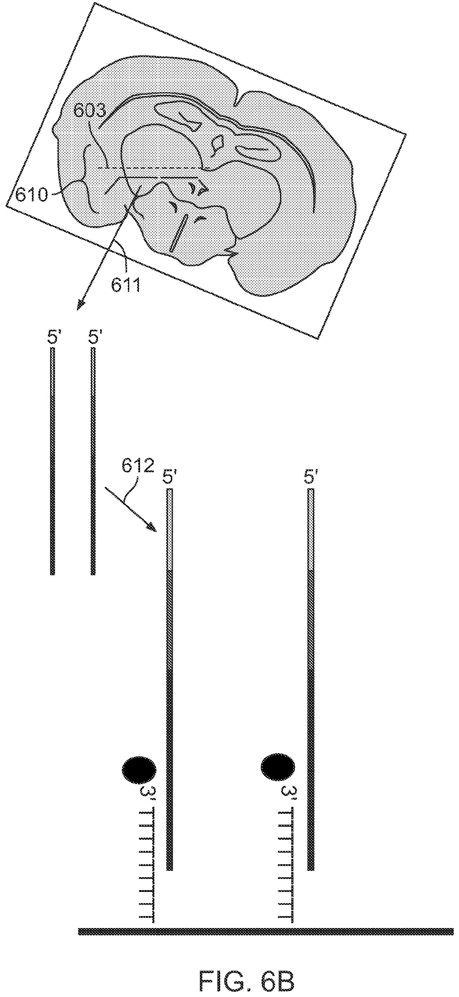

As shown in **FIGs. 6A-6B****,** a biological sample **601** is contacted with a substrate **602** including a plurality of capture probes, where each capture probe includes a capture domain and the capture probe includes a blocking moiety on the 3' end. An mRNA molecule **603** is contacted with the first probe oligonucleotide (LHS probe) **604** and the second probe oligonucleotide (RHS poly (dA)) **605.** The first probe oligonucleotide **604** includes a functional sequence **606** and a sequence **607** that is substantially complementary to a sequence that is 3' of the analyte sequence to which the second probe oligonucleotide hybridizes. The second probe oligonucleotide **605** includes a capture probe binding domain **608** and a sequence **609** that is substantially complementary to an analyte sequence that is 5' of the sequence to which the first probe oligonucleotide hybridizes. In some instances, as shown in **FIG. 6A****,** a blocking moiety is hybridized to the second probe oligonucleotide (RHS poly (dA)) **605** to prevent pre-mature capture of the second probe oligonucleotide on the substrate. In other embodiments, as shown in **FIG. 6B****,** a blocking moiety is hybridized to the capture probe binding domain to prevent premature capture of the second probe oligonucleotide on the substrate. The first probe oligonucleotide and the second probe oligonucleotide hybridize **610** to the mRNA molecule **603.** Briefly, hybridization buffer with the DNA probes are added to the tissue samples and the tissues are incubated at 50°C of approximately 2 ½ hrs. The hybridization/DNA probe buffer is removed and the tissues are washed by addition of a post hybridization buffer without DNA probes and incubation at 50°C for 5 minutes, for a total of 3 post hybridization washes.

Excess first and second probes are washed off and the first and second probe oligonucleotides are ligated, creating a ligation product. To ligate the two DNA probe oligonucleotides that adjacently hybridized on the target mRNAs as described above, a ligase is added to each tissue sample, the tissues are incubated at 37°C for 60 minutes. Following DNA probe ligation the tissue samples are washed.

The biological sample is permeabilized and the ligation product is released (as indicated by the arrow labeled as numeral **611)** from the biological sample. RNase H is added to digest the RNA strand of the hybridized RNA:DNA duplex. The biological sample is permeabilized to release the ligated RTL probes from the tissue for hybridizing with the capture domains on the capture probes attached to a substrate. The concentration of the permeabilization enzyme, the temperature of permeabilization and the time of permeabilization could all be factors that are varied to determine the desired permeabilization conditions for a biological sample to allow the ligation products to be released from the biological sample for hybridization to the capture domains on the capture probes. Following the permeabilization condition being tested, the samples are washed to remove the protease. In some instances, a general range for permeabilization testing conditions could include using Proteinase K (as an exemplary protease) at a concentration range greater than or less than 1.25mg/ml, temperature conditions of approximately 22°C to approximately 40°C and times of incubation from 1 min to 30 or more minutes.

Prior to hybridizing the ligation product to the capture domain on the substrate (as indicated by the arrow label as **612),** the capture probe binding domain blocking moiety is removed from the capture probe binding domain of the ligation product or the capture domain of the capture probe, wherever the blocking moiety is attached. The released, ligated DNA probes that serve as a proxy for the target mRNA are allowed to hybridize to the capture domain on the capture probe immobilized on the spatial array via the poly(dA) tail on the 3' end of the RHS probe.

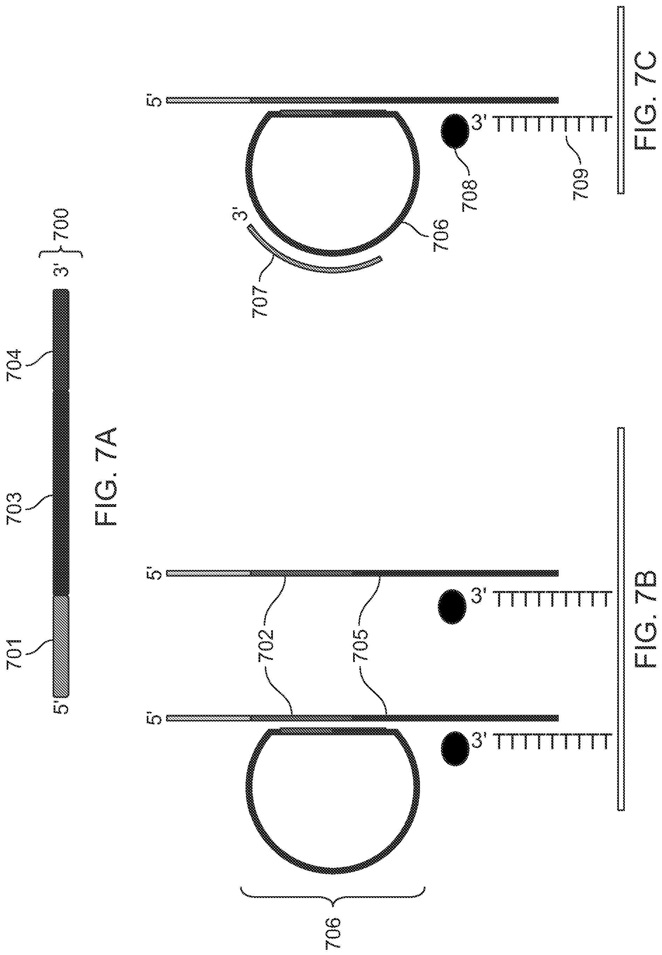

A padlock oligonucleotide is contacted with the ligation product under conditions that allow the padlock oligonucleotide to hybridize to the ligation product. As shown in **FIGs. 7A-7B****,** a padlock oligonucleotide **700** includes a first sequence **701** that is substantially complementary to a first portion **702** of the ligation product, a backbone sequence **703,** and a second sequence **704** that is substantially complementary to a second portion **705** of the ligation product. Therefore, the first sequence and the second sequence are directly adjacent when hybridized to the ligation product. As a result, the second sequence **704** can be ligated to the first sequence **701,** thereby creating a circularized padlock oligonucleotide **706.**

As shown in **FIG. 7C****,** following circularization of the padlock oligonucleotide, an amplification primer **707** is hybridized to the circularized padlock oligonucleotide **706.** Rolling circle amplification (RCA) is used to amplify the circularized padlock oligonucleotide **706** using a Phi29 DNA polymerase, for example. To prevent the capture probe **709** from being extended during the amplification step, the capture probe includes a blocking moiety **708** on the 3' end.

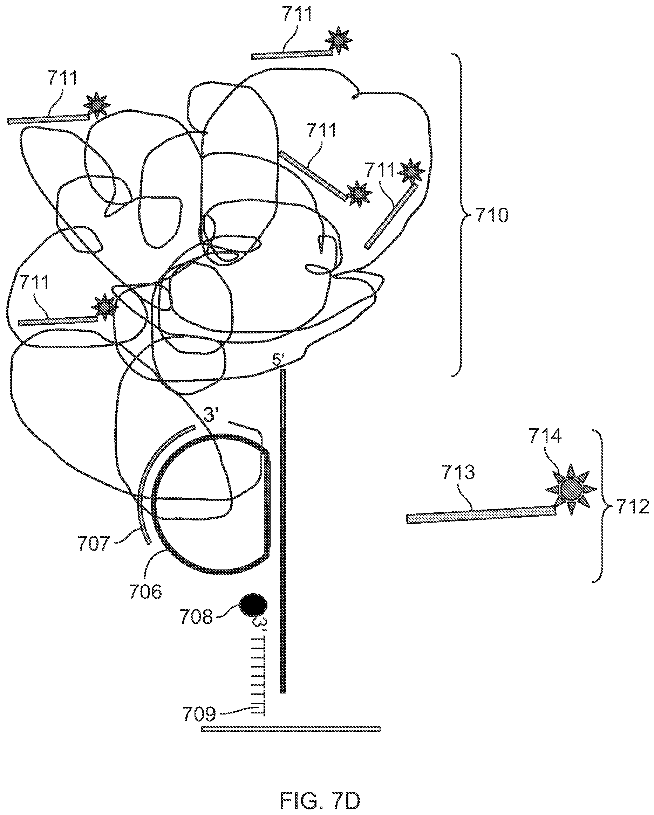

As shown in **FIG. 7D****,** RCA synthesizes continuous single-stranded copies (e.g., amplified circularized padlock oligonucleotide) of the circularized padlock oligonucleotide **706.** Following RCA, the amplified circularized padlock oligonucleotide **710** is contacted with a plurality of detection probes **711,** where a detection probe **712** of the plurality of detection probes include a sequence **713** that is substantially complementary to a sequence of the padlock oligonucleotide and a fluorophore **714.**

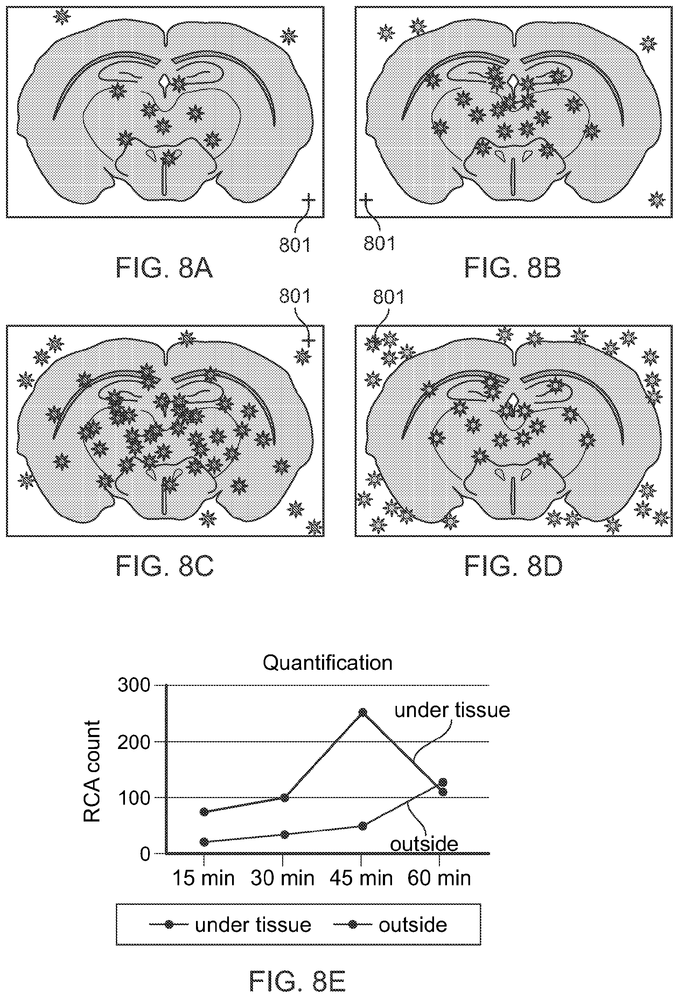

As shown in **FIGs. 8A-8D****,** exemplary images of the detected amplified circularized padlock oligonucleotides (i.e., an amplified circularized padlock oligonucleotide bound by detection probes) are obtained over time and the image coordinates are registered to a fiducial marker **801.** For example, a biological sample is optimized for permeabilization conditions and at different time points detection is measured. The image coordinates of a detected signal are used to identify the location of the analyte in the biological sample. In order to quantify a detected signal, image coordinates corresponding to locations within the tissue are compared to signals detected at image coordinates corresponding to locations outside of the tissue (as shown in **FIG. 8E****).** This data demonstrates the permeabilization conditions that release the analytes for capture on the substrate. For example, **FIG. 8A** and **FIG. 8B** exemplify a biological tissue that might be under permeabilized so that few analytes are captured on the substrate under the tissue relative to analyte capture around the tissue (time points of 15 min and 30 min, respectively). **FIG. 8C** exemplifies permeabilization conditions which could be optimal for the particular tissue in question such that the increase in fluorescence under the tissue is high (i.e., increased analyte capture) comparative to the surrounding tissue (e.g., diffusion of analytes off the tissue), thereby demonstrating permeabilization conditions that will release the analytes for capture, but limit diffusion of analytes off tissue. Conversely, **FIG. 8D** exemplifies a potentially over permeabilized biological sample where the fluorescence under the tissue and off tissue are comparable, thereby exemplifying a permeabilization condition that results in greater diffusion of analytes away from their location in the biological sample, as such decreasing target analyte capture specificity.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

### EMBODIMENTS OF THE INVNETION:

1. A method of identifying abundance and location of an analyte in a biological sample, the method comprising:
   (a) contacting the biological sample with a substrate comprising a plurality of capture probes, wherein a capture probe comprises a capture domain and a blocking moiety at its 3' end;
   (b) hybridizing the analyte to the capture domain;
   (c) hybridizing a padlock oligonucleotide to the analyte hybridized to a capture domain;
   (d) generating a circularized padlock oligonucleotide;
   (e) amplifying the circularized padlock oligonucleotide using rolling circle amplification, thereby generating an amplified circularized padlock oligonucleotide; and
   (f) detecting the amplified circularized padlock oligonucleotide, thereby identifying the abundance and the location of the analyte in the biological sample.
2. A method of identifying abundance and location of an analyte in a biological sample, the method comprising:
   (a) contacting the biological sample with a substrate comprising a plurality of capture probes, wherein a capture probe comprises a capture domain and a blocking moiety at its 3' end;
   (b) delivering a plurality of templated ligation probes, wherein the plurality of probes comprises templated ligation probe pairs, wherein each probe pair comprises a first probe and a second probe, wherein the first probe and the second probe each comprise sequences that are substantially complementary to sequences of the analyte, and wherein the second probe comprises a capture probe capture domain that is complementary to all or a portion of the capture domain of the capture probe on the substrate;
   (c) hybridizing the first probe and the second probe to the analyte;
   (d) generating a ligation product by ligating the first probe and the second probe;
   (e) releasing the ligation product from the analyte;
   (f) hybridizing the ligation product to the capture domain;
   (g) hybridizing a padlock oligonucleotide to the ligation product hybridized to the capture domain;
   (h) generating a circularized padlock oligonucleotide;
   (i) amplifying the circularized padlock oligonucleotide using rolling circle amplification, thereby generating an amplified circularized padlock oligonucleotide; and
   (j) detecting the amplified circularized padlock oligonucleotide, thereby identifying the abundance and the location of the analyte in the biological sample.
3. The method of embodiment 1 or 2, further comprising determining one or more permeabilization conditions.
4. The method of embodiment 3, wherein the method comprises applying the one or more permeabilization conditions to the biological sample.
5. The method of embodiment 3 or 4, wherein the one or more permeabilization conditions are quantitated, wherein the one or more permeabilization conditions are selected from time of permeabilization, temperature of permeabilization, pH of permeabilization, enzyme concentration, and any combination thereof.
6. The method of any one of the preceding embodiments, wherein the padlock oligonucleotide comprises:
   (i) a first sequence that is substantially complementary to a first portion of the analyte,
   (ii) a backbone sequence comprising a barcode sequence that is unique to the ligation product and/or the analyte, and
   (iii) a second sequence that is substantially complementary to a second portion of the analyte,
   wherein the first sequence and the second sequence are substantially complementary to adjacent sequences of the analyte.
7. The method of any one of the preceding embodiments, wherein the analyte is a nucleic acid.
8. The method of embodiment 7, wherein the analyte is DNA.
9. The method of embodiment 7, wherein the analyte is RNA.
10. The method of embodiment 9, wherein the RNA is mRNA.
11. A method of identifying abundance and location of an analyte in a biological sample, the method comprising:
   (a) contacting the biological sample with a substrate comprising a plurality of capture probes, wherein a capture probe comprises a capture domain and a blocking moiety at its 3' end;
   (b) contacting the biological sample with a plurality of analyte capture agents, wherein an analyte capture agent of the plurality of analyte capture agents comprises:
      (i) an analyte binding moiety that binds specifically to the analyte,
      (ii) an analyte binding moiety barcode, and
      (iii) an analyte capture sequence, wherein the analyte capture sequence comprises a sequence that is complementary to the capture domain;
   (c) binding the analyte to the analyte binding moiety;
   (d) hybridizing the analyte capture sequence to the capture domain;
   (e) hybridizing a padlock oligonucleotide to the analyte binding moiety barcode bound to a capture domain;
   (f) generating a circularized padlock oligonucleotide;
   (g) amplifying the circularized padlock oligonucleotide using rolling circle amplification, thereby generating an amplified circularized padlock oligonucleotide; and
   (h) detecting the amplified circularized padlock oligonucleotide, thereby identifying the abundance and the location of the analyte in the biological sample.
12. The method of embodiment 11, further comprising determining one or more permeabilization conditions.
13. The method of embodiment 12, wherein the method comprises applying the one or more permeabilization conditions to the biological sample; optionally, wherein the one or more permeabilization conditions are quantitated, wherein the one or more permeabilization conditions are selected from time of permeabilization, temperature of permeabilization, pH of permeabilization, enzyme concentration, and any combination thereof.
14. The method of any one of embodiments 1-13, wherein the padlock oligonucleotide comprises:
   (i) a first sequence that is substantially complementary to a first portion of the analyte capture sequence,
   (ii) a backbone sequence comprising a barcode sequence that is unique to the analyte capture sequence and/or the analyte, and
   (iii) a second sequence that is substantially complementary to a second portion of the analyte capture sequence,
   wherein the first sequence and the second sequence are substantially complementary to adjacent sequences of the analyte capture sequence.
15. The method of any one of embodiments 11-14, wherein the analyte is selected from of a lipid, a carbohydrate, a peptide, a protein, a glycoproteins (N-linked or O-linked), a lipoprotein, a phosphoprotein, a specific phosphorylated or acetylated variants of a protein, a amidation variant of a protein, a hydroxylation variants of a protein, a methylation variant of a protein, a ubiquitylation variant of a proteins, a sulfation variant of a protein, a viral coat protein, an extracellular protein, an intracellular protein, an antibody, an antigen binding fragment, or any combination thereof.
16. The method of any one of embodiments 11-15, wherein the analyte is a protein.
17. The method of any one of the preceding embodiments, wherein generating the circularized padlock oligonucleotide comprises ligating the first sequence of the padlock oligonucleotide to the second sequence of the padlock oligonucleotide.
18. The method of embodiment 17, wherein the ligating comprises enzymatic ligation or chemical ligation.
19. The method of embodiment 18, wherein the enzymatic ligation utilizes ligase.
20. The method of embodiment 19, wherein ligase comprises a T4 DNA ligase.
21. The method of any one of the preceding embodiments, further comprising digesting the amplified circularized padlock oligonucleotide.
22. The method of embodiment 21, wherein digesting comprises contacting the amplified circularized padlock oligonucleotide with an enzyme that cleaves nucleic acid sequences.
23. The method of embodiment 22, wherein the enzyme is uracil-DNA glycosylase (UDG).
24. The method of any one of the preceding embodiments, wherein the amplifying step comprises:
   hybridizing one or more amplification primers to the padlock oligonucleotide, ligation product, the analyte, the analyte binding moiety barcode or a combination thereof; and amplifying the padlock oligonucleotide with a polymerase.
25. The method of embodiment 24, wherein the polymerase has strand displacement activity.
26. The method of embodiment 24 or 25, wherein the polymerase is a Phi29 DNA polymerase.
27. The method of any one of embodiments 24-26, wherein the one or more amplification primers is substantially complementary to the backbone sequence of the padlock oligonucleotide.
28. The method of any one of embodiments 24-27, wherein the one or more amplification primers comprises a sequence substantially complementary to the backbone sequence of the padlock oligonucleotide and a sequence substantially complementary to a portion of the ligation product, the analyte, or the analyte binding moiety barcode.
29. The method of any one of the preceding embodiments, further comprising tethering the primer to the capture probe on the substrate.
30. The method of embodiment 29, wherein tethering comprises ligating the capture probe to the primer.
31. The method of embodiment 30, further comprising generating a free 3' OH on the capture probe.
32. The method of any one of embodiments 29-31, wherein the tethering comprises:
   generating a free 3' OH on the capture probe;
   extending the capture probe to generate an extended capture probe; and
   ligating the extended capture probe to the primer.
33. The method of embodiment 31 or 32, wherein generating a free '3 OH on the capture probe comprises:
   removing a blocking moiety from the 3' end of the capture probe; or
   adding additional nucleic acids to the 3' end of the capture probe.
34. The method of 33, wherein removing the blocking moiety from the 3' end of the capture probe comprises cleaving the oligonucleotide with an endonuclease.
35. The method any one of the preceding embodiments, detecting the amplified circularized padlock oligonucleotide comprises detecting a signal corresponding to the amplified circularized padlock oligonucleotide.
36. The method of embodiment 35, wherein detecting the signal comprises detecting a fluorescently labelled detection probe that is hybridized to the amplified circularized padlock oligonucleotide.
37. The method of any one of the preceding embodiments, wherein detecting the amplified circularized padlock oligonucleotide further:
   contacting the amplified circularized padlock oligonucleotide with a plurality of detection probes, wherein a detection probe from the plurality of detection probes comprises:
   a sequence that is substantially complementary to a sequence of the padlock oligonucleotide, and
   a detectable label.
38. The method of embodiment 37, wherein the detection probe comprises a sequence that is substantially complementary to a backbone sequence.
39. The method of embodiment 37 or 38, further comprising quantitating the detectable label using microscopy.
40. The method of any one of the preceding embodiments, further comprising obtaining an image corresponding to the amplified circularized padlock oligonucleotide.
41. The method of any one of the preceding embodiments, wherein the substrate comprises a plurality of fiducial markers.
42. The method of embodiment 41, further comprising registering image coordinates to one or more of the plurality of fiducial markers.
43. The method of any one of embodiments 37-42, wherein the detectable label comprises a fluorophore.
44. The method of any one of the preceding embodiments, wherein the capture probe is affixed to the substrate at a 5' end of the capture probe.
45. The method of any one of the preceding embodiments, wherein the capture probe further comprises a spatial barcode.
46. The method of any one of the preceding embodiments, wherein the plurality of capture probes are uniformly distributed on a surface of the substrate.
47. The method of any one of the preceding embodiments, wherein the plurality of capture probes are not uniformly distributed on the surface of the substrate.
48. The method of any one of the preceding embodiments, wherein the capture domain comprises a sequence that is at least partially complementary to the ligation product, the analyte, or the analyte binding moiety barcode.
49. The method of any one of the preceding embodiments, wherein the capture domain of the capture probe comprises a homopolymeric sequence.
50. The method of any one of the preceding embodiments, wherein the capture domain of the capture probe comprises a poly(T) sequence.
51. The method of any one of the preceding embodiments, wherein the capture probe further comprises one or more functional domains, a unique molecular identifier, a cleavage domain, and combinations thereof.
52. The method of any one of the preceding embodiments, wherein the blocking moiety is a reversible blocking moiety.
53. The method of embodiment 52, wherein the blocking moiety is a removable blocking domain, wherein removal restores the free 3' end of the capture probe.
54. The method of any one of the preceding embodiments, wherein the 3' end of the capture probe comprises a removable hairpin, wherein the hairpin blocks extension of the 3' end of the capture probe.
55. The method of embodiment 54, wherein removing the hairpin results in the capture probe comprising a free 3' end.
56. The method of embodiment 55, wherein additional nucleotides are added in order to reveal and/or restore the blocked 3' end of the capture probe, thereby creating a free 3' end.
57. The method of any one of the preceding embodiments, wherein the biological sample comprises a formalin-fixed, paraffin-embedded (FFPE) sample.
58. The method of any one of the preceding embodiments, wherein the biological sample comprises a tissue section.
59. The method of any one of the preceding embodiments, wherein the biological sample comprises a fresh frozen sample.
60. The method of any one of the preceding embodiments, wherein the biological sample comprises live cells.
61. A kit, comprising:
   (a) one or more padlock oligonucleotides and a ligase;
   (b) one or more primers and a polymerase;
   (c) a substrate comprising a plurality of capture probes, wherein a capture probe of the plurality of capture probes comprises a capture domain, and a blocking moiety at its 3' end; and
   (d) instructions for performing the method of any one of the preceding embodiments.
62. The kit of embodiment 61, further comprising one or more fluorescent dyes and/or one or a plurality of detection probes, wherein a detection probe from the plurality of detection probes comprises a sequence that is substantially complementary to a sequence of the padlock oligonucleotide and a detectable label.
63. The kit of embodiment 61 or 62, further comprising a uracil-DNA glycosylase enzyme.
64. The kit of any one of embodiments 61-63, further comprising a plurality of templated ligation probes, wherein the plurality of templated ligation probes comprises probe pairs, wherein each probe pair comprises a first probe and a second probe, wherein the first probe and the second probe comprise sequences that are substantially complementary to sequences of the analyte, and wherein either the first or the second probe comprises a capture probe capture domain that is complementary to all or a portion of the capture domain.
65. The kit of any one of embodiments 61-64, further comprising a plurality of analyte capture agents, wherein an analyte capture agent of the plurality of analyte capture agents comprises:
   (i) an analyte binding moiety that binds specifically to the analyte,
   (ii) an analyte binding moiety barcode, and
   (iii) an analyte capture sequence, wherein the analyte capture sequence comprises a sequence that is complementary to the capture domain.
66. The kit of any one of embodiments 61-65, wherein the capture probe comprises a spatial barcode.
67. A composition comprising:
   (a) one or more padlock oligonucleotides and a ligase;
   (b) one or more primers and a polymerase;
   (c) a substrate comprising a plurality of capture probes, wherein a capture probe of the plurality of capture probes comprises a capture domain, and a blocking moiety at its 3' end;
   (d) a biological sample placed on the substrate; and
   (e) one or more fluorescent dyes and/or one or a plurality of detection probes,
   wherein a detection probe from the plurality of detection probes comprises a sequence that is substantially complementary to a sequence of the padlock oligonucleotide and a detectable label.
68. The composition of embodiment 67, further comprising a plurality of templated ligation probes, wherein the plurality of probes comprises probe pairs, wherein each probe pair comprises a first probe and a second probe, wherein the first probe and the second probe comprise sequences that are substantially complementary to sequences of the analyte, and wherein the first or the second probe comprises a capture probe capture domain that is complementary to all or a portion of the capture domain.
69. The composition of embodiment 67 or 68, further comprising a plurality of analyte capture agents, wherein an analyte capture agent of the plurality of analyte capture agents comprises:
   (i) an analyte binding moiety that binds specifically to the analyte,
   (ii) an analyte binding moiety barcode, and
   (iii) an analyte capture sequence, wherein the analyte capture sequence comprises a sequence that is complementary to the capture domain.
70. The composition of any one of embodiments 67-69, wherein the capture probe comprises a spatial barcode.

## Claims

1. A method of identifying abundance and location of a nucleic acid analyte in a tissue sample, the method comprising:
(a) contacting the tissue sample with a substrate comprising a plurality of capture probes, wherein a capture probe of the plurality of capture probes comprises a capture domain and a blocking moiety at its 3' end;
(b) hybridizing the nucleic acid analyte to the capture domain;
(c) hybridizing a padlock oligonucleotide to the nucleic acid analyte hybridized to the capture domain, wherein the padlock oligonucleotide comprises:
(i) a first sequence that is substantially complementary to a first portion of the nucleic acid analyte,
(ii) a backbone sequence comprising a barcode sequence that uniquely identifies the nucleic acid analyte, and
(iii) a second sequence that is substantially complementary to a second portion of the nucleic acid analyte,
wherein the first sequence and the second sequence are substantially complementary to adjacent sequences of the nucleic acid analyte;
(d) generating a circularized padlock oligonucleotide;
(e) amplifying the circularized padlock oligonucleotide using rolling circle amplification, thereby generating an amplified circularized padlock oligonucleotide, wherein the method further comprises tethering one or more amplification primers to the capture probe on the substrate, wherein amplifying the circularized padlock oligonucleotide comprises hybridizing the one or more amplification primers to the padlock oligonucleotide and amplifying the padlock oligonucleotide with a polymerase, and wherein the one or more amplification primers is substantially complementary to the backbone sequence of the padlock oligonucleotide; and
(f) detecting the amplified circularized padlock oligonucleotide, thereby identifying the abundance and the location of the nucleic acid analyte in the tissue sample;
wherein detecting the amplified circularized padlock oligonucleotide comprises:
contacting the amplified circularized padlock oligonucleotide with a plurality of detection probes, wherein a detection probe from the plurality of detection probes comprises:
a sequence that is substantially complementary to a sequence of the padlock oligonucleotide, wherein the sequence of the detection probe that is substantially complementary to a sequence of the padlock oligonucleotide is substantially complementary to the barcode sequence of the padlock oligonucleotide, and
a detectable label,
and wherein the detection probe hybridizes to the amplified circularized padlock oligonucleotide.

2. A method of identifying abundance and location of a nucleic acid analyte in a tissue sample, the method comprising:
(a) contacting the tissue sample with a substrate comprising a plurality of capture probes, wherein a capture probe comprises a capture domain and a blocking moiety at its 3' end;
(b) delivering a plurality of templated ligation probes, wherein the plurality of probes comprises templated ligation probe pairs, wherein each probe pair comprises a first probe and a second probe, wherein the first probe and the second probe each comprise sequences that are substantially complementary to sequences of the nucleic acid analyte, and wherein the second probe comprises a capture probe capture domain that is complementary to all or a portion of the capture domain of the capture probe on the substrate;
(c) hybridizing the first probe and the second probe to the nucleic acid analyte;
(d) generating a ligation product by ligating the first probe and the second probe;
(e) releasing the ligation product from the nucleic acid analyte;
(f) hybridizing the ligation product to the capture domain;
(g) hybridizing a padlock oligonucleotide to the ligation product hybridized to the capture domain, wherein the padlock oligonucleotide comprises:
(i) a first sequence that is substantially complementary to the sequence of the first probe of the ligation product,
(ii) a backbone sequence comprising a barcode sequence that uniquely identifies the ligation product, and
(iii) a second sequence that is substantially complementary to the sequence of the first probe of the ligation product,
wherein the first sequence and the second sequence are substantially complementary to adjacent sequences of the ligation product;
(h) generating a circularized padlock oligonucleotide;
(i) amplifying the circularized padlock oligonucleotide using rolling circle amplification, thereby generating an amplified circularized padlock oligonucleotide, wherein the method further comprises tethering one or more amplification primers to the capture probe on the substrate, wherein amplifying the circularized padlock oligonucleotide comprises hybridizing the one or more amplification primers to the padlock oligonucleotide and amplifying the padlock oligonucleotide with a polymerase, and wherein the one or more amplification primers is substantially complementary to the backbone sequence of the padlock oligonucleotide; and
(j) detecting the amplified circularized padlock oligonucleotide, thereby identifying the abundance and the location of the nucleic acid analyte in the tissue sample wherein detecting the amplified circularized padlock oligonucleotide comprises:
contacting the amplified circularized padlock oligonucleotide with a plurality of detection probes, wherein a detection probe from the plurality of detection probes comprises:
a sequence that is substantially complementary to a sequence of the padlock oligonucleotide, wherein the sequence of the detection probe that is substantially complementary to a sequence of the padlock oligonucleotide is substantially complementary to the barcode sequence of the padlock oligonucleotide, and
a detectable label,
and wherein the detection probe hybridizes to the amplified circularized padlock oligonucleotide.

3. The method of claim 1 or 2, wherein the nucleic acid analyte is DNA, RNA, or mRNA.

4. A method of identifying abundance and location of a protein analyte in a tissue sample, the method comprising:
(a) contacting the tissue sample with a substrate comprising a plurality of capture probes, wherein a capture probe comprises a capture domain and a blocking moiety at its 3' end;
(b) contacting the tissue sample with a plurality of analyte capture agents, wherein an analyte capture agent of the plurality of analyte capture agents comprises:
(i) an analyte binding moiety that binds specifically to the protein analyte,
(ii) an analyte binding moiety barcode that identifies the analyte binding moiety, and
(iii) an analyte capture sequence, wherein the analyte capture sequence comprises a sequence that is complementary to the capture domain;
(c) binding the protein analyte to the analyte binding moiety;
(d) hybridizing the analyte capture sequence of an analyte capture agent bound to the protein analyte to the capture domain;
(e) hybridizing a padlock oligonucleotide to the analyte binding moiety barcode of an analyte capture agent bound to the protein analyte and bound to a capture domain, wherein the padlock oligonucleotide comprises:
(i) a first sequence that is substantially complementary to a first portion of the analyte binding moiety barcode,
(ii) a backbone sequence comprising a barcode sequence that uniquely identifies the analyte capture sequence, and
(iii) a second sequence that is substantially complementary to a second portion of the analyte binding moiety barcode,
wherein the first sequence and the second sequence are substantially complementary to adjacent sequences of the analyte binding moiety barcode;
(f) generating a circularized padlock oligonucleotide;
(g) amplifying the circularized padlock oligonucleotide using rolling circle amplification, thereby generating an amplified circularized padlock oligonucleotide, wherein the method further comprises tethering one or more amplification primers to the capture probe on the substrate, wherein amplifying the circularized padlock oligonucleotide comprises hybridizing the one or more amplification primers to the padlock oligonucleotide and amplifying the padlock oligonucleotide with a polymerase, and wherein the one or more amplification primers is substantially complementary to the backbone sequence of the padlock oligonucleotide; and
(h) detecting the amplified circularized padlock oligonucleotide, thereby identifying the abundance and the location of the protein analyte in the tissue sample; wherein detecting the amplified circularized padlock oligonucleotide further comprises:
contacting the amplified circularized padlock oligonucleotide with a plurality of detection probes, wherein a detection probe from the plurality of detection probes comprises:
a sequence that is substantially complementary a sequence of the padlock oligonucleotide, wherein the sequence of the detection probe that is substantially complementary to a sequence of the padlock oligonucleotide is substantially complementary to the barcode sequence of the padlock oligonucleotide, and
a detectable label,
and wherein the detection probe hybridizes to the amplified circularized padlock oligonucleotide.

5. The method of claim 4, wherein the protein analyte is selected from a glycoprotein (N-linked or O-linked), a lipoprotein, a phosphoprotein, a specific phosphorylated or acetylated variants of a protein, a amidation variant of a protein, a hydroxylation variants of a protein, a methylation variant of a protein, a ubiquitylation variant of a proteins, a sulfation variant of a protein, a viral coat protein, an extracellular protein, an intracellular protein, an antibody, an antigen binding fragment, or any combination thereof.

6. The method of any of the preceding claims, further comprising determining one or more permeabilization conditions, preferably wherein the method comprises applying the one or more permeabilization conditions to the tissue sample; optionally, wherein the one or more permeabilization conditions are quantitated, wherein the one or more permeabilization conditions are selected from time of permeabilization, temperature of permeabilization, pH of permeabilization, enzyme concentration, or any combination thereof.

7. The method of any one of the preceding claims, wherein generating the circularized padlock oligonucleotide comprises ligating the first sequence of the padlock oligonucleotide to the second sequence of the padlock oligonucleotide; preferably wherein the ligating comprises enzymatic ligation or chemical ligation.

8. The method of any one of the preceding claims, further comprising digesting the amplified circularized padlock oligonucleotide; preferably wherein digesting comprises contacting the amplified circularized padlock oligonucleotide with an enzyme that cleaves nucleic acid sequences, such as wherein the enzyme is uracil-DNA glycosylase (UDG).

9. The method of any one of the preceding claims, wherein the polymerase has strand displacement activity and/or wherein the polymerase is a Phi29 DNA polymerase.

10. The method of any one of the preceding claims, wherein: tethering comprises: (i) ligating the capture probe to the primer, optionally wherein the method further comprises generating a free 3' OH on the capture probe; and/or (ii) generating a free 3' OH on the capture probe; extending the capture probe to generate an extended capture probe; and ligating the extended capture probe to the primer.

11. The method of any one of the preceding claims, wherein detecting the amplified circularized padlock oligonucleotide comprises detecting a fluorescently labelled detection probe that is hybridized to the amplified circularized padlock oligonucleotide.

12. The method of any preceding claim, further comprising quantitating the detectable label using microscopy.

13. The method of any preceding claim, wherein the detectable label comprises a fluorophore.

14. The method of any one of the preceding claims, wherein the capture probe is affixed to the substrate at a 5' end of the capture probe.
